# EUROPÄISCHE PATENTANMELDUNG

(11) **EP 2 623 504 A1**
(43) Veröffentlichungstag der Anmeldung: **07.08.2013**
(21) Anmeldenummer: 12194641.2
(22) Anmeldetag: 05.12.2008
(51) Int. Cl.: C07D 401/14, A61K 31/496, A61K 31/4545, A61P 3/10, A61P 7/02, A61P 9/00, A61P 25/00

(54) **5,6-Disubstituierte Oxindol-Derivate und ihre Verwendung zur Herstellung eines Medikaments zur Behandlung von Vasopressin-abhängigen Erkrankungen**

(30) Priorität: 07.12.2007 US 12265 P
(62) Teilanmeldung aus: 08857372.0
(71) Anmelder: Abbott GmbH & Co. KG, 65205 Wiesbaden (DE)
(72) Erfinder: Braje, Wilfried, 67061 Ludwigshafen (DE); Oost, Thorsten, 88400 Biberach an der Riss (DE); Netz, Astrid, 67061 Ludwigshafen (DE); Wernet, Wolfgang, 67061 Ludwigshafen (DE); Unger, Liliane, 67061 Ludwigshafen (DE); Hornberger, Wilfried, 67061 Ludwigshafen (DE); Lubisch, Wilfried, 69118 Heidelberg (DE)
(74) Vertreter: Schuster-Haberhauer, Andreea

(57) **Zusammenfassung**

Die vorliegende Erfindung betrifft neue 5,6-disubstituierte Oxindol-Derivate der Formel I, diese enthaltende pharmazeutische Mittel und ihre Verwendung zur Herstellung eines Medikaments. 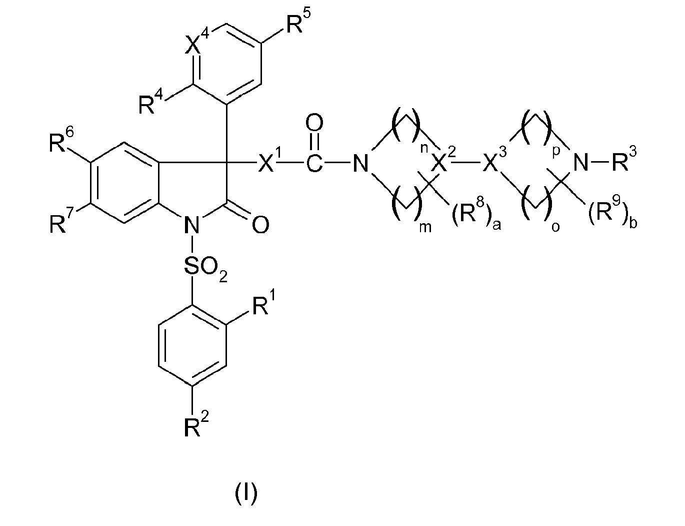 worin
R¹ und R² unabhängig voneinander für Wasserstoff, C₁-C₃-Alkyl, C₁-C₃-Fluoralkyl, C₁-C₃-Alkoxy, C₁-C₃-Fluoralkoxy, Halogen oder CN stehen;
R³ für Wasserstoff oder C₁-C₄-Alkyl steht;
R⁴ für Ethoxy, fluoriertes Ethoxy oder Isopropoxy steht;
R⁵ für Wasserstoff oder Methyl steht;
R⁶ für Br, Cl, F oder CN steht;
R⁷ für Cl, F oder CN steht;
R⁸ und R⁹ unabhängig voneinander für C₁-C₃-Alkyl oder C₁-C₃-Fluoralkyl stehen;
X¹ für O, NH oder CH₂ steht;
X² und X³ für N oder CH stehen, unter der Maßgabe, dass X² und X³ nicht gleichzeitig für N stehen;
X⁴ für N oder CH steht;
a und b unabhängig voneinander für 0, 1 oder 2 stehen; und
m, n, o und p unabhängig voneinander für 1, 2 oder 3 stehen.

## Beschreibung

Die vorliegende Erfindung betrifft neue substituierte Oxindol-Derivate, diese enthaltende pharmazeutische Mittel und ihre Verwendung zur Behandlung von Vasopressin-abhängigen Erkrankungen.

Vasopressin ist ein endogenes Hormon, das verschiedenste Wirkungen an Organen und Gewebe ausübt. Bei verschiedenen Krankheitszuständen, wie zum Beispiel Herzinsuffizienz und Bluthochdruck, vermutet man, dass das Vasopressin-System eine Rolle spielt. Derzeit sind drei Rezeptoren (V1a, V1b bzw. V3 und V2) bekannt, über die Vasopressin seine zahlreichen Wirkungen vermittelt. Daher werden Antagonisten dieser Rezeptoren als mögliche neue therapeutische Ansätze zur Behandlung von Krankheiten untersucht (M. Thibonnier, Exp.Opin. Invest. Drugs 1998, 7(5), 729-740).

Vorliegend werden neue substituierte Oxindole beschrieben, die in der 1-Position eine Phenylsulfonyl-Gruppe tragen. 1-Phenylsulfonyl-1,3-dihydro-2*H*-indol-2-one wurden bereits als Liganden der Vasopressin-Rezeptoren beschrieben. Auch die WO 93/15051, WO 95/18105, WO 98/25901, WO 01/55130, WO 01/55134, WO 01/164668 und WO 01/98295 beschreiben Derivate, die in der 1-Position des Oxindolgerüsts Arylsulfonlygruppen tragen. Diese Verbindungen unterscheiden sich von den erfindungsgemäßen Verbindungen wesentlich durch die Substituenten in der 3-Position.

So werden in der WO 93/15051 und WO 98/25901 1-Phenylsulfonyl-1,3-dihydro-2H-indol-2-one als Liganden der Vasopressinrezeptoren beschrieben, bei denen das Oxindol-Gerüst in der 3-Position durch zwei Alkylreste substituiert ist, die gemeinsam auch einen Cycloalkylrest (Spiroverknüpfung) bilden können. Als Alternative kann der Spiroring Heteroatome, wie Sauerstoff und Stickstoff (wahlweise mit Substituenten), enthalten.

Die WO 95/18105 beschreibt 1-Phenylsulfonyl-1,3-dihydro-2H-indol-2-one als Liganden der Vasopressinrezeptoren, die ein Stickstoffatom in der 3-Position besitzen. Zusätzlich sind in der 3-Position Reste gebunden, die unter gegebenenfalls substituierten Alkyl-, Cycloalkyl-, Phenyl- oder Benzylresten ausgewählt sind.

In der WO 03/008407 werden 1-Phenylsulfonyloxindole beschrieben, in denen in der 3-Position Pyridylpiperazine über eine Harnstoff-, Carbamat- oder 2-Oxoethyl-Gruppe am Oxindol gebunden sind.

Die WO 2005/030755 betrifft 1-Phenylsulfonyloxindole, in denen in der 3-Position Piperidylpiperazine oder Piperazinylpiperidine über eine Harnstoff-, Carbamat- oder 2-Oxoethyl-Gruppe am Oxindol gebunden sind. 5,6-Disubstituierte Oxindole werden allerdings nicht konkret beschrieben.

Neben der Bindungsaffinität zum Vasopressin V1 b Rezeptor können weitere Eigenschaften vorteilhaft für die Behandlung und/oder Prophylaxe von Vasopressin-abhängigen Erkrankungen sein, wie beispielsweise:
1.) eine Selektivität zum Vasopressin V1 b Rezeptor im Vergleich zum Vasopressin V1 a Rezeptor, d.h. der Quotient aus der Bindungsaffinität zum V1a Rezeptor (Ki(V1a) (bestimmt in der Einheit "Nanomolar (nM)") und der Bindungsaffinität zum V1b Rezeptor (Ki(V1 b)) (bestimmt in der Einheit "Nanomolar (nM)"). Je größer der Quotient Ki(V1 a)/Ki(V1 b) ist, desto größer ist die V1b-Selektivität;
2.) eine Selektivität zum Vasopressin V1 b Rezeptor im Vergleich zum Vasopressin V2 Rezeptor, d.h. der Quotient aus der Bindungsaffinität zum V2 Rezeptor (Ki(V2) (bestimmt in der Einheit "Nanomolar (nM)") und der Bindungsaffinität zum V1 b Rezeptor (Ki(V1 b)) (bestimmt in der Einheit "Nanomolar (nM)"). Je größer der Quotient Ki(V2)/Ki(V1 b) ist, desto größer ist die V1 b-Selektivität;
3.) eine Selektivität zum Vasopressin V1 b Rezeptor im Vergleich zum Oxytozin OT Rezeptor, d.h. der Quotient aus der Bindungsaffinität zum OT Rezeptor (Ki(OT) (bestimmt in der Einheit "Nanomolar (nM)") und der Bindungsaffinität zum V1 b Rezeptor (Ki(V1b)) (bestimmt in der Einheit "Nanomolar (nM)"). Je größer der Quotient Ki(OT)/Ki(V1 b) ist, desto größer ist die V1 b-Selektivität.
4.) die metabolische Stabilität, beispielsweise bestimmt anhand der in-vitro ermittelten Halbwertszeiten in Lebermikrosomen von verschiedenen Spezies (z.B. Ratte oder Mensch);
5.) keine oder nur geringe Inhibierung von Cytochrom P450 (CYP) Enzymen: Cytochrom P450 (CYP) ist die Bezeichnung für eine Superfamilie von Hämproteinen mit enzymatischer Aktivität (Oxidase). Besondere Bedeutung besitzen sie auch für den Abbau (Metabolismus) von Fremdstoffen wie Pharmaka oder Xenobiotika in Säugetierorganismen. Die wichtigsten Vertreter der Typen und Untertypen von CYP im menschlichen Organismus sind: CYP 1A2, CYP 2C9, CYP 2D6 und CYP 3A4. Bei gleichzeitiger Anwendung von CYP 3A4-Hemmstoffen (z.B. Grapefruitsaft, Cimetidin, Erythromycin) und Arzneistoffen, die über dieses Enzymsystem abgebaut werden und somit um die gleiche Bindungsstelle am Enzym konkurrieren, kann deren Abbau verlangsamt und dadurch Wirkungen und Nebenwirkungen des verabreichten Arzneistoffs unerwünscht verstärkt werden;
6.) eine geeignete Wasserlöslichkeit (in mg/ml);
7.) eine geeignete Pharmakokinetik (zeitlicher Verlauf der Konzentration der erfindungsgemässen Verbindung im Plasma bzw. in Geweben, zum Beispiel Gehirn). Die Pharmakokinetik kann durch die folgenden Parameter beschrieben werden: Halbwertszeit (in h), Verteilungsvolumen (in 1-kg⁻¹), Plasma-Clearance (in 1-h⁻¹kg⁻¹), AUC ("area under the curve", Fläche unter der Konzentrations-Zeit-Kurve, in ng·h·l⁻¹), orale Bioverfügbarkeit (das Dosis-normalisierte Verhältnis von AUC nach oraler Gabe und AUC nach intravenöser Gabe), das sog. Brain-Plasma-Ratio (das Verhältnis von AUC im Hirngewebe und AUC im Plasma);
8.) keine oder nur geringe Blockade des hERG-Kanals: Verbindungen, die den hERG-Kanal blockieren, können eine Verlängerung des QT-Intervalls verursachen und dadurch zu ernsten Herz-Rhythmus-Störungen führen (zum Beispiel sogenannte "torsade de pointes"). Mittels eines in der Literatur beschriebenen Verdrängungsassays mit radioaktiv markiertem Dofetilid (G. J. Diaz et al., Journal of Pharmacological and Toxicological Methods, 50 (2004), 187-199) kann das Potential von Verbindungen, den hERG-Kanal zu blockieren, bestimmt werden. Je geringer der IC50 in diesem "dofetilide assay", desto wahrscheinlicher ist eine potente hERG-Blockade. Darüber hinaus kann die Blockade des hERG-Kanals durch elektrophysiologische Experimente an Zellen, die mit dem hERG-Kanal transfiziert wurden, durch sogenanntes "whole-cell patch clamping" gemessen werden (G. J. Diaz et al., Journal of Pharmacological and Toxicological Methods, 50 (2004), 187-199).

Aufgabe der vorliegenden Erfindung war es, Verbindungen zur Behandlung oder Prophylaxe von verschiedenen Vasopressin-abhängigen Krankheiten zur Verfügung zu stellen. Die Verbindungen sollten eine hohe Aktivität und Selektivität aufweisen, vor allem eine hohe Affinität und Selektivität gegebenüber dem Vasopressin V1 b Rezeptor. Zusätzlich sollte die erfindungsgemäße Substanz einen oder mehrere der vorstehend genannten Vorteile 1.) bis 8.) besitzen.

Die Aufgabe wird durch Verbindungen der Formel I gelöst 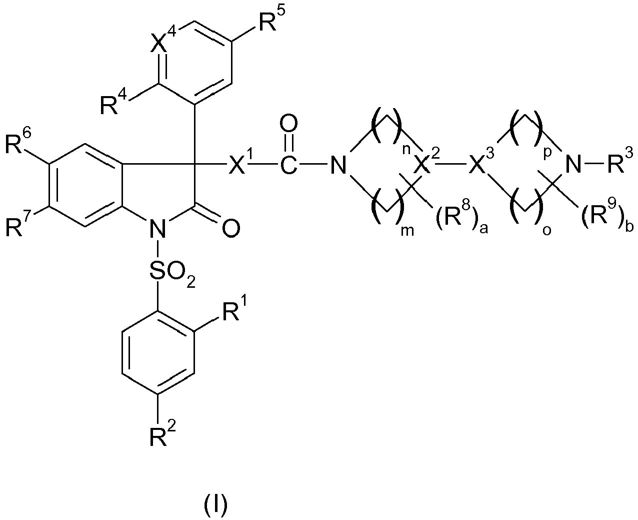 worin
- R¹ und R²: unabhängig voneinander für Wasserstoff, C₁-C₃-Alkyl, C₁-C₃-Fluoralkyl, C₁-C₃-Alkoxy, C₁-C₃-Fluoralkoxy, Halogen oder CN stehen;
- R³: für Wasserstoff oder C₁-C₄-Alkyl steht;
- R⁴: für Ethoxy, fluoriertes Ethoxy oder Isopropoxy steht;
- R⁵: für H oder Methyl steht;
- R⁶: für Br, Cl, F oder CN steht;
- R⁷: für Cl, F oder CN steht;
- R⁸ und R⁹: unabhängig voneinander für C₁C₃-Alkyl oder C₁-C₃-Fluoralkyl stehen;
- X¹: für O, NH oder CH₂ steht;
- X² und X³: für N oder CH stehen, unter der Maßgabe, dass X² und X³ nicht gleichzeitig für N stehen;
- X⁴: für N oder CH steht;
- a und b: unabhängig voneinander für 0, 1 oder 2 stehen; und

- m, n, o und p: unabhängig voneinander für 1, 2 oder 3 stehen;
sowie deren pharmazeutisch verträglichen Salze und Prodrugs davon.

Dementsprechend betrifft die vorliegende Erfindung Verbindungen der Formel I (im Folgenden auch "Verbindungen I") sowie die pharmazeutisch verträgliche Salze der Verbindungen I und die Prodrugs der Verbindungen I.

Die pharmazeutisch verträglichen Salze von Verbindungen der Formel I, die auch als physiologisch verträgliche Salze bezeichnet werden, sind in der Regel durch Umsetzung der freien Base der erfindungsgemäßen Verbindungen I (d.h. der Verbindungen I gemäß Strukturformel I) mit geeigneten Säuren erhältlich. Geeignete Säuren sind beispielsweise aufgeführt in "Fortschritte der Arzneimittelforschung", 1966, Birkhäuser Verlag, Bd.10, S.224-285. Darunter fallen beispielsweise Salzsäure, Zitronensäure, Weinsäure, Milchsäure, Phosphorsäure, Methansulfonsäure, Essigsäure, Ameisensäure, Maleinsäure und Fumarsäure.

Unter dem Begriff "Prodrugs" werden solche Verbindungen verstanden, welche in vivo zu den erfindungsgemäßen Verbindungen I metabolisiert werden. Typische Beispiele von Prodrugs sind beschrieben in C.G. Wermeth (Hrsg.): The Practice of Medicinal Chemistry, Academic Press, San Diego, 1996, Seiten 671-715. Darunter fallen beispielsweise Phosphate, Carbamate, Aminosäuren, Ester, Amide, Peptide, Harnstoffe und dergleichen. Im vorliegenden Fall können geeignete Prodrugs beispielsweise solche Verbindungen I sein, in denen das äußere Stickstoffatom des äußeren stickstoffhaltigen Rings eine Amid- /Peptidbindung bildet, indem dieses Stickstoffatom durch eine C₁-C₄-Alkylcarbonylgruppe, z.B. durch Acetyl, Propionyl, n-Propylcarbonyl, Isopropylcarbonyl, n-Butylcarbonyl oder tert-Butylcarbonyl (Pivaloyl), durch Benzoyl, oder durch einen über CO gebundenen Aminosäurerest, z.B. über CO gebundenes Glycin, Alanin, Serin, Phenylalanin und dergleichen, in der Position des Restes R³ substituiert ist. Als Prodrugs geeignet sind weiterhin Alkylcarbonyloxyalkylcarbamate, in denen das äußere Stickstoffatom des äußeren stickstoffhaltigen Rings in der Position des Restes R³ eine Gruppe der Formel -C(=O)-O-CHR^{a}-O-C(=O)-R^{b} trägt, worin R^{a} und R^{b} unabhängig voneinander für C₁-C₄-Alkyl stehen. Solche Carbamate sind beispielsweise in J. Alexander, R. Cargill, S. R. Michelson, H. Schwam, J. Medicinal Chem. 1988, 31(2), 318-322 beschrieben. Diese Gruppen können dann unter metabolischen Bedingungen abgespalten werden und resultieren in Verbindungen I, in denen R³ für H steht.

C₁-C₃-Alkyl steht im Rahmen der vorliegenden Erfindung für einen linearen oder verzweigten Alkylrest mit 1 bis 3 Kohlenstoffatomen, wie Methyl, Ethyl, n-Propyl oder Isopropyl.

C₁-C₄-Alkyl steht im Rahmen der vorliegenden Erfindung für einen linearen oder verzweigten Alkylrest mit 1 bis 4 Kohlenstoffatomen, wie Methyl, Ethyl, n-Propyl, Isopropyl, n-Butyl, sek-Butyl, Isobutyl oder tert-Butyl.

C₁-C₃-Fluoralkyl steht im Rahmen der vorliegenden Erfindung für einen linearen oder verzweigten Alkylrest mit 1 bis 3 Kohlenstoffatomen, wie oben definiert, in welchem wenigstens ein Wasserstoffatom, z.B. 1, 2, 3, 4 oder 5 Wasserstoffatome, durch Fluoratome ersetzt sind. Beispiel hierfür sind Fluormethyl, Difluormethyl, Trifluormethyl, 1- und 2-Fluorethyl, 1,1-, 1,2- und 2,2-Difluorethyl, 1,1,2-, 1,2,2 und 2,2,2-Trifluorethyl, 1,1,2,2-Tetrafluorethyl, 1,2,2,2-Tetrafluorethyl, Pentafluorethyl, 1-, 2- und 3-Fluorprop-1-yl, 1,1-, 1,2-, 1,3-, 2,2-, 2,3- und 3,3-Difluorprop-1-yl, 1,1,2-, 1,2,2-, 1,1,3-, 2,2,3-, 1,2,3- und 3,3,3-Trifluorprop-1-yl, 1- und 2-Fluorprop-2-yl, 1,1- und 1,3-Difluorprop-2-yl, 1,1,1-Trifluorprop-2-yl und dergleichen.

C₁-C₃-Alkoxy steht im Rahmen der vorliegenden Erfindung für einen über ein Sauerstoffatom gebundenen linearen oder verzweigten Alkylrest mit 1 bis 3 Kohlenstoffatomen. Beispiele sind Methoxy, Ethoxy, n-Propoxy und Isopropoxy.

C₁-C₃-Fluoralkoxy steht im Rahmen der vorliegenden Erfindung für einen über ein Sauerstoffatom gebundenen linearen oder verzweigten Alkylrest mit 1 bis 3 Kohlenstoffatomen, wie oben definiert, in welchem wenigstens ein Wasserstoffatom, z.B. 1, 2, 3, 4 oder 5 Wasserstoffatome, durch Fluoratome ersetzt sind. Beispiel hierfür sind Fluormethoxy, Difluormethoxy, Trifluormethoxy, 1- und 2-Fluorethoxy, 1,1-, 1,2- und 2,2-Difluorethoxy, 1,1,2-, 1,2,2 und 2,2,2-Trifluorethoxy, 1,1,2,2-Tetrafluorethoxy, 1,2,2,2-Tetrafluorethoxy, Pentafluorethoxy, 1-, 2- und 3-Fluorprop-1-oxy, 1,1-, 1,2-, 1,3-, 2,2-, 2,3- und 3,3-Difluorprop-1-oxy, 1,1,2-, 1,2,2-, 1,1,3-, 2,2,3-, 1,2,3- und 3,3,3-Trifluorprop-1-oxy, 1- und 2-Fluorprop-2-oxy, 1,1- und 1,3-Difluorprop-2-oxy, 1,1,1-Trifluorprop-2-oxy und dergleichen.

Fluoriertes Ethoxy steht im Rahmen der vorliegenden Erfindung für Ethoxy, in welchem 1, 2, 3, 4 oder 5 der Wasserstoffatome durch Fluoratome substituiert sind. Beispiele sind 1-Fluorethoxy, 2-Fluorethoxy, 1,1-Difluorethoxy, 1,2-Difluorethoxy, 2,2-Difluorethoxy, 1,1,2-Trifluorethoxy, 1,2,2-Trifluorethoxy, 2,2,2-Trifluorethoxy, 1,1,2,2-Tetrafluorethoxy, 1,2,2,2-Tetrafluorethoxy und 1,1,2,2,2-Pentafluorethoxy.

Halogen steht im Rahmen der vorliegenden Erfindung für Fluor, Chlor, Brom oder Iod.

Die erfindungsgemäßen Verbindungen der Formel I, ihre pharmakologisch verträglichen Salze und ihre Prodrugs können auch in Form von Solvaten oder Hydraten vorliegen. Unter Solvaten versteht man im Rahmen der vorliegenden Erfindung kristalline Formen der Verbindungen I bzw. ihrer pharmazeutisch verträglichen Salze oder Prodrugs davon, die im Kristallgitter Lösungsmittelmoleküle eingebaut enthalten. Vorzugsweise sind die Lösungsmittelmoleküle in stöchiometrischen Verhältnissen eingebaut. Hydrate sind eine Spezialform der Solvate; das Lösungsmittel ist hier Wasser.

Die nachstehend gemachten Angaben zu geeigneten und bevorzugten Merkmalen der Erfindung, insbesondere zu den Variablen R¹, R², R³, R⁴, R⁵, R⁶, R⁷, R⁸, R⁹, X¹, X², X³, X⁴, a, b, m, n, o und p in der Verbindung I, aber auch zu den Merkmalen des erfindungsgemäßen Verfahrens und der erfindungsgemäßen Verwendung gelten sowohl für sich allein genommen als auch vorzugsweise in jeder möglichen Kombination miteinander.

Die Verbindungen I werden bevorzugt in Form der freien Base (d.h. gemäß der Strukturformel I) oder in Form ihrer Säureadditionssalze bereitgestellt.

In einer bevorzugten Ausführungsform stehen R¹ und R² unabhängig voneinander für Wasserstoff, C₁-C₃-Alkoxy oder C₁-C₃-Fluoralkoxy. Dabei steht in der Definition der Reste R¹ und R² C₁-C₃-Alkoxy vorzugsweise für Ethoxy oder Methoxy und speziell für Methoxy. C₁-C₃-Fluoralkoxy steht vorzugsweise für C₁-C₂-Fluoralkoxy, d.h. für Fluormethoxy, Difluormethoxy, Trifluormethoxy, 1- und 2-Fluorethoxy, 1,1-, 1,2- und 2,2-Difluorethoxy, 1,1,2-, 1,2,2 und 2,2,2-Trifluorethoxy, 1,1,2,2-Tetrafluorethoxy, 1,2,2,2-Tetrafluorethoxy, Pentafluorethoxy, bevorzugt für Fluormethoxy, Difluormethoxy, Trifluormethoxy, 2,2-Difluorethoxy und 2,2,2-Trifluorethoxy, und speziell für Trifluormethoxy.

In einer bevorzugten Ausführungsform steht R¹ für Wasserstoff, Methoxy, Ethoxy, Fluormethoxy, Difluormethoxy oder Trifluormethoxy, besonders bevorzugt für Wasserstoff, Methoxy oder Trifluormethoxy, stärker bevorzugt für Wasserstoff oder Methoxy und speziell für Methoxy.

In einer bevorzugten Ausführungsform steht R² für Wasserstoff oder Methoxy und speziell für Methoxy.

In einer besonders bevorzugten Ausführungsform steht wenigstens einer der Reste R¹ und R² für Methoxy. Speziell stehen R¹ und R² für Methoxy.

In einer bevorzugten Ausführungsform steht R³ für Wasserstoff, Methyl, Ethyl, n-Propyl oder Isopropyl, besonders bevorzugt für Wasserstoff, Methyl oder Ethyl, insbesondere für Methyl oder Ethyl und speziell für Methyl.

In einer bevorzugten Ausführungsform steht R⁴ für Ethoxy und R⁵ steht für H. Dabei steht X⁴ für N oder CH und vorzugsweise für N.

In einer alternativ bevorzugten Ausführungsform steht R⁴ für Ethoxy und R⁵ steht für Methyl. Dabei steht X⁴ vorzugsweise für N.

In einer alternativ bevorzugten Ausführungsform steht R⁴ für Isopropoxy und R⁵ steht für H. Dabei steht X⁴ vorzugsweise für N.

In einer alternativ bevorzugten Ausführungsform steht R⁴ für fluoriertes Ethoxy, vorzugsweise für 2,2-Difluorethoxy oder 2,2,2-Trifluorethoxy und besonders bevorzugt für 2,2-Difluorethoxy, und R⁵ steht für H. Dabei steht X⁴ für N oder CH und speziell für CH.

Besonders bevorzugt steht X⁴ für N.

Besonders bevorzugt steht R⁴ für Ethoxy und R⁵ steht für H. Dabei steht X⁴ für N oder CH und vorzugsweise für N.

In einer bevorzugten Ausführungsform stehen R⁶ und R⁷nicht gleichzeitig für CN.

Vorzugsweise steht wenigstens einer der Reste R⁶ und R⁷ für Fluor. Besonders bevorzugt steht dabei für R⁷ Fluor und R⁶ steht für Fluor, Chlor, Brom oder CN, bevorzugt für Fluor, Chlor oder CN und besonders bevorzugt für Cl oder CN.

In einer bevorzugten Ausführungsform stehen R⁸ und R⁹ für Methyl oder Ethyl.

In einer bevorzugten Ausführungsform steht X¹ für NH.

In einer alternativ bevorzugten Ausführungsform steht X¹ für O.

In einer alternativ bevorzugten Ausführungsform steht X¹ für CH₂.

Besonders bevorzugt steht X¹ für NH oder O und insbesondere für NH.

In einer bevorzugten Ausführungsform steht eine der Variablen X², X³ für N und die andere für CH.

In einer besonders bevorzugten Ausführungsform steht dabei X² für N und X³ steht für CH.

In einer alternativ besonders bevorzugten Ausführungsform steht X² für CH und X³ steht für N.

In einer alternativ bevorzugten Ausführungsform stehen beide Variablen X², X³ für CH.

In einer bevorzugten Ausführungsform stehen a und b unabhängig voneinander für 0 oder 1 und insbesondere für 0.

Wenn a und/oder b ungleich 0 sind, versteht es sich von selbst, dass die Reste R⁸ und/oder R⁹ an eine der m, n, o oder p CH₂-Gruppen gebunden sind, wo sie jeweils ein Wasserstoffatom dieser CH₂-Gruppe ersetzen.

In einer bevorzugten Ausführungsform stehen m, n, o und p unabhängig voneinander für 1 oder 2.

Dementsprechend stehen vorzugsweise m und n für 1 oder m und n stehen für 2 oder m steht für 1 und n steht für 2 oder m steht für 2 und n steht für 1. Besonders bevorzugt stehen m und n für 2.

Dementsprechend stehen vorzugsweise o und p für 1 oder o und p stehen für 2 oder o steht für 1 und p steht für 2 oder o steht für 2 und p steht für 1. Besonders bevorzugt stehen o und p für 2.

In einer besonders bevorzugten Ausführungsform betrifft die vorliegende Erfindung Verbindungen der Formel I.A 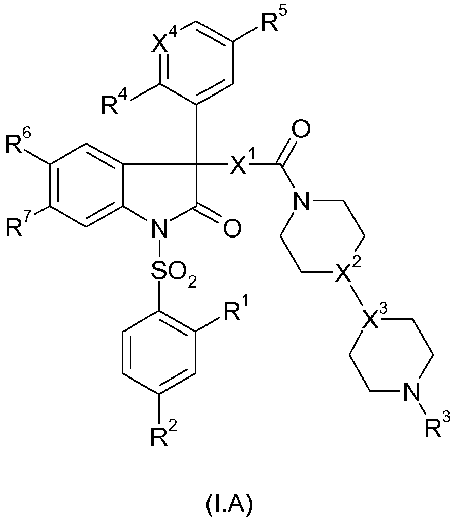 worin R¹, R², R³, R⁴, R⁵, R⁶, R⁷, X¹, X², X³ und X⁴ die zuvor angegebenen allgemeinen oder insbesondere die zuvor angegebenen bevorzugten Bedeutungen haben.

Ein bevorzugter Gegenstand der Erfindung sind Verbindungen der Formel I.A, worin
- R¹: Wasserstoff, Methoxy oder Trifluormethoxy, vorzugsweise Wasserstoff oder Methoxy ist;
- R²: Wasserstoff oder Methoxy ist;
- R³: Wasserstoff, Methyl Ethyl, n-Propyl oder Isopropyl; vorzugsweise Wasserstoff, Methyl oder Ethyl, besonders bevorzugt Methyl oder Ethyl ist;
- R⁴: Ethoxy ist;

- R⁵: Wasserstoff ist;
- R⁶: Cl, F oder CN, vorzugsweise Cl oder CN ist;
- R⁷: F oder Cl, vorzugsweise F ist;
- X¹: NH, O oder CH₂ ist;
- X²: N oder CH ist;
- X³: N oder CH ist;
- X⁴: N ist
wobei X² und X³ nicht gleichzeitig für N stehen;
sowie die pharmazeutisch verträglichen Salze und Prodrugs davon.

Ein alternativ bevorzugter Gegenstand der Erfindung sind Verbindungen der Formel I.A, worin
- R¹: Wasserstoff, Methoxy oder Trifluormethoxy, vorzugsweise Wasserstoff oder Methoxy ist;
- R²: Wasserstoff oder Methoxy ist;
- R³: Wasserstoff, Methyl Ethyl, n-Propyl oder Isopropyl; vorzugsweise Wasserstoff, Methyl oder Ethyl, besonders bevorzugt Methyl oder Ethyl ist;
- R⁴: 2,2-Difluorethoxy oder Ethoxy ist;
- R⁵: Wasserstoff ist;
- R⁶: Cl, F oder CN, vorzugsweise Cl oder CN ist;
- R⁷: F oder Cl, vorzugsweise F ist;
- X¹: NH, O oder CH₂ ist;
- X²: N oder CH ist;
- X³: N oder CH ist;
- X⁴: CH ist
wobei X² und X³ nicht gleichzeitig für N stehen;
sowie die pharmazeutisch verträglichen Salze und Prodrugs davon.

Ein besonders bevorzugter Gegenstand der Erfindung sind Verbindungen der Formel I.A, worin
- R¹: Wasserstoff oder Methoxy ist;
- R²: Wasserstoff oder Methoxy ist;
- R³: Methyl oder Ethyl ist;
- R⁴: Ethoxy ist;
- R⁵: Wasserstoff ist;
- R⁶: Cl, F oder CN, vorzugsweise Cl oder CN ist;
- R⁷: F oder Cl, vorzugsweise F ist;
- X¹: NH, O oder CH₂ ist;
- X²: N oder CH ist;
- X³: N oder CH ist;
- X⁴: N ist;
wobei X² und X³ nicht gleichzeitig für N stehen;
sowie die pharmazeutisch verträglichen Salze und Prodrugs davon.
Ein alternativ besonders bevorzugter Gegenstand der Erfindung sind Verbindungen der Formel I.A, worin
- R¹: Wasserstoff oder Methoxy ist;
- R²: Wasserstoff oder Methoxy ist;
- R³: Methyl oder Ethyl ist;
- R⁴: 2,2-Difluorethoxy oder Ethoxy ist;
- R⁵: Wasserstoff ist;
- R⁶: Cl, F oder CN, vorzugsweise Cl oder CN ist;
- R⁷: F oder Cl, vorzugsweise F ist;
- X¹: NH, O oder CH₂ ist;
- X²: N oder CH ist;
- X³: N oder CH ist;
- X⁴: CH ist;
wobei X² und X³ nicht gleichzeitig für N stehen;
sowie die pharmazeutisch verträglichen Salze und Prodrugs davon.

Ein stärker bevorzugter Gegenstand der Erfindung sind Verbindungen der Formel I.A, worin
- R¹: Methoxy oder H ist;
- R²: Methoxy ist;
- R³: Methyl oder Ethyl ist;
- R⁴: Ethoxy ist;
- R⁵: Wasserstoff ist;
- R⁶: Cl, F oder CN, vorzugsweise Cl oder CN ist;
- R⁷: F ist;
- X¹: NH, O oder CH₂ ist;
- X²: N oder CH ist;
- X³: N oder CH ist;
- X⁴: N ist;
wobei X² und X³ nicht gleichzeitig für N stehen;
sowie die pharmazeutisch verträglichen Salze und Prodrugs davon.

Ein alternativ stärker bevorzugter Gegenstand der Erfindung sind Verbindungen der Formel I.A, worin
- R¹: Methoxy oder H ist;
- R²: Methoxy ist;
- R³: Methyl oder Ethyl ist;
- R⁴: Ethoxy ist;
- R⁵: Wasserstoff ist;
- R⁶: Cl, F oder CN, vorzugsweise Cl oder CN ist;
- R⁷: F ist;
- X¹: NH, O oder CH₂ ist;
- X²: N oder CH ist;
- X³: N oder CH ist;
- X⁴: CH ist;
wobei X² und X³ nicht gleichzeitig für N stehen;
sowie die pharmazeutisch verträglichen Salze und Prodrugs davon.

Ein noch stärker bevorzugter Gegenstand der Erfindung sind Verbindungen der Formel I.A, worin
- R¹: Methoxy ist;
- R²: Methoxy ist;
- R³: Methyl oder Ethyl ist;
- R⁴: Ethoxy ist;
- R⁵: Wasserstoff ist;
- R⁶: Cl oder CN ist;
- R⁷: F ist;
- X¹: NH ist;
- X²: N ist;
- X³: CH ist;
- X⁴: N ist;
sowie die pharmazeutisch verträglichen Salze und Prodrugs davon.

Ein alternativ noch stärker bevorzugter Gegenstand der Erfindung sind Verbindungen der Formel I.A, worin
- R¹: Methoxy ist;
- R²: Methoxy ist;
- R³: Methyl oder Ethyl ist;
- R⁴: Ethoxy ist;
- R⁵: Wasserstoff ist;
- R⁶: Cl oder CN ist;
- R⁷: F ist;
- X¹: NH ist;
- X²: CH ist;
- X³: N ist;
- X⁴: N ist;
sowie die pharmazeutisch verträglichen Salze und Prodrugs davon.

Ein alternativ noch stärker bevorzugter Gegenstand der Erfindung sind Verbindungen der Formel I.A, worin
- R¹: Methoxy ist;
- R²: Methoxy ist;
- R³: Methyl oder Ethyl ist;
- R⁴: Ethoxy ist;
- R⁵: Wasserstoff ist;
- R⁶: Cl oder CN ist;
- R⁷: F ist;
- X¹: CH₂ ist;
- X²: N ist;
- X³: CH ist;
- X⁴: N ist;
sowie die pharmazeutisch verträglichen Salze und Prodrugs davon.

Ein alternativ noch stärker bevorzugter Gegenstand der Erfindung sind Verbindungen der Formel I.A, worin
- R¹: Methoxy ist;
- R²: Methoxy ist;
- R³: Methyl oder Ethyl ist;
- R⁴: Ethoxy ist;
- R⁵: Wasserstoff ist;
- R⁶: Cl oder CN ist;
- R⁷: F ist;
- X¹: CH₂ ist;
- X²: CH ist;
- X³: N ist;
- X⁴: N ist;
sowie die pharmazeutisch verträglichen Salze und Prodrugs davon.

Ein alternativ noch stärker bevorzugter Gegenstand der Erfindung sind Verbindungen der Formel I.A, worin
- R¹: Methoxy oder Wasserstoff ist;
- R²: Methoxy ist;
- R³: Methyl oder Ethyl ist;
- R⁴: Ethoxy ist;
- R⁵: Wasserstoff ist;
- R⁶: Cl oder CN ist;
- R⁷: F ist;
- X¹: O ist;
- X²: N ist;
- X³: CH ist;
- X⁴: N ist;
sowie die pharmazeutisch verträglichen Salze und Prodrugs davon.

Ein alternativ noch stärker bevorzugter Gegenstand der Erfindung sind Verbindungen der Formel I.A, worin
- R¹: Methoxy oder Wasserstoff ist;
- R²: Methoxy ist;
- R³: Methyl oder Ethyl ist;
- R⁴: Ethoxy ist;
- R⁵: Wasserstoff ist;
- R⁶: Cl oder CN ist;
- R⁷: F ist;
- X¹: O ist;
- X²: CH ist;
- X³: N ist;
- X⁴: N ist;
sowie die pharmazeutisch verträglichen Salze und Prodrugs davon.

Ein alternativ noch stärker bevorzugter Gegenstand der Erfindung sind Verbindungen der Formel I.A, worin
- R¹: Methoxy oder Wasserstoff ist;
- R²: Methoxy ist;
- R³: Methyl oder Ethyl ist;
- R⁴: Ethoxy ist;
- R⁵: Wasserstoff ist;
- R⁶: Cl oder CN ist;
- R⁷: F ist;
- X¹: NH ist;
- X²: N ist;
- X³: CH ist;
- X⁴: CH ist;
sowie die pharmazeutisch verträglichen Salze und Prodrugs davon.

Ein alternativ noch stärker bevorzugter Gegenstand der Erfindung sind Verbindungen der Formel I.A, worin
- R¹: Methoxy oder Wasserstoff ist;
- R²: Methoxy ist;
- R³: Methyl oder Ethyl ist;
- R⁴: Ethoxy ist;
- R⁵: Wasserstoff ist;
- R⁶: Cl oder CN ist;
- R⁷: F ist;
- X¹: NH ist;
- X²: CH ist;
- X³: N ist;
- X⁴: CH ist;
sowie die pharmazeutisch verträglichen Salze und Prodrugs davon.

Insbesondere sind Verbindungen der Formel I.AGegenstand der Erfindung, worin
- R¹: Methoxy ist;
- R²: Methoxy ist;
- R³: Methyl oder Ethyl ist;
- R⁴: Ethoxy ist;
- R⁵: Wasserstoff ist;
- R⁶: Cl ist;
- R⁷: F ist;
- X¹: NH ist;
- X²: N ist;
- X³: CH ist;
- X⁴: N ist;
sowie die pharmazeutisch verträglichen Salze und Prodrugs davon.

Insbesondere sind auch Verbindungen der Formel I.AGegenstand der Erfindung, worin
- R¹: Methoxy ist;
- R²: Methoxy ist;
- R³: Methyl oder Ethyl ist;
- R⁴: Ethoxy ist;
- R⁵: Wasserstoff ist;
- R⁶: CI ist;
- R⁷: F ist;
- X¹: NH ist;
- X²: CH ist;
- X³: N ist;
- X⁴: N ist;
sowie die pharmazeutisch verträglichen Salze und Prodrugs davon.

Insbesondere sind auch Verbindungen der Formel I.AGegenstand der Erfindung, worin
- R¹: Methoxy ist;
- R²: Methoxy ist;
- R³: Methyl oder Ethyl ist;
- R⁴: Ethoxy ist;
- R⁵: Wasserstoff ist;
- R⁶: Cl ist;
- R⁷: F ist;
- X¹: CH₂ ist;
- X²: N ist;
- X³: CH ist;
- X⁴: N ist;
sowie die pharmazeutisch verträglichen Salze und Prodrugs davon.

Insbesondere sind auch Verbindungen der Formel I.AGegenstand der Erfindung, worin
- R¹: Methoxy ist;
- R²: Methoxy ist;
- R³: Methyl oder Ethyl ist;
- R⁴: Ethoxy ist;
- R⁵: Wasserstoff ist;
- R⁶: CI ist;
- R⁷: F ist;
- X¹: CH₂ ist;
- X²: CH ist;
- X³: N ist;
- X⁴: N ist;
sowie die pharmazeutisch verträglichen Salze und Prodrugs davon.

Insbesondere sind auch Verbindungen der Formel I.AGegenstand der Erfindung, worin
- R¹: Methoxy ist;
- R²: Methoxy ist;
- R³: Methyl oder Ethyl ist;
- R⁴: Ethoxy ist;
- R⁵: Wasserstoff ist;
- R⁶: CN ist;
- R⁷: F ist;
- X¹: NH ist;
- X²: N ist;
- X³: CH ist;
- X⁴: N ist;
sowie die pharmazeutisch verträglichen Salze und Prodrugs davon.

Insbesondere sind auch Verbindungen der Formel I.AGegenstand der Erfindung, worin
- R¹: Methoxy ist;
- R²: Methoxy ist;
- R³: Methyl oder Ethyl ist;
- R⁴: Ethoxy ist;
- R⁵: Wasserstoff ist;
- R⁶: CN ist;
- R⁷: F ist;
- X¹: NH ist;
- X²: CH ist;
- X³: N ist;
- X⁴: N ist;
sowie die pharmazeutisch verträglichen Salze und Prodrugs davon.

Insbesondere sind auch Verbindungen der Formel I.AGegenstand der Erfindung, worin
- R¹: Methoxy oder Wasserstoff ist;
- R²: Methoxy ist;
- R³: Methyl oder Ethyl ist;
- R⁴: Ethoxy ist;
- R⁵: Wasserstoff ist;
- R⁶: CN ist;
- R⁷: F ist;
- X¹: O ist;
- X²: N ist;
- X³: CH ist;
- X⁴: N ist;
sowie die pharmazeutisch verträglichen Salze und Prodrugs davon.

Insbesondere sind auch Verbindungen der Formel I.AGegenstand der Erfindung, worin
- R¹: Methoxy oder Wasserstoff ist;
- R²: Methoxy ist;
- R³: Methyl oder Ethyl ist;
- R⁴: Ethoxy ist;
- R⁵: Wasserstoff ist;
- R⁶: CN ist;
- R⁷: F ist;
- X¹: O ist;
- X²: CH ist;
- X³: N ist;
- X⁴: N ist;
sowie die pharmazeutisch verträglichen Salze und Prodrugs davon.

Beispiele für bevorzugte Ausführungsform der vorliegenden Erfindung sind Verbindungen der Formel I.1 bis I.60 sowie die pharmazeutisch verträglichen Salze und Prodrugs davon, worin die Reste X², X³, R¹, R² und R³ jeweils die in der folgenden Tabelle 1 pro Zeile genannten Bedeutungen annehmen.

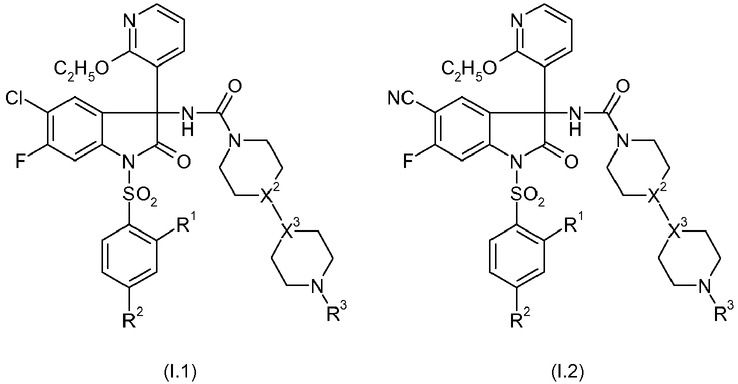

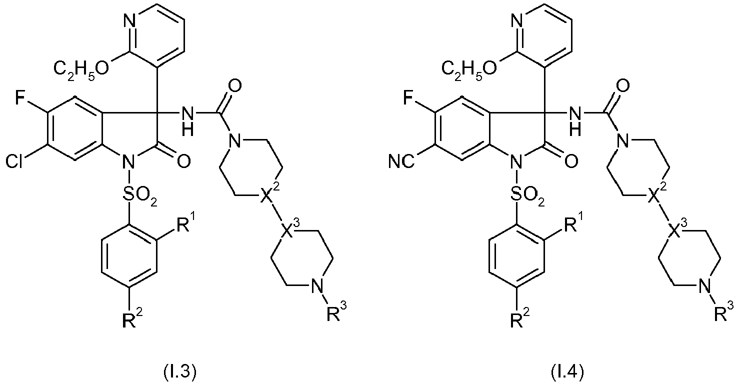

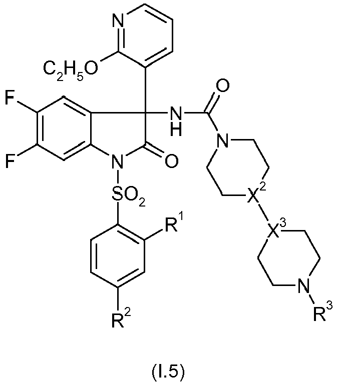

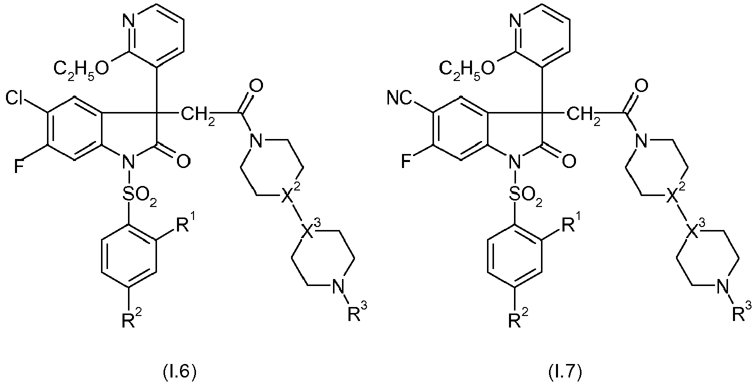

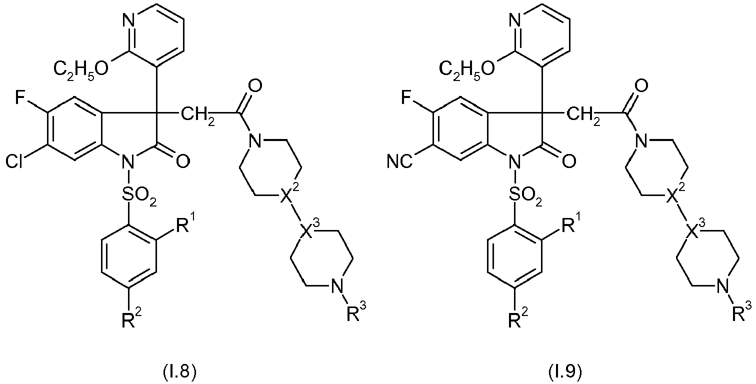

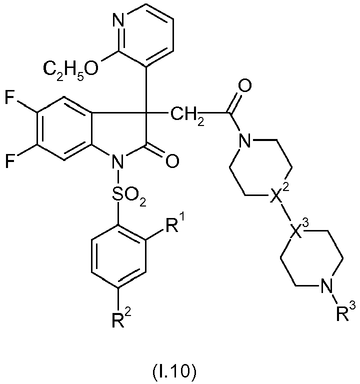

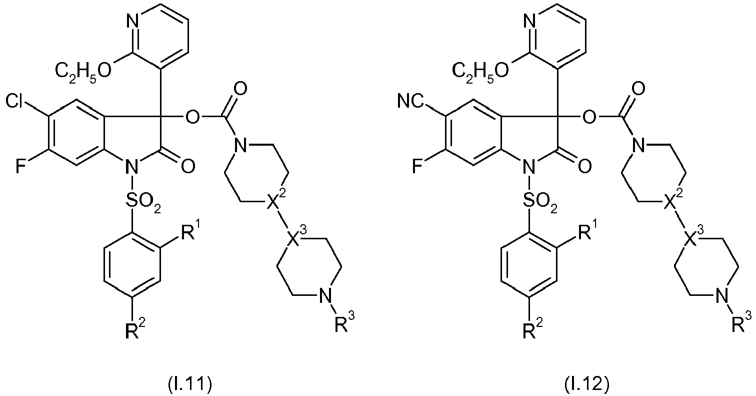

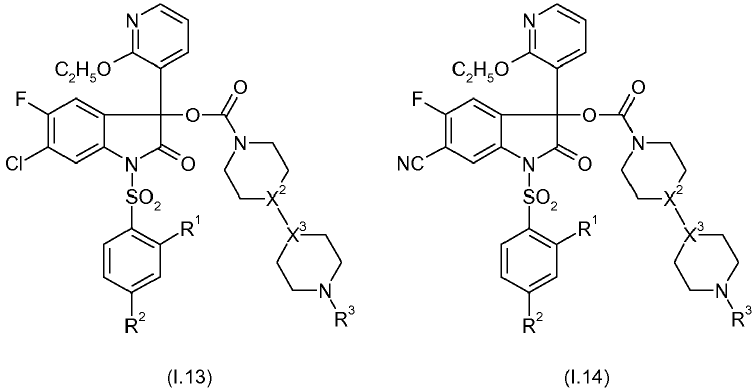

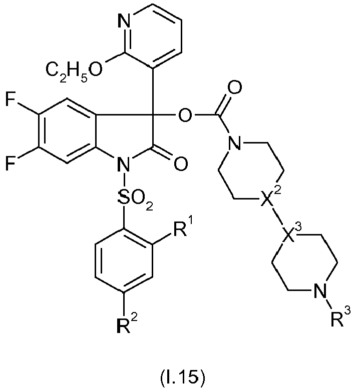

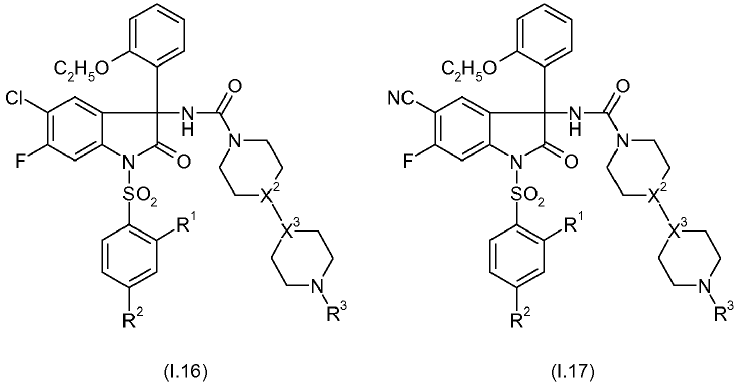

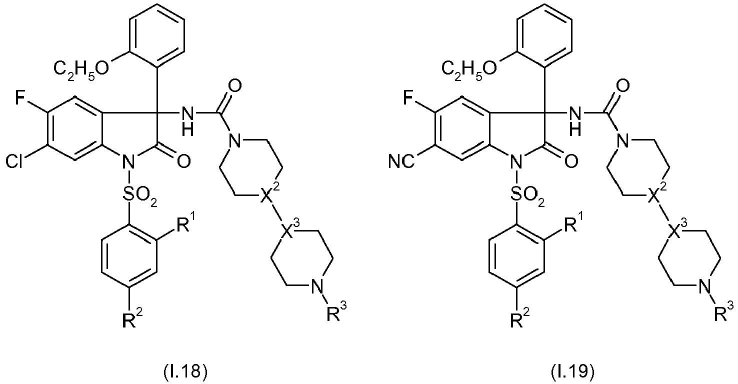

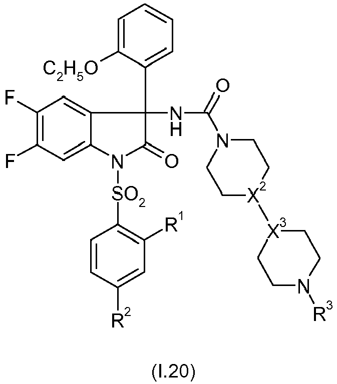

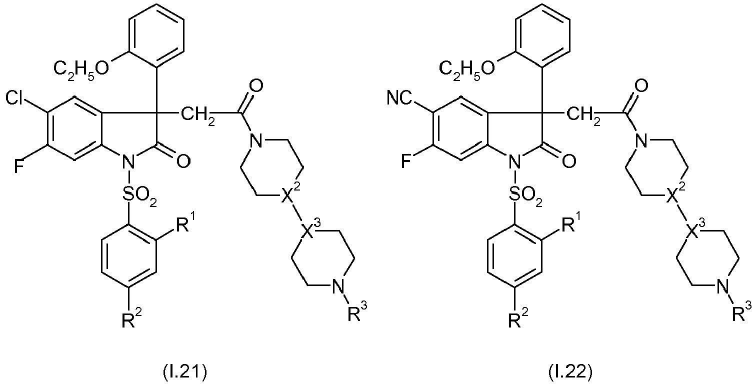

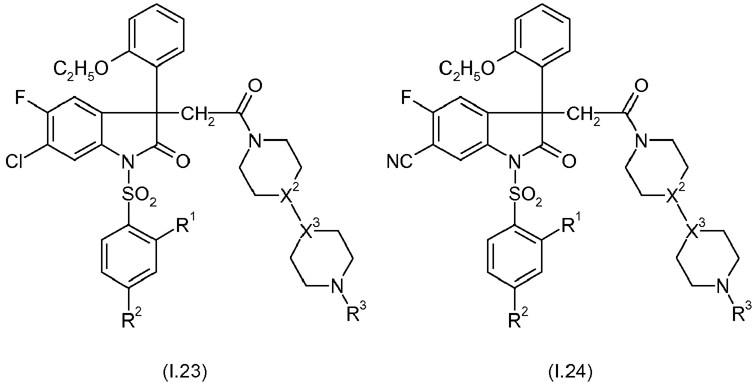

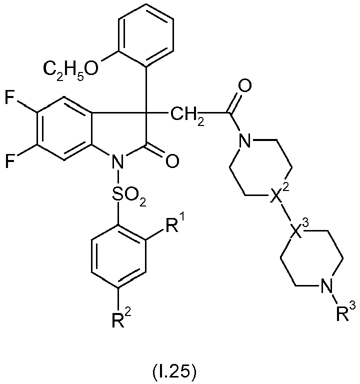

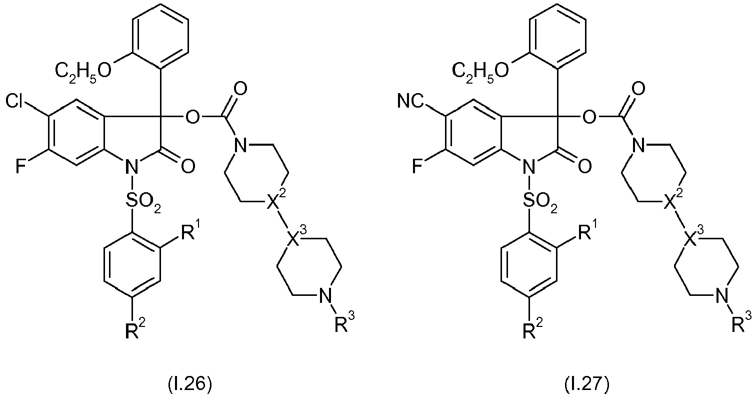

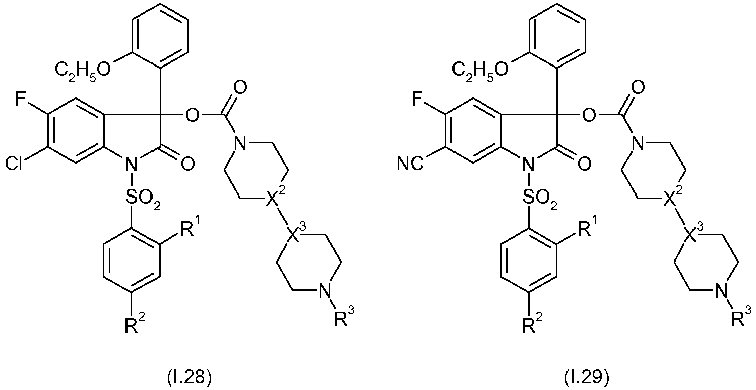

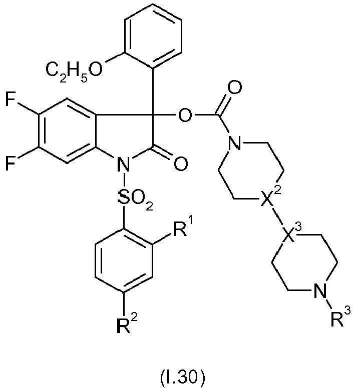

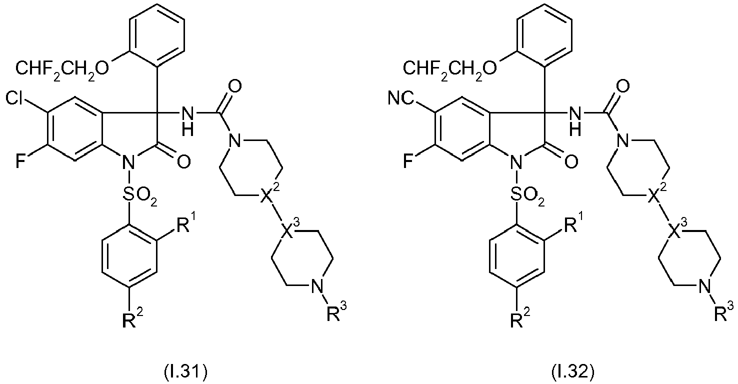

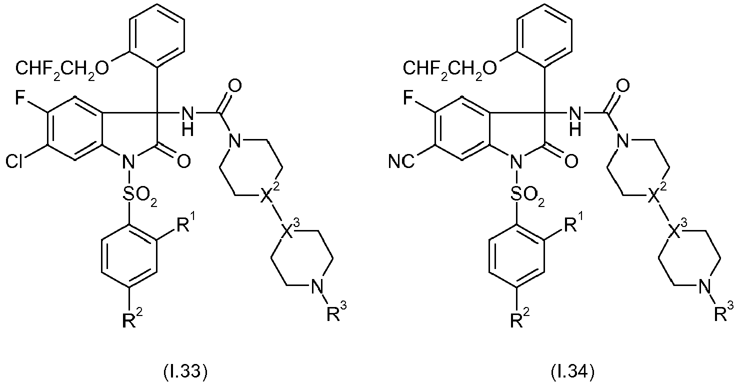

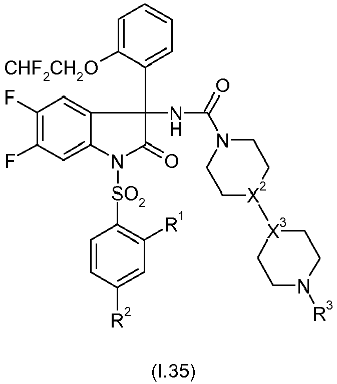

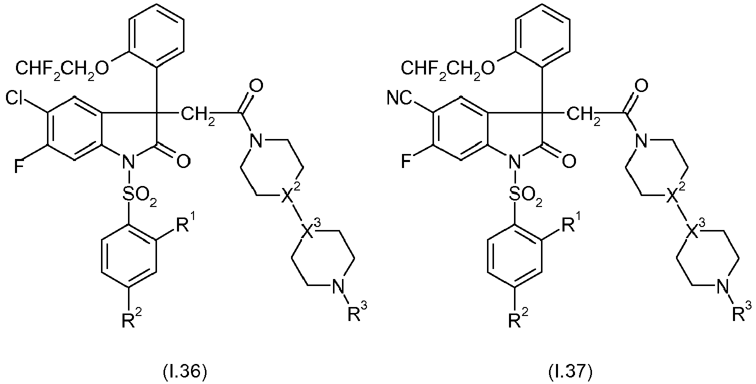

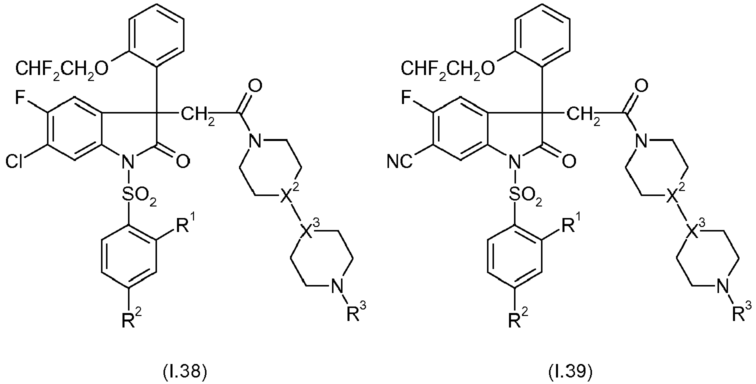

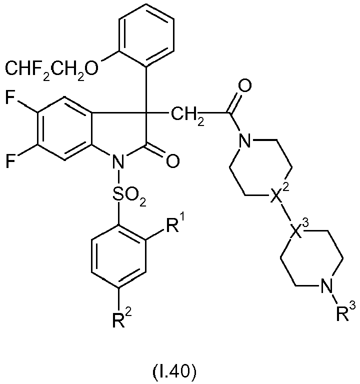

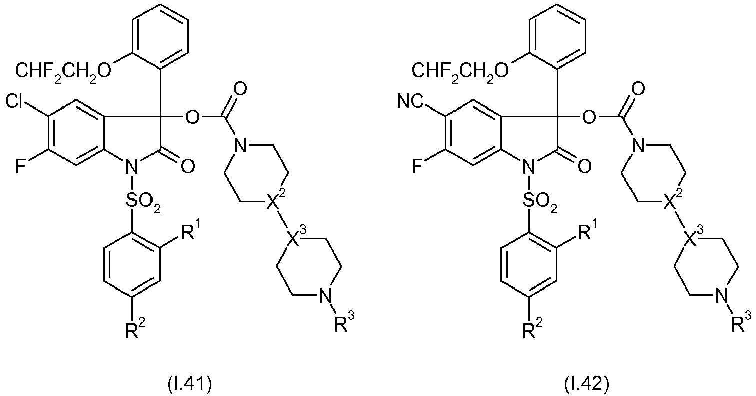

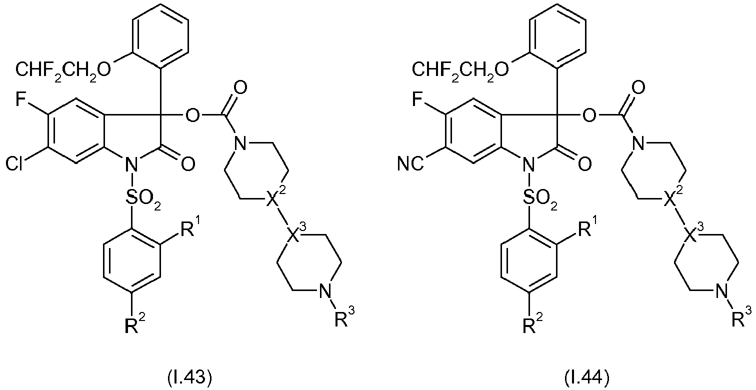

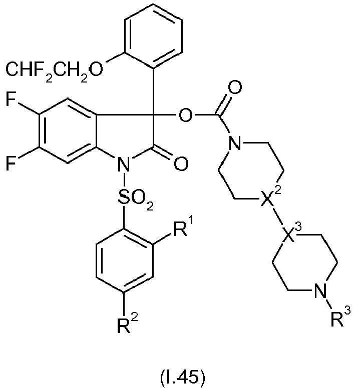

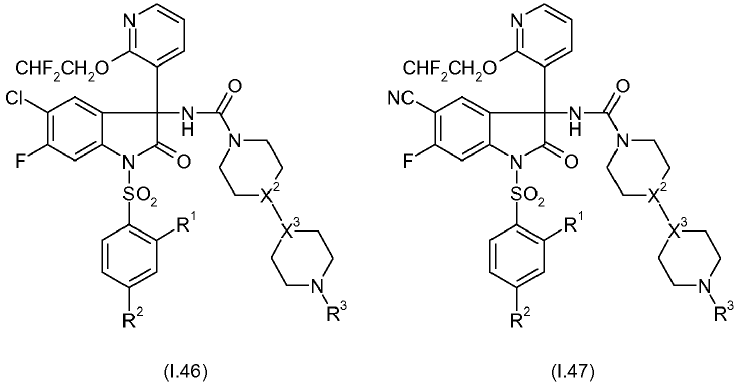

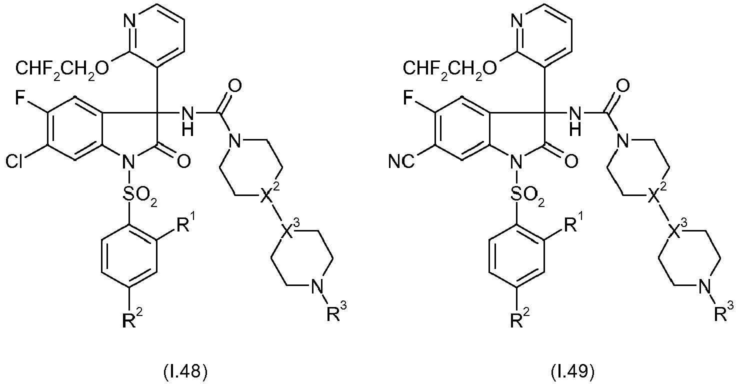

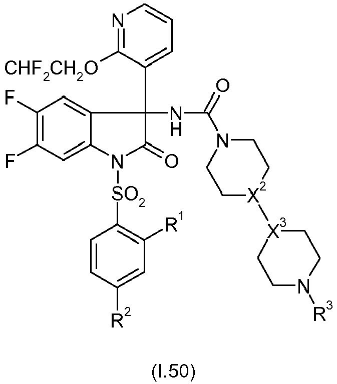

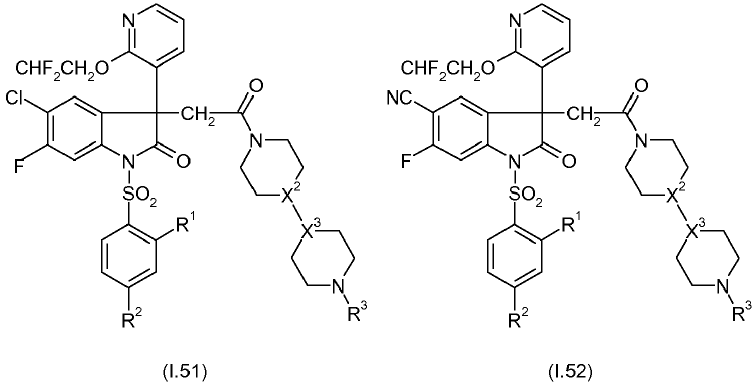

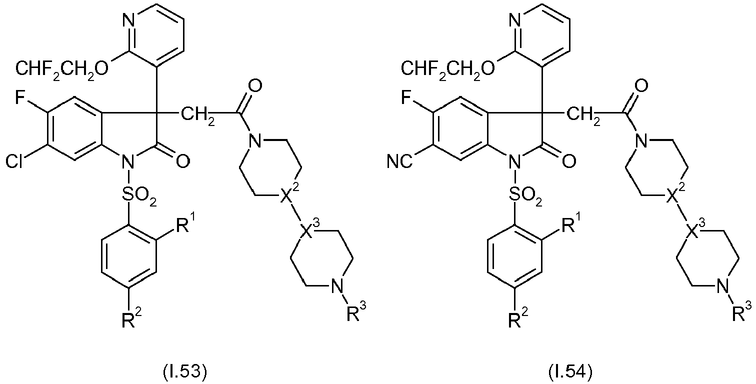

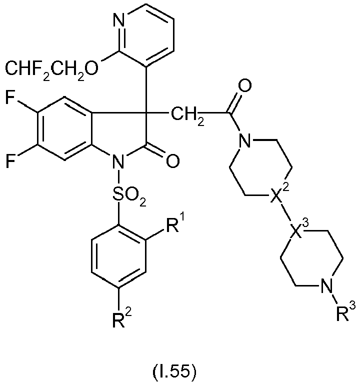

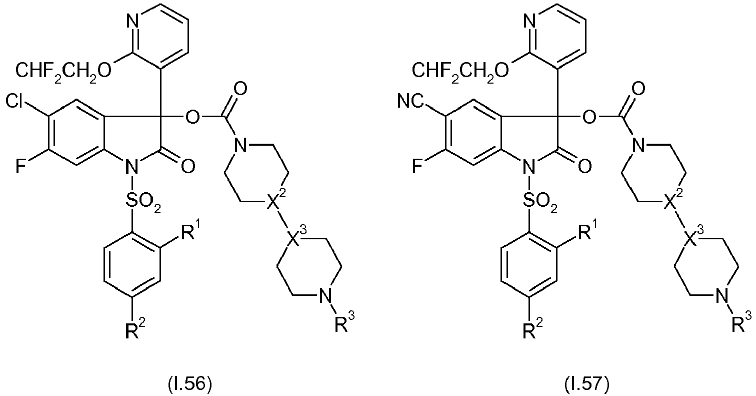

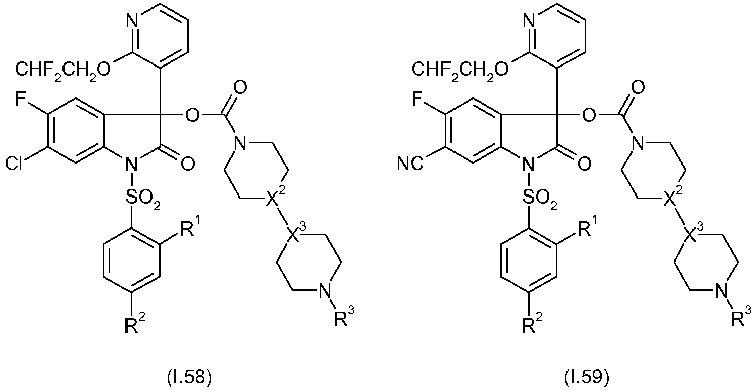

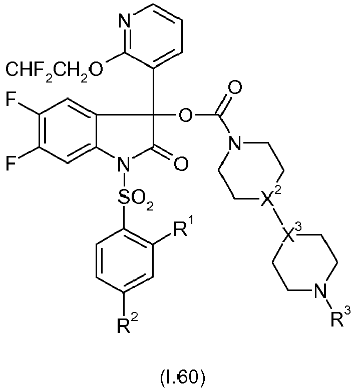

**Tabelle 1:**

| **Beispiel Nr.** | **X²** | **X³** | **R¹** | **R²** | **R³** |
|---|---|---|---|---|---|
| A-1. | N | CH | Methoxy | Methoxy | Methyl |
| A-2. | N | CH | Methoxy | H | Methyl |
| A-3. | N | CH | Ethoxy | H | Methyl |
| A-4. | N | CH | H | H | Methyl |
| A-5. | N | CH | H | Methoxy | Methyl |
| A-6. | N | CH | Ethoxy | Methoxy | Methyl |
| A-7. | N | CH | Methoxy | Methoxy | Ethyl |
| A-8. | N | CH | Methoxy | H | Ethyl |
| A-9. | N | CH | Ethoxy | H | Ethyl |
| A-10. | N | CH | H | H | Ethyl |
| A-11. | N | CH | H | Methoxy | Ethyl |
| A-12. | N | CH | Ethoxy | Methoxy | Ethyl |
| A-13. | N | CH | Methoxy | Methoxy | n-Propyl |
| A-14. | N | CH | Methoxy | H | n-Propyl |
| A-15. | N | CH | Ethoxy | H | n-Propyl |
| A-16. | N | CH | H | H | n-Propyl |
| A-17. | N | CH | H | Methoxy | n-Propyl |
| A-18. | N | CH | Ethoxy | Methoxy | n-Propyl |
| A-19. | N | CH | Methoxy | Methoxy | Isopropyl |
| A-20. | N | CH | Methoxy | H | Isopropyl |
| A-21. | N | CH | Ethoxy | H | Isopropyl |
| A-22. | N | CH | H | H | Isopropyl |
| A-23. | N | CH | H | Methoxy | Isopropyl |
| A-24. | N | CH | Ethoxy | Methoxy | Isopropyl |
| A-25. | N | CH | Methoxy | Methoxy | H |
| A-26. | N | CH | Methoxy | H | H |
| A-27. | N | CH | Ethoxy | H | H |
| A-28. | N | CH | H | H | H |
| A-29. | N | CH | H | Methoxy | H |
| A-30. | N | CH | Ethoxy | Methoxy | H |
| A-31. | CH | N | Methoxy | Methoxy | Methyl |
| A-32. | CH | N | Methoxy | H | Methyl |
| A-33. | CH | N | Ethoxy | H | Methyl |
| A-34. | CH | N | H | H | Methyl |
| A-35. | CH | N | H | Methoxy | Methyl |
| A-36. | CH | N | Ethoxy | Methoxy | Methyl |
| A-37. | CH | N | Methoxy | Methoxy | Ethyl |
| A-38. | CH | N | Methoxy | H | Ethyl |
| A-39. | CH | N | Ethoxy | H | Ethyl |
| A-40. | CH | N | H | H | Ethyl |
| A-41. | CH | N | H | Methoxy | Ethyl |
| A-42. | CH | N | Ethoxy | Methoxy | Ethyl |
| A-43. | CH | N | Methoxy | Methoxy | n-Propyl |
| A-44. | CH | N | Methoxy | H | n-Propyl |
| A-45. | CH | N | Ethoxy | H | n-Propyl |
| A-46. | CH | N | H | H | n-Propyl |
| A-47. | CH | N | H | Methoxy | n-Propyl |
| A-48. | CH | N | Ethoxy | Methoxy | n-Propyl |
| A-49. | CH | N | Methoxy | Methoxy | Isopropyl |
| A-50. | CH | N | Methoxy | H | Isopropyl |
| A-51. | CH | N | Ethoxy | H | Isopropyl |
| A-52. | CH | N | H | H | Isopropyl |
| A-53. | CH | N | H | Methoxy | Isopropyl |
| A-54. | CH | N | Ethoxy | Methoxy | Isopropyl |
| A-55. | CH | N | Methoxy | Methoxy | H |
| A-56. | CH | N | Methoxy | H | H |
| A-57. | CH | N | Ethoxy | H | H |
| A-58. | CH | N | H | H | H |
| A-59. | CH | N | H | Methoxy | H |
| A-60. | CH | N | Ethoxy | Methoxy | H |
| A-61. | CH | CH | Methoxy | Methoxy | Methyl |
| A-62. | CH | CH | Methoxy | H | Methyl |
| A-63. | CH | CH | Ethoxy | H | Methyl |
| A-64. | CH | CH | H | H | Methyl |
| A-65. | CH | CH | H | Methoxy | Methyl |
| A-66. | CH | CH | Ethoxy | Methoxy | Methyl |
| A-67. | CH | CH | Methoxy | Methoxy | Ethyl |
| A-68. | CH | CH | Methoxy | H | Ethyl |
| A-69. | CH | CH | Ethoxy | H | Ethyl |
| A-70. | CH | CH | H | H | Ethyl |
| A-71. | CH | CH | H | Methoxy | Ethyl |
| A-72. | CH | CH | Ethoxy | Methoxy | Ethyl |
| A-73. | CH | CH | Methoxy | Methoxy | n-Propyl |
| A-74. | CH | CH | Methoxy | H | n-Propyl |
| A-75. | CH | CH | Ethoxy | H | n-Propyl |
| A-76. | CH | CH | H | H | n-Propyl |
| A-77. | CH | CH | H | Methoxy | n-Propyl |
| A-78. | CH | CH | Ethoxy | Methoxy | n-Propyl |
| A-79. | CH | CH | Methoxy | Methoxy | Isopropyl |
| A-80. | CH | CH | Methoxy | H | Isopropyl |
| A-81. | CH | CH | Ethoxy | H | Isopropyl |
| A-82. | CH | CH | H | H | Isopropyl |
| A-83. | CH | CH | H | Methoxy | Isopropyl |
| A-84. | CH | CH | Ethoxy | Methoxy | Isopropyl |
| A-85. | CH | CH | Methoxy | Methoxy | H |
| A-86. | CH | CH | Methoxy | H | H |
| A-87. | CH | CH | Ethoxy | H | H |
| A-88. | CH | CH | H | H | H |
| A-89. | CH | CH | H | Methoxy | H |
| A-90. | CH | CH | Ethoxy | Methoxy | H |

Unter den oben genannten Verbindungen I.1 bis 1.60 sind Verbindungen der Formeln I.1, I.2, I.5, I.6, I.7, I.10, I.11, I.12, I.15, I.16, I.17, I.20, I.21, I.22, I.25, I.26, I.27, I.30, I.31, I.32, I.35, I.36, I.37, I.40, I.41, I.42, I.45, I.46, I.47, I.50, I.51, I.52, I.55, I.56, I.57 und I.60, worin die Reste X², X³, R¹, R² und R³ jeweils die in der Tabelle 1 pro Zeile genannten Bedeutungen annehmen bevorzugt. Hierunter bevorzugt sind wiederum die Verbindungen der Formeln I.1, I.2, I.6, I.7, I.11, I.12, I.16, I.17, I.21, I.22, I.26, I.27, I.31, I.32, I.36, I.37, I.41, I.42, I.46, I.47, I.51, I.52, I.56 und I.57, worin die Reste X², X³, R¹ R² und R³ jeweils die in der Tabelle 1 pro Zeile genannten Bedeutungen annehmen. Stärker bevorzugt sind hierunter die Verbindungen der Formeln I.1, I.2, I.6, I.7, I.11, I.12, I.16, I.17, I.21, I.22, I.26 und I.27, worin die Reste X², X³, R¹, R² und R³ jeweils die in der Tabelle 1 pro Zeile genannten Bedeutungen annehmen. Besonders bevorzugt sind hierunter die Verbindungen der Formeln I.1, I.2, I.6, I.7, I.11 und I.12, worin die Reste X², X³, R¹, R² und R³ jeweils die in der Tabelle 1 pro Zeile genannten Bedeutungen annehmen. Ganz besonders bevorzugt sind hierunter die Verbindungen der Formeln I.1 und I.2, worin die Reste X², X³, R¹, R² und R³ jeweils die in der Tabelle 1 pro Zeile genannten Bedeutungen annehmen.

Die erfindungsgemäßen Verbindungen I weisen in der 3-Position des 2-Oxindolrings ein Chiralitätszentrum auf. Die erfindungsgemäßen Verbindungen können daher als ein 1:1-Gemisch von Enantiomeren (Racemat) oder als ein nicht-racemisches Gemisch von Enantiomeren, in dem eines der beiden Enantiomere, entweder das die Schwingungsebene von linear polarisiertem Licht nach links drehende (d.h. minusdrehende) Enantiomer (im Folgenden (-)-Enantiomer) oder das die Schwingungsebene von linear polarisiertem Licht nach rechts drehende (d.h. plusdrehende) Enantiomer (im Folgenden (+)-Enantiomer), angereichert ist, oder als im Wesentlichen enantiomerenreine Verbindungen, also als im Wesentlichen enantiomerenreines (-)-Enantiomer oder (+)-Enantiomer, vorliegen. Da bei den erfindungsgemäßen Verbindungen ein einziges Asymmetriezentrum und keine Chiralitätsachse/-ebene existiert, kann man ein nicht-racemisches Gemisch auch als ein Gemisch von Enantiomeren definieren, in welchem entweder das R- oder das S-Enantiomer überwiegt. Im Wesentlichen enantiomerenreine Verbindungen können dementsprechend auch als im Wesentlichen enantiomerenreines R-Enantiomer bzw. im Wesentlichen enantiomerenreines S-Enantiomer definiert werden.

Unter "im Wesentlichen enantiomerenreinen Verbindungen" versteht man im Rahmen der vorliegenden Erfindung solche Verbindungen, die einen Enantiomerenüberschuss (enantiomeric excess, ee; % ee = (R-S)/(R+S) x 100 bzw. (S-R)/(S+R) x 100) von wenigstens 80 % ee, vorzugsweise wenigsten 85 % ee, stärker bevorzugt wenigstens 90 % ee, noch stärker bevorzugt wenigstens 95 % ee und insbesondere wenigstens 98 % ee aufweisen.

In einer Ausführungsform der Erfindung liegen die erfindungsgemäßen Verbindungen als im Wesentlichen enantiomerenreine Verbindungen vor. Besonders bevorzugt sind Verbindungen, die einen Enantiomerenüberschuss von wenigstens 85 % ee, stärker bevorzugt von wenigstens 90 % ee, noch stärker bevorzugt von wenigstens 95 % ee und insbesondere von wenigstens 98 % ee aufweisen.

Gegenstand der Erfindung sind somit sowohl die reinen Enantiomere als auch deren Gemische, z.B. Gemische, in denen ein Enantiomer in angereichter Form vorliegt, aber auch die Racemate. Gegenstand der Erfindung sind auch die pharmazeutisch verträglichen Salze und die Prodrugs der reinen Enantiomere von Verbindungen I sowie die racemischen und nicht-racemischen Enantiomerengemische in Form der pharmazeutisch verträglichen Salze und der Prodrugs von Verbindungen I.

Die im Rahmen der vorliegenden Erfindung gemachten Angaben zur Drehrichtung des polarisierten Lichts beziehen sich vorzugsweise auf Vorzeichen [(+) oder (-)] wie sie in Chloroform als Lösungsmittel oder in Chloroform-haltigen Lösungsmittelgemischen, insbesondere in Chloroform, ermittelt werden.

Im Folgenden werden beispielhafte Synthesewege zur Herstellung der erfindungsgemäßen Oxindol-Derivate beschrieben.

Die Herstellung der erfindungsgemäßen Verbindungen kann unter Verwendung der in WO 2005/030755 und WO 2006/005609 beschriebenen Vorschriften zur Synthese analoger Verbindungen erfolgen und ist beispielhaft in den Syntheseschemata 1 bis 3 skizziert. In diesen Syntheseschemata besitzen die Variablen die gleichen Bedeutungen wie in der Formel I.

Die 3-Hydroxy-1,3-dihydroindol-2-one IV können durch Addition von metallierten Benzolen oder Heterocyclen III an die 3-Ketogruppe der Isatine II erhalten werden. Die metallierten Benzole oder Heterocyclen, wie beispielsweise die entsprechenden Grignard- (Mg) oder Organyllithium-Verbindung, können in üblicher Weise aus Halogen- oder Kohlenwasserstoffverbindungen erhalten werden. Beispielhafte Vorschriften sind im Houben-Weyl, Methoden der Organischen Chemie, Bd. 13, 1-2, Kap. Mg- bzw. Li-Verbindungen, enthalten. Die Isatine II sind entweder kommerziell erhältlich oder wurden in Analogie zu in der Literatur beschriebenen Methoden hergestellt (Advances in Heterocyclic Chemistry, A.R. Katritzky and A.J. Boulton, Academic Press, New York, 1975, 18, 2-58; J. Brazil. Chem. Soc. 12, 273-324, 2001).

Die 3-Hydroxyoxindole IV, die im 6-Ring-Aromaten beispielsweise in der 5- oder 6-Position, d.h. in der Position der Reste R⁶ oder R⁷ ein Iod enthalten, können mit KCN oder Zn(CN)₂ unter Pd(0)-Katalyse in Lösungsmitteln wie Dimethylformamid oder Tetrahydrofuran, gegebenenfalls auch unter Zusatz von Basen wie K₂CO₃ oder anderen Carbonaten oder Aminen, bei höherer Temperatur in das analoge cyanhaltige 3-Hydroxyoxindol IV überführt werden. Als Pd(0)-Salze kann man zum Beispiel Übergangsmetallkomplexe nehmen, die in situ aus PdCl₂ oder PdOAc₂ durch Zugabe von Phosphinen wie Tris(ortho-tolyl)phosphin hergestellt werden. Ebenso können kommerzielle Palladium-Komplexe wie beispielsweise der Katalysator Tetrakis(triphenylphosphin)-palladium(0) und / oder Zusätze von Phosphin-Liganden eingesetzt werden.

Die 3-Hydroxyoxindole IV können in die Verbindungen V überführt werden, welche eine Fluchtgruppe LG' in 3-Stellung tragen, wobei die Fluchtgruppe LG' eine übliche Abgangsgruppe ist, wie zum Beispiel Chlorid oder Bromid. Das Zwischenprodukt V mit zum Beispiel LG' = Chlor kann durch Behandlung des Alkohols IV mit Thionylchlorid in Gegenwart einer Base, wie zum Beispiel Pyridin, in einem geeigneten Lösungsmittel, wie zum Beispiel Dichlormethan, hergestellt werden.

Die Verbindungen V können anschließend mit Aminen, wie beispielsweise Ammoniak, in einer Substitutionsreaktion zu den Aminen VI umgesetzt werden. Die Verbindungen VI können anschließend durch Behandlung mit Sulfonsäurechloriden VII nach Deprotonierung mit einer starken Base, wie zum Beispiel Kalium-*tert*-butylat oder Natriumhydrid in DMF, in das sulfonylierte Produkt VIII überführt werden. Die eingesetzten Sulfonsäurechloride VII können entweder käuflich erworben oder nach bekannten Verfahren (zum Beispiel J. Med. Chem. 40, 1149 (1997)) hergestellt werden.

Die Herstellung der erfindungsgemäßen Verbindungen der allgemeinen Formel I, welche eine Harnstoff-Gruppe in der 3-Stellung tragen, kann wie in WO 2005/030755 und WO 2006/005609 beschrieben und in Syntheseschema 1 gezeigt in einem zweistufigen Verfahren erfolgen: Zuerst werden die Verbindungen VIII mit Chlorameisensäurephenylester in Gegenwart einer Base, wie zum Beispiel Pyridin, zu dem entsprechenden Phenylcarbamat IX umgesetzt.

Die anschließende Umsetzung mit Aminen X, gegebenenfalls bei erhöhter Temperatur und unter Zusatz von Hilfsbasen wie beispielsweise Triethylamin oder Diisopropylethylamin führt zu den erfindungsgemäßen Verbindungen der allgemeinen Formel (I) mit Harnstoff-Brücke (X¹ = NH). Die Amine X können entweder käuflich erworben oder nach Literatur-bekannten Methoden hergestellt werden. Die Darstellung der erfindungsgemäßen Verbindungen I mit R³ = H kann durch Verwendung entsprechender Boc-geschützter Amine X (R³ = Boc) erfolgen. Die Boc-Schutzgruppe kann anschließend entfernt werden, zum Beispiel durch Behandlung mit Trifluoressigsäure in Dichlormethan.

### SYNTHESESCHEMA 1

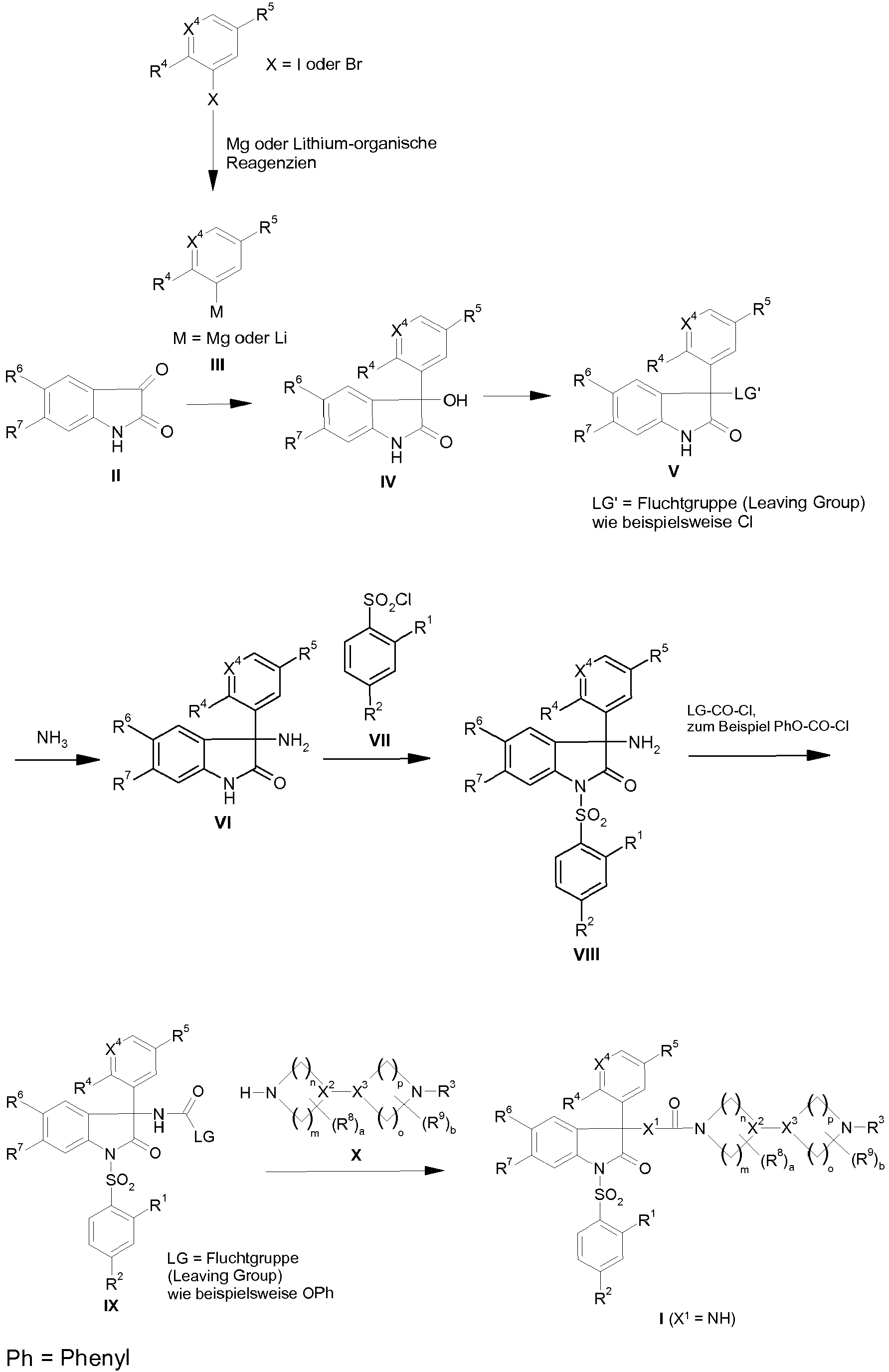

Die Herstellung der erfindungsgemäßen Verbindungen der allgemeinen Formel I, welche eine Carbamat-Gruppe in der 3-Position tragen (X¹ = O), kann wie in WO 2006/005609 beschrieben und in Syntheseschema 2 gezeigt erfolgen: Zunächst wird die 3-HydroxyVerbindung IV mit Chlorameisensäurephenylester zu den Phenylcarbonat-Derivaten XIa und / oder XIb umgesetzt. Mit einem Überschuss an Amin X werden die Carbamat-Derivate XII erhalten, die anschliessend unter den üblichen Bedingungen (Deprotonierung mit einer starken Base, wie zum Beispiel Natriumhydrid oder Kalium-*tert*-butylat in einem geeigneten Lösungsmittel, wie zum Beispiel DMF, gefolgt von Behandlung mit Sulfonsäurechloriden VII) in die erfindungsgemäßen Verbindungen I mit Carbamat-Brücke überführt werden können.

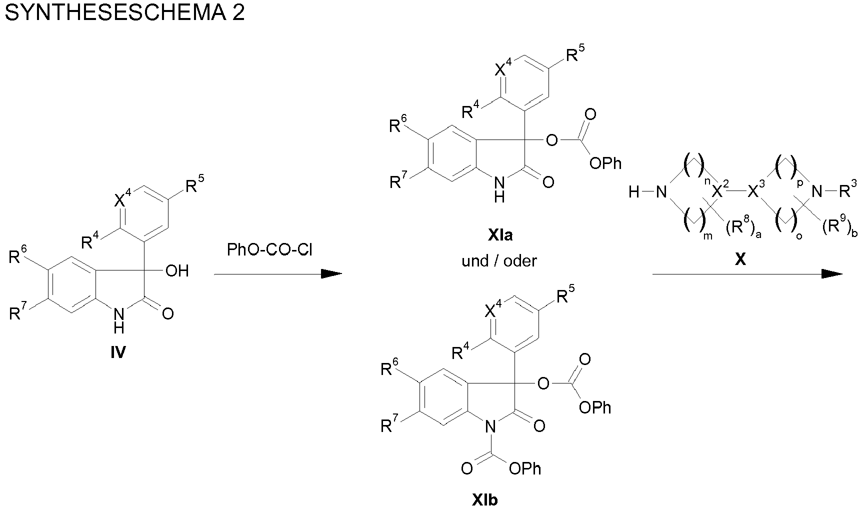

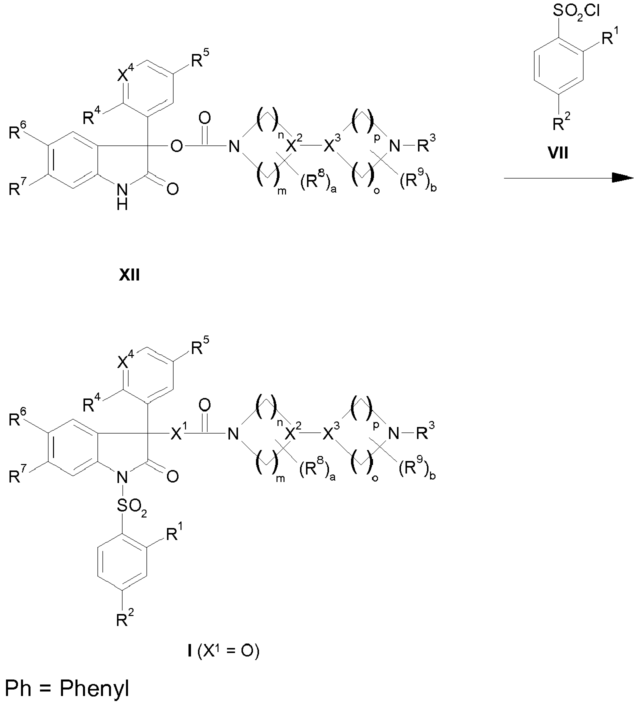

Die Herstellung der erfindungsgemäßen Verbindungen der allgemeinen Formel I, welche eine 2-Oxoethyl-Gruppe in der 3-Stellung tragen (X¹ = CH₂), kann wie in Syntheseschema 3 gezeigt erfolgen. Die Einführung der Essigsäure-Gruppierung kann wie in WO 2006/005609 beschrieben in einer 4-Stufen-Sequenz (1. Substitution der Fluchtgruppe LG' in V mit dem Natriumsalz von Dimethylmalonat, 2. Verseifung der ersten Estergruppe, 3. thermische Decarboxylierung, 4. Verseifung der zweiten Estergruppe) erfolgen. Die Amin-Seitenkette X kann unter Verwendung der aus der Peptid-Chemie bekannten Standard-Kupplungsreagenzien, wie zum Beispiel EDC (N-(3-Dimethylaminopropyl)-N'-ethyl-carbodiimid-hydrochlorid) und HOBT (1-Hydroxy-benzotriazol) in einem Lösungsmittel, wie zum Beispiel N,N-Dimethylformamid, oder BOP (1-Benzotriazolyloxy-tris-(dimethylamino)-phosphoniumhexafluorophosphat) in Gegenwart einer Base, wie Triethylamin oder Düsopropyethylamin, an die Carbonsäure XV gekuppelt werden. Die Sulfonylierung kann durch Deprotonierung der Kupplungsprodukte XVI mit einer starken Base, wie zum Beispiel Natriumhydrid oder Kalium-*tert*-butylat, und anschließende Behandlung mit Sulfonsäurechloriden VII in einem Lösungsmittel, wie zum Beispiel DMF, erfolgen und führt zu den erfindungsgemäßen Verbindungen I mit Amid-Brücke.

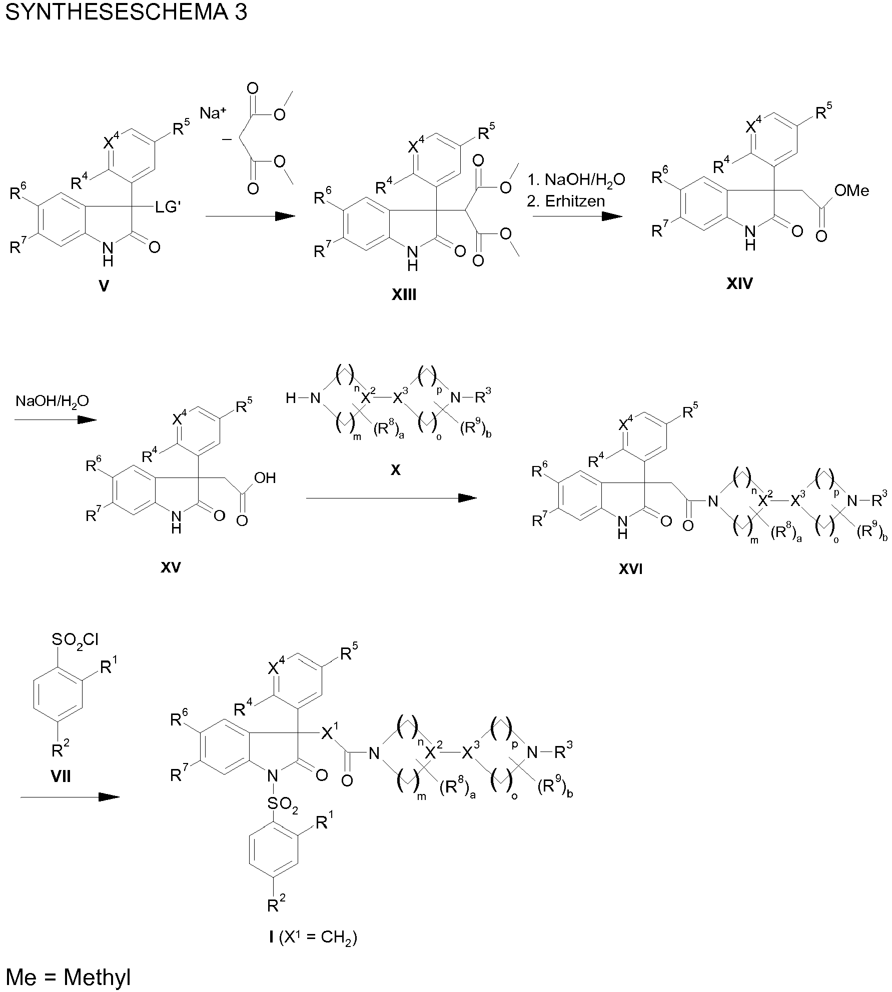

Ein weiterer Gegenstand der vorliegenden Erfindung betrifft ein pharmazeutisches Mittel, enthaltend mindestens eine Verbindung der allgemeinen Formel I und/oder ein pharmazeutisch verträgliches Salz oder eine Prodrug davon, wie vorstehend ausgeführt, und einen pharmazeutisch verträglichen Träger. Geeignete Träger hängen unter anderem von der Darreichungsform des Mittels ab und sind dem Fachmann grundsätzlich bekannt. Einige geeignete Träger sind weiter unten beschrieben.

Ein weiterer Gegenstand der vorliegenden Erfindung betrifft die Verwendung von Verbindungen der Formel I und/oder von pharmazeutisch verträglichen Salzen oder Prodrugs davon zur Herstellung eines Medikaments zur Behandlung und/oder Prophylaxe von Vasopressin-abhängigen Krankheiten.

Vasopressin-abhängige Krankheiten sind solche, bei denen der Krankheitsverlauf zumindest teilweise von Vasopressin abhängt, d.h. Krankheiten, die einen erhöhten Vasopressin-Spiegel zeigen, der unmittelbar oder indirekt zum Krankheitsbild beitragen kann. Anders ausgedrückt sind Vasopressin-abhängige Krankheiten solche, die durch Modulation des Vasopressinrezeptors, beispielsweise durch Gabe eines Vasopressinrezeptorliganden (Agonist, Antagonist, partieller Antagonist/Agonist, inverser Agonist etc.), beeinflusst werden können.

In einer bevorzugten Ausführungsform betrifft die vorliegende Erfindung die Verwendung von erfindungsgemäßen Verbindungen der Formel I oder von pharmazeutisch verträglichen Salzen oder Prodrugs zur Herstellung eines Medikaments zur Behandlung und/oder Prophylaxe von Krankheiten, die ausgewählt sind unter Diabetes, Insulin-Resistenz, Enuresis nocturna, Inkontinenz und Krankheiten, bei denen Blutgerinnungsstörungen auftreten, und/oder zur Verzögerung der Miktion. Unter dem Begriff "Diabetes" sind alle Diabetesformen zu verstehen, vor allem Diabetes mellitus (einschließlich Typ I und insbesondere Typ II), Diabetes renalis und insbesondere Diabetes insipidus. Bevorzugt handelt es sich bei den Diabetesformen um Diabetes mellitus vom Typ II (mit Insulinresistenz) oder Diabetes insipidus.

In einer weiteren bevorzugten Ausführungsform betrifft die vorliegende Erfindung die Verwendung von erfindungsgemäßen Verbindungen der Formel I oder von pharmazeutisch verträglichen Salzen oder Prodrugs davon zur Herstellung eines Medikaments zur Behandlung und/oder Prophylaxe von Krankheiten, die ausgewählt sind unter Hypertonie, pulmonaler Hypertonie, Herzinsuffizienz, Myocardinfarkt, koronarem Spasmus, instabiler Angina, PTCA (percutaneous transluminal coronary angioplastie), Ischämien des Herzens, Störungen des renalen Systems, Ödemen, renalem Vasospasmus, Nekrose des renalen Cortex, Hyponatriämie, Hypokaliämie, Schwartz-Bartter Syndrom, Störungen des Gastrointestinaltraktes, gastritischem Vasospasmus, Hepatozirrhose, Magen- und Darmulkus, Emesis, auftretender Emesis bei der Chemotherapie, und Reisekrankheit.

Die erfindungsgemäßen Verbindungen der Formel I bzw. ihre pharmazeutisch verträglichen Salze oder Prodrugs oder das erfindungsgemäße pharmazeutische Mittel können auch zur Behandlung von verschiedenen Vasopressin-abhängigen Beschwerden, die zentralnervöse Ursachen oder Veränderungen in der HPA-Achse (hypothalamic pituitary adrenal axis) aufweisen, verwendet werden, zum Beispiel bei affektiven Störungen, wie depressiven Störungen und bipolaren Störungen. Dazu gehören zum Beispiel dysthyme Störungen, Phobien, posttraumatische Belastungsstörungen, generelle Angsstörungen, Panikstörungen, saisonale Depressionen und Schlafstörungen.

Ebenso können die erfindungsgemäßen Verbindungen der Formel I bzw. ihre pharmazeutisch verträglichen Salze oder Prodrugs oder das erfindungsgemäße pharmazeutische Mittel zur Behandlung bei Angststörungen und stressabhängigen Angststörungen eingesetzt werden, wie zum Beispiel generalisierten Angststörungen, Phobien, posttraumatischen Angststörungen, panischen Angststörungen, obsessiv-zwanghaften Angststörungen, akuten stressabhängigen Angststörungen und Sozialphobie.

Weiterhin können die erfindungsgemäßen Verbindungen auch zur Behandlung von Gedächnisleistungsstörungen, Morbus Alzheimer, Psychosen, psychotischen Störungen, Schlafstörungen und/oder dem Cushing Syndrom sowie allen stressabhängigen Krankheiten eingesetzt werden.

Dementsprechend betrifft eine weitere bevorzugte Ausführungsform der vorliegenden Erfindung die Verwendung von erfindungsgemäßen Verbindungen der Formel I oder von pharmazeutisch verträglichen Salzen oder Prodrugs davon zur Herstellung eines Medikaments zur Behandlung von affektiven Störungen.

In einer weiteren bevorzugten Ausführungsform betrifft die vorliegende Erfindung die Verwendung von erfindungsgemäßen Verbindungen der Formel I oder von pharmazeutisch verträglichen Salzen oder Prodrugs davon zur Herstellung eines Medikaments zur Behandlung von Angststörungen und/oder stressabhängigen Angststörungen.

In einer weiteren bevorzugten Ausführungsform betrifft die vorliegende Erfindung die Verwendung von erfindungsgemäßen Verbindungen der Formel I oder von pharmazeutisch verträglichen Salzen oder Prodrugs davon zur Herstellung eines Medikaments zur Behandlung von Gedächtnisleistungsstörungen und/oder Morbus Alzheimer.

In einer weiteren bevorzugten Ausführungsform betrifft die vorliegende Erfindung die Verwendung von erfindungsgemäßen Verbindungen der Formel I oder von pharmazeutisch verträglichen Salzen oder Prodrugs davon zur Herstellung eines Medikaments zur Behandlung von Psychosen und/oder psychotischen Störungen.

In einer weiteren bevorzugten Ausführungsform betrifft die vorliegende Erfindung die Verwendung von erfindungsgemäßen Verbindungen der Formel I oder von pharmazeutisch verträglichen Salzen oder Prodrugs davon zur Herstellung eines Medikaments zur Behandlung des Cushing-Syndroms oder sonstigen stressabhängigen Krankheiten.

In einer weiteren bevorzugten Ausführungsform betrifft die vorliegende Erfindung die Verwendung von erfindungsgemäßen Verbindungen der Formel I oder von pharmazeutisch verträglichen Salzen oder Prodrugs davon zur Herstellung eines Medikaments zur Behandlung von Schlafstörungen.

In einer weiteren bevorzugten Ausführungsform betrifft die vorliegende Erfindung die Verwendung von erfindungsgemäßen Verbindungen der Formel I oder von pharmazeutisch verträglichen Salzen oder Prodrugs davon zur Herstellung eines Medikaments zur Behandlung von depressiven Erkrankungen. Eine besondere Form von depressiven Erkrankungen sind sogenannte childhood onset mood disorders, d.h. depressive Verstimmungen, die bereits in der Kindheit einsetzen.

In einer weiteren bevorzugten Ausführungsform betrifft die vorliegende Erfindung die Verwendung von erfindungsgemäßen Verbindungen der Formel I oder von pharmazeutisch verträglichen Salzen oder Prodrugs davon zur Herstellung eines Medikaments zur Behandlung von vasomotorischen Symptomen und/oder thermoregulatorischen Fehlfunktionen, wie beispielsweise das "hot flush"-Symptom.

In einer weiteren bevorzugten Ausführungsform betrifft die vorliegende Erfindung die Verwendung von erfindungsgemäßen Verbindungen der Formel I oder von pharmazeutisch verträglichen Salzen oder Prodrugs davon zur Herstellung eines Medikaments zur Behandlung und/oder Prophylaxe von Drogen-, Arzneimittel- und/oder durch sonstige Faktoren vermittelten Abhängigkeiten, zur Behandlung und/oder Prophylaxe von Stress bedingt durch den Entzug von einem oder mehreren die Abhängigkeit vermittelnden Faktoren und/oder zur Behandlung und/oder Prophylaxe von Stress-induzierten Rückfällen in die Drogen-, Arzneimittel- und/oder durch sonstige Faktoren vermittelten Abhängigkeiten.

In einer weiteren bevorzugten Ausführungsform betrifft die vorliegende Erfindung die Verwendung von erfindungsgemäßen Verbindungen der Formel I oder von pharmazeutisch verträglichen Salzen oder Prodrugs davon zur Herstellung eines Medikaments zur Behandlung und/oder Prophylaxe von Schizophrenie und/oder Psychose.

Ein weiterer Gegenstand der Erfindung betrifft ein Verfahren zur Behandlung und/oder Prophylaxe von Vasopressin-abhängigen Krankheiten, bei dem man einem Patienten eine wirksame Menge wenigstens einer erfindungsgemäßen Verbindung der Formel I der wenigstens eines pharmazeutisch verträglichen Salzes oder einer Prodrug davon oder eines erfindungsgemäßen pharmazeutischen Mittels verabreicht.

Bezüglich der Definition von Vasopressin-abhängigen Krankheiten wird auf die vorstehenden Ausführungen Bezug genommen.

In einer bevorzugten Ausführungsform der Erfindung dient das erfindungsgemäße Verfahren zur Behandlung und/oder Prophylaxe von Erkrankungen, die ausgewählt sind unter Diabetes, Insulin-Resistenz, Enuresis nocturna, Inkontinenz und Krankheiten, bei denen Blutgerinnungsstörungen auftreten, und/oder zur Verzögerung der Miktion. Bezüglich der Definition von Diabetes wird auf die vorstehenden Ausführungen verwiesen.

In einer weiteren bevorzugten Ausführungsform dient das erfindungsgemäße Verfahren zur Behandlung und/oder Prophylaxe von Erkrankungen, die ausgewählt sind unter Hypertonie, pulmonaler Hypertonie, Herzinsuffizienz, Myocardinfarkt, koronarem Spasmus, instabiler Angina, PTCA (percutaneous transluminal coronary angioplastie), Ischämien des Herzens, Störungen des renalen Systems, Ödeme, renalem Vasospasmus, Nekrose des renalen Cortex, Hyponatriämie, Hypokaliämie, Schwartz-Bartter Syndrom, Störungen des Gastrointestinaltraktes, gastritischem Vasospasmus, Hepatozirrhose, Magen- und Darmulkus, Emesis, auftretender Emesis bei der Chemotherapie und Reisekrankheit.

In einer weiteren bevorzugten Ausführungsform dient das erfindungsgemäße Verfahren zur Behandlung und/oder Prophylaxe von affektiven Störungen.

In einer weiteren bevorzugten Ausführungsform dient das erfindungsgemäße Verfahren zur Behandlung und/oder Prophylaxe von Angststörungen und/oder stressabhängigen Angststörungen.

In einer weiteren bevorzugten Ausführungsform dient das erfindungsgemäße Verfahren zur Behandlung und/oder Prophylaxe Gedächtnisleistungsstörungen und/oder Morbus Alzheimer.

In einer weiteren bevorzugten Ausführungsform dient das erfindungsgemäße Verfahren zur Behandlung und/oder Prophylaxe von Psychosen und/oder psychotischen Störungen.

In einer weiteren bevorzugten Ausführungsform dient das erfindungsgemäße Verfahren zur Behandlung und/oder Prophylaxe des Cushing-Syndroms.

In einer weiteren bevorzugten Ausführungsform dient das erfindungsgemäße Verfahren zur Behandlung und/oder Prophylaxe von Schlafstörungen bei einem Patienten.

In einer weiteren bevorzugten Ausführungsform dient das erfindungsgemäße Verfahren zur Behandlung und/oder Prophylaxe von depressiven Erkrankungen. Bei den depressiven Erkrankungen sind speziell auch die chlidhood onset mood disorders zu nennen, d.h. depressive Verstimmungen, die bereits im Kindesalter einsetzen.

In einer weiteren bevorzugten Ausführungsform dient das erfindungsgemäße Verfahren zur Behandlung und/oder Prophylaxe von von vasomotorischen Symptomen und/oder thermoregulatorischen Fehlfunktionen, wie beispielsweise dem "hot flush"-Symptom.

In einer weiteren bevorzugten Ausführungsform dient das erfindungsgemäße Verfahren zur Behandlung und/oder Prophylaxe von Drogen-, Arzneimittel- und/oder durch sonstige Faktoren vermittelte Abhängigkeiten, zur Behandlung und/oder Prophylaxe von Stress bedingt durch den Entzug von einem oder mehreren die Abhängigkeit vermittelnden Faktoren und/oder zur Behandlung und/oder Prophylaxe von Stress-induzierten Rückfällen in die Drogen-, Arzneimittel- und/oder durch sonstige Faktoren vermittelten Abhängigkeiten.

In einer weiteren bevorzugten Ausführungsform dient das erfindungsgemäße Verfahren zur Behandlung und/oder Prophylaxe von Schizophrenie und/oder Psychose.

Bei dem mit dem erfindungsgemäßen Verfahren prophylaktisch oder therapeutisch zu behandelnden Patienten handelt es sich vorzugsweise um ein Säugetier, beispielsweise um einen Menschen oder um ein nichtmenschliches Säugetier oder um ein nichtmenschliches transgenes Säugetier. Speziell handelt es sich um einen Menschen.

Die Verbindungen der allgemeinen Formel I, ihre pharmazeutisch verträglichen Salze und Prodrugs, wie vorstehend ausgeführt, können von einem Fachmann in Kenntnis der erfindungsgemäßen technischen Lehre in Ausführung und/oder in analoger Ausführung von an sich bekannten Verfahrensschritten hergestellt werden.

Die Verbindungen I bzw. deren Prodrugs und/oder deren pharmazeutisch verträglichen Salze zeichnen sich dadurch aus, dass sie eine Selektivität zum Vasopressin-Rezeptorsubtyp V1b gegenüber mindestens einem der nahe verwandten Vasopressin/Oxytozin-Rezeptorsubtypen (zum Beispiel Vasopressin V1a, Vasopressin V2 und/oder Oxytozin) aufweisen.

Alternativ oder vorzugsweise zusätzlich zeichnen sich die Verbindungen I bzw. deren Prodrugs und/oder deren pharmazeutisch verträglichen Salze dadurch aus, dass sie eine verbesserte metabolische Stabilität aufweisen.

Die metabolische Stabilität einer Verbindung kann beispielsweise gemessen werden, indem man eine Lösung dieser Verbindung mit Lebermikrosomen von bestimmten Spezies (zum Beispiel Ratte, Hund oder Mensch) inkubiert und die Halbwertszeit der Verbindung unter diesen Bedingungen bestimmt (RS Obach, Curr Opin Drug Discov Devel. 2001, 4, 36-44). Dabei kann aus einer beobachteten größeren Halbwertszeit auf eine verbesserte metabolische Stabilität der Verbindung geschlossen werden. Die Stabilität in Gegenwart von humanen Lebermikrosomen ist von besonderem Interesse, da sie eine Vorhersage für den metabolischen Abbau der Verbindung in der menschlichen Leber ermöglicht. Verbindungen mit erhöhter metabolischer Stabilität (gemessen in dem Lebermikrosomen-Test) werden deshalb wahrscheinlich auch in der Leber langsamer abgebaut. Der langsamere metabolische Abbau in der Leber kann zu höheren und/oder länger anhaltenden Konzentrationen (Wirkspiegel) der Verbindung im Körper führen, so dass die Eliminierungs-Halbwertszeit der erfindungsgemässen Verbindungen erhöht ist. Erhöhte und/oder länger anhaltende Wirkspiegel können zu einer besseren Wirksamkeit der Verbindung in der Behandlung oder Prophylaxe von verschiedenen Vasopressin-abhängigen Krankheiten führen. Außerdem kann eine verbesserte metabolische Stabilität zu einer erhöhten Bioverfügbarkeit nach oraler Gabe führen, da die Verbindung nach erfolgter Resorption im Darm einem geringeren metabolischen Abbau in der Leber (sogenannter "first pass effect") unterliegt. Eine erhöhte orale Bioverfügbarkeit kann aufgrund erhöhter Konzentration (Wirkspiegel) der Verbindung zu einer besseren Wirksamkeit der Verbindung nach oraler Gabe führen.

Alternativ oder vorzugsweise zusätzlich zeichnen sich die Verbindungen I bzw. deren Prodrugs und/oder deren pharmazeutisch verträglichen Salze dadurch aus, dass sie in Patienten oder relevanten Tiermodellen, die prognostische Aussagen für die Anwendung in der Behandlung ermöglichen, gegenüber den aus dem Stand der Technik bekannten Oxindol-Verbindungen eine verbesserte pharmakologische Aktivität aufweisen.

Die erfindungsgemäßen Verbindungen sind nach Verabreichung auf verschiedenen Wegen wirksam. Die Verabreichung kann beispielsweise intravenös, intramuskulär, subkutan, topisch, intratracheal, intranasal, transdermal, vaginal, rektal, sublingual, bukkal oder oral erfolgen und erfolgt häufig intravenös, intramuskulär oder insbesondere oral.

Die vorliegende Erfindung betrifft auch pharmazeutische Zusammensetzungen, die eine wirksame Dosis einer erfindungsgemäßen Verbindung I, eines pharmazeutisch verträglichen Salzes oder einer Prodrug davon und geeignete pharmazeutische Träger (Arzneiträger) enthalten.

Diese Arzneiträger werden entsprechend der pharmazeutischen Form und der gewünschten Applikationsart gewählt und sind dem Fachmann grundsätzlich bekannt.

Die erfindungsgemäßen Verbindungen der Formel I oder gegebenenfalls geeignete Salze dieser Verbindungen können zur Herstellung von pharmazeutischen Zusammensetzungen zur oralen, sublingualen, bukkalen, subkutanen, intramuskulären, intravenösen, topischen, intratrachealen, intranasalen, transdermalen, vaginalen oder rektalen Verabreichung verwendet werden und Tieren oder Menschen in einheitlichen Verabreichungsformen, gemischt mit herkömmlichen pharmazeutischen Trägern, zur Prophylaxe oder Behandlung der obigen Störungen oder Krankheiten verabreicht werden.

Die geeigneten Verabreichungsformen (Dosiereinheiten) umfassen Formen zur oralen Verabreichung, wie Tabletten, Gelatinekapseln, Pulver, Körnchen und Lösungen oder Suspensionen zur oralen Einnahme, Formen zur sublingualen, bukkalen, intratrachealen oder intranasealen Verabreichung, Aerosole, Implantate, Formen der subkutanen, intramuskulären oder intravenösen Verabreichung und Formen der rektalen Verabreichung.

Zur topischen Verabreichung können die erfindungsgemäßen Verbindungen in Cremes, Salben oder Lotionen verwendet werden.

Um den gewünschten prophylaktischen oder therapeutischen Effekt zu erzielen, kann die Dosis des Wirkstoffs zwischen 0.01 und 50 mg pro kg Körpergewicht und pro Tag variieren.

Jede Einheitsdosis kann 0.05 bis 5000 mg, vorzugsweise 1 bis 1000 mg, des Wirkstoffs in Kombination mit einem pharmazeutischen Träger enthalten. Diese Einheitsdosis kann 1-bis 5-mal am Tag verabreicht werden, so dass eine tägliche Dosis von 0,5 bis 25000 mg, vorzugsweise 1 bis 5000 mg, verabreicht wird.

Falls eine feste Zusammensetzung in Form von Tabletten zubereitet wird, wird der Wirkstoff mit einem festen pharmazeutischen Träger, wie Gelatine, Stärke, Laktose, Magnesiumstearat, Talk, Siliziumdioxid oder Ähnlichem, gemischt.

Die Tabletten können mit Saccharose, einem Cellulosederivat oder einer anderen geeigneten Substanz beschichtet werden oder anders behandelt werden, um eine anhaltende oder verzögerte Aktivität aufzuweisen und um eine vorbestimmte Menge des Wirkstoffs kontinuierlich freizugeben.

Eine Zubereitung in Form von Gelatinekapseln wird durch Mischen des Wirkstoffs mit einem Streckmittel und Aufnehmen der resultierenden Mischung in weiche oder harte Gelatinekapseln erhalten.

Eine Zubereitung in Form eines Sirups oder Elixiers oder zur Verabreichung in Form von Tropfen kann Wirkstoffe zusammen mit einem Süßstoff, der vorzugsweise kalorienfrei ist, Methylparaben oder Propylparaben als Antiseptika, einen Aromastoff und einen geeigneten Farbstoff enthalten.

Die wasserdispergierbaren Pulver oder Körnchen können die Wirkstoffe, gemischt mit Dispergiermitteln, Benetzungsmitteln oder Suspensionsmitteln, wie Polyvinylpyrrolidone, sowie Süßstoffe oder Geschmackskorrigentien, enthalten.

Eine rektale oder vaginale Verabreichung wird durch Verwendung von Zäpfchen erreicht, die mit Bindemitteln zubereitet werden, die bei rektaler Temperatur schmelzen, zum Beispiel Kakaobutter oder Polyethylenglykole. Eine parenterale Verabreichung erfolgt unter Verwendung von wässrigen Suspensionen, isotonischen Salzlösungen oder sterilen und injizierbaren Lösungen, die pharmakologisch verträgliche Dispergiermittel und/oder Benetzungsmittel, zum Beispiel Propylenglykol oder Polyethylenglykol, enthalten.

Der Wirkstoff kann auch als Mikrokapseln oder Zentrosome, falls geeignet mit einem oder mehreren Trägern oder Additiven, formuliert werden.

Zusätzlich zu den erfindungsgemäßen Verbindungen können die erfindungsgemäßen Mittel andere Wirkstoffe enthalten, die zur Behandlung der oben angegebenen Störungen oder Krankheiten nützlich sein können.

Die vorliegende Erfindung betrifft somit weiterhin pharmazeutische Mittel, in denen mehrere Wirkstoffe zusammen anwesend sind, wobei mindestens einer von diesen eine erfindungsgemäße Verbindung I, Salz oder eine Prodrug davon ist.

Die Erfindung wird nachstehend anhand von Beispielen näher erläutert, wobei die Beispiele nicht einschränkend zu verstehen sind.

Die Herstellung der erfindungsgemäßen Verbindungen kann über verschiedene Syntheserouten erfolgen. Die genannten Vorschriften, wie entsprechend in den Syntheseschemata 1, 2 und 3 beschrieben, sind nur exemplarisch anhand der genannten Beispiele näher erläutert, ohne ausschließlich auf die genannten Synthesewege 1, 2 oder 3 oder Analogvorschriften beschränkt zu sein.

### EXPERIMENTELLER TEIL

Verwendete Abkürzungen:
- THF:: Tetrahydrofuran
- DMSO:: Dimethylsulfoxid
- TFA:: Trifluoressigsäure
- BOP:: 1-Benzotriazolyloxy-tris-(dimethylamino)-phosphoniumhexafluorophosphat
- p:: pseudo (beispielsweise pt pseudo Triplett)
- b:: breit (beispielsweise bs breites Singulett)
- s:: Singulett
- d:: Doublett
- t:: Triplett
- m:: Multiplett
- dd:: doppeltes Doublett
- dt:: doppeltes Triplett
- tt:: dreifaches Triplett

### I. Herstellung der Zwischenverbindungen

5,6-Difluorisatin ist kommerziell z B: von den Anbietern ASYMCHEM, BUTT PARK, TIMECHEM und UKRORGSYN-BB erhältlich.
5-Brom-6-fluor-isatin ist kommerziell z. B. von dem Anbieter BUTT PARK und UKRORGSYN-BB erhältlich.
5-Chlor-6-fluor-isatin ist kommerziell z. B. von dem Anbieter UKRORGSYN-BB erhältlich. 5-Fluor-6-chlor-isatin ist kommerziell z. B. von dem Anbieter BUTT PARK erhältlich.

### a) 3-Hydroxy-1,3-dihydroindol-2-one der Formel IV

### a.1 5-Brom-3-(2-ethoxypyridin-3-yl)-6-fluor-3-hydroxy-1,3-dihydroindol-2-on

Bildung des Isatin-Natriumsalzes: Zu 12.5 g (51.2 mmol) 5-Brom-6-fluorisatin in 250 ml THF gab man bei 0 °C portionsweise 2.46 g (51.2 mmol) Natriumhydrid (60% in Mineralöl) und rührte eine Stunde bei 0 °C.

Bildung des Grignard-Reagenzes: Zu einer Lösung von 2-Ethoxy-3-iodpyridin (12.76 g, 51.2 mmol) in 250 ml THF tropfte man Ethylmagnesiumbromid (61.5 mmol, 61.5 ml einer 1 M Lösung in THF), wobei man die Temperatur zwischen 15 und 22 °C hielt. Anschließend rührte man das Reaktionsgemisch 15 min bei Raumtemperatur.

Grignard-Addition: Man pumpte die Lösung des Grignard-Reagenzes zu der eisgekühlten Lösung des Isatin-Natriumsalzes und rührte das Reaktionsgemisch anschließend drei Stunden bei Raumtemperatur. Man goss den Ansatz in 10% Ammoniumchlorid-Lösung und extrahierte dreimal mit Ethylacetat. Die vereinigten organischen Phasen wurden mit Wasser und gesättigter Kochsalzlösung gewaschen, mit Magnesiumsulfat getrocknet und unter vermindertem Druck eingeengt. Der nach Stehen über Nacht bei Raumtemperatur gebildete kristalline Niederschlag wurde abgesaugt und mit Ethylacetat gewaschen. Man erhielt 10.0 g der Titelverbindung als Feststoff.
ESI-MS: 367.00/369.00 [M+H]⁺

### a.2 5-Chlor-3-(2-ethoxypyridin-3-yl)-6-fluor-3-hydroxy-1,3-dihydro-indol-2-on

Man stellte die Titelverbindung in Analogie zu Beispiel a.1 unter Verwendung von 5-Chlor-6-fluorisatin als Isatin II her.
ESI-MS: 323.05 [M+H]⁺

### a.3 5-Chlor-3-[2-(2,2-difluorethoxy)-phenyl]-6-fluor-3-hydroxy-1,3-dihydro-indol-2-on

Man stellte die Titelverbindung in Analogie zu Beispiel a.1 unter Verwendung von 5-Chlor-6-fluorisatin als Isatin II und 2,2-Difluorethoxyiodbenzol (für die Bildung des Grignard-Reagenzes) her.

### a.4 5-Chlor-3-(2-ethoxy-phenyl)-6-fluor-3-hydroxy-1,3-dihydro-indol-2-on

Man stellte die Titelverbindung in Analogie zu Beispiel a.1 unter Verwendung von 5-Chlor-6-fluorisatin als Isatin II und Ethoxyiodbenzol (für die Bildung des Grignard-Reagenzes) her.

### a.5 3-(2-Ethoxy-pyridin-3-yl)-6-fluor-3-hydroxy-2-oxo-2,3-dihydro-1 H-indol-5-carbonitril

Zu einer Lösung von 1.7 g (4.63 mmol) 5-Brom-3-(2-ethoxy-pyridin-3-yl)-6-fluor-3-hydroxy-1,3-dihydroindol-2-on aus Beispiel a.1 und 544 mg (4.63 mmol) Zinkcyanid in 18 ml Dimethylformamid (DMF) gab man 161 mg (0.14 mmol) Tetrakis(triphenylphosphin)-palladium (0) und rührte 1 h bei 150°C in einem Biotage Mikrowellengerät. Zur Aufarbeitung verdünnte man die Reaktionslösung mit 300 ml Wasser, extrahierte mit Essigsäureethylester (3x) und wusch mit gesättigter Natriumchlorid-Lösung (1 x). Die vereinigten organischen Phasen wurden über Magnesiumsulfat getrocknet, filtriert und das Lösungsmittel im Vakuum entfernt. Man erhielt 1.67 g 3-(2-Ethoxy-pyridin-3-yl)-6-fluor-3-hydroxy-2-oxo-2,3-dihydro-1H-indol-5-carbonitril.
ESI-MS: 313.10 [M+H]⁺
¹H-NMR (400 MHz, d₆-DMSO): δ [ppm] 7.85 (d, 1 H); 7.30 (t, 1 H); 7.25 (d, 1 H); 7.05 (t, 1 H); 6.95 (d, 1 H); 6.85 (d, 1 H); 6.80 (s, 1 H); 3.75 (m, 2H); 0.95 (t, 3H).

### II. Herstellung der Verbindungen der Formel I

### II.1 Verbindungen der Formel I, worin X¹ für NH steht (Beispiele 1 bis 37)

### BEISPIEL 1:

### 4-(1-Methyl-piperidin-4-yl)-piperazin-1-carbonsäure-[5-cyano-1-(2,4-dimethoxy-phenylsulfonyl)-3-(2-ethoxy-pyridin-3-yl)-6-fluor-2-oxo-2,3-dihydro-1H-indol-3-yl]-amid

### 1.1 3-Chlor-3-(2-ethoxypyridin-3-yl)-6-fluor-2-oxo-2,3-dihydro-1H-indol-5-carbonitril

Zu 1.3 g (4.15 mmol) 3-(2-Ethoxy-pyridin-3-yl)-6-fluor-3-hydroxy-2-oxo-2,3-dihydro-1H-indol-5-carbonitril in 30 ml Dichlormethan gab man 0.47 ml (5.81 mmol) Pyridin. Nach dem Kühlen der Reaktionsmischung auf 0 °C tropfte man 0.42 ml (5.81 mmol) Thionylchlorid zu. Man rührte die Mischung bei Raumtemperatur eine Stunde und goss anschließend in Eiswasser. Nach 15 Minuten Rühren trennte man die organische Phase ab. Die wässrige Phase wurde mehrmals mit Dichlormethan extrahiert. Man trocknete die vereinigte organische Phase über Magnesiumsulfat, filtrierte und entfernte das Lösungsmittel im Vakuum. Man erhielt 1.13 g 3-Chlor-3-(2-ethoxy-pyridin-3-yl)-6-fluor-2-oxo-2,3-dihydro-1 H-indol-5-carbonitril als Feststoff, der ohne weitere Aufreinigung in der nächsten Stufe eingesetzt wurde.
ESI-MS: 332.00 [M+H]⁺

### 1.2 3-Amino-3-(2-ethoxy-pyridin-3-yl)-6-fluor-2-oxo-2,3-dihydro-1H-indol-5-carbonitril

Zu einer gekühlten Lösung von 1.13 g (3.41 mmol) 3-Chlor-3-(2-ethoxy-pyridin-3-yl)-6-fluor-2-oxo-2,3-dihydro-1H-indol-5-carbonitril in 20 ml Dichlormethan tropfte man unter Stickstoffatmosphäre 2.4 ml (17.0 mmol) einer 7 N methanolischen Ammoniaklösung und rührte danach das Reaktionsgemisch über Nacht bei Raumtemperatur. Das Reaktionsgemisch wurde mit gesättigter NaCl-Lösung versetzt und mit Essigsäureethylester (3x) extrahiert. Man trocknete die vereinigten organischen Phasen über Magnesiumsulfat, filtrierte und entfernte das Lösungsmittel im Vakuum. Der Rückstand wurde mit 20 ml Diethylether versetzt. Nach 5 min. Rühren fiel ein weißer Feststoff aus, der abfiltriert und im Vakuumtrockenschrank getrocknet wurde. Man erhielt 800 mg der Titelverbindung als weißen Feststoff.
ESI-MS: 313.05 [M+H]⁺

### 1.3 3-Amino-1-(2,4-dimethoxyphenylsulfonyl)-3-(2-ethoxy-pyridin-3-yl)-6-fluor-2-oxo-2,3-dihydro-1H-indol-5-carbonitril

Zu einer Lösung von 350 mg (1.12 mmol) 3-Amino-3-(2-ethoxy-pyridin-3-yl)-6-fluor-2-oxo-2,3-dihydro-1H-indol-5-carbonitril in 15 ml wasserfreiem Dimethylformamid gab man unter Stickstoffatmosphäre und unter Kühlung durch ein Eisbad 80 mg (1.68 mmol) Natriumhydrid (60% Dispersion in Mineralöl) portionsweise zu. Man rührte 10 min bei 0 °C und gab danach 398 mg (1.68 mmol) 2,4-Dimethoxyphenylsulfonylchlorid zu und rührte 15 min. bei Raumtemperatur nach. Die Reaktionsmischung wurde in Eiswasser gegossen und anschließend mit Essigester extrahiert. Man wusch die organische Phase mit gesättigter Natriumchlorid-Lösung, trocknete über Magnesiumsulfat und verdampfte das Lösungsmittel. Der Rückstand wurde chromatographisch über Kieselgel (Redisep-Kartusche, Laufmittel-Gradient von 0 bis 3% Methanol in Essigester) gereinigt. Man erhielt 236 mg der Titelverbindung als weißen Feststoff.
ESI-MS: 513.15 [M+H]⁺
¹H-NMR (500 MHz, d₆-DMSO): δ [ppm] 8.25 (d, 1 H); 8.10 (m, 1 H); 7.95 (d, 1 H); 7.75 (d, 1 H); 7.45 (d, 1 H); 7.10 (m, 1 H); 6.75 (m, 2H); 4.00 (m, 2H); 3.90 (s, 3H); 3.75 (s, 3H); 3.40 (m, 2H); 0.85 (t, 3H).

### 1.4 [5-Cyano-1-(2,4-dimethoxyphenylsulfonyl)-3-(2-ethoxy-pyridin-3-yl)-6-fluor-2-oxo-2,3-dihydro-1H-indol-3-yl]-carbaminsäurephenylester

Zu einer auf 0 °C gekühlten Lösung von 226 mg (0.44 mmol) 3-Amino-1-(2,4-dimethoxy-phenylsulfonyl)-3-(2-ethoxy-pyridin-3-yl)-6-fluor-2-oxo-2,3-dihydro-1H-indol-5-carbonitril in 4 ml Pyridin tropfte man langsam 61 µl (0.49 mmol) Chlorameisensäurephenylester. Nach 5 Minuten wurde das Lösungsmittel verdampft. Der Rückstand wurde mit 20 ml Wasser versetzt und mit Diethlyether (2x) extrahiert. Die organische Phase wurde mit Wasser und gesättigter Kochsalzlösung gewaschen, über Magnesiumsulfat getrocknet und unter vermindertem Druck eingeengt. Der Rückstand wurde chromatographisch über Kieselgel (Redisep-Kartusche, Laufmittel-Gradient von 0 bis 5% Methanol in Ethylacetat) gereinigt. Man erhielt 200 mg der Titelverbindung.
ESI-MS: 633.15 [M+H]⁺

### 1.5 4-(1-Methyl-piperidin-4-yl)-piperazin-1-carbonsäure-[5-cyano-1-(2,4-dimethoxy-phenylsulfonyl)-3-(2-ethoxypyridin-3-yl)-6-fluor-2-oxo-2,3-dihydro-1 H-indol-3-yl]-amid

Man rührte eine Mischung aus 50 mg (0.07 mmol) [5-Cyano-1-(2,4-dimethoxy-phenylsulfonyl)-3-(2-ethoxypyridin-3-yl)-6-fluor-2-oxo-2,3-dihydro-1H-indol-3-yl]-carbaminsäurephenylester, 26 mg (0.14 mmol) 1-(1-Methylpiperidin-4-yl)-piperazin und 5 ml getrocknetem THF 2 Stunden bei Raumtemperatur. Man verdünnte das Reaktionsgemisch mit Dichlormethan, wusch mit Wasser und gesättigter Kochsalzlösung, trocknete die organische Phase über Magnesiumsulfat und engte unter vermindertem Druck ein. Der Rückstand wurde chromatographisch über Kieselgel (Redisep-Kartusche, Laufmittel-Gradient von 0 bis 20% Methanol in Dichlormethan) gereinigt. Man erhielt 27 mg der Titelverbindung als weißen Feststoff.
ESI-MS: 722.25 [M+H]⁺
¹H-NMR (500 MHz, d₆-DMSO): δ [ppm] 8.15 (m, 1 H); 7.85 (d, 1 H); 7.80 (d, 1 H); 7.70 (m, 2H); 7.65 (s, 1 H); 7.05 (m, 1 H); 6.70 (m, 2H); 4.15 (m, 2H); 3.85 (s, 3H); 3.50 (s, 3H); 3.20 (m, 4H); 2.80 (m, 2H); 2.35 (m, 4H); 2.15 (m, 4H); 1.85 (m, 2H); 1.65 (m, 2H); 1.40 (m, 2H); 1.10 (t, 3H).

### Racemattrennung von Verbindungen der Formel I

Die Trennung von racemischen Verbindungen der Formel I kann beispielsweise durch Separation auf einer präparativen chiralen Säule, z. B. Chiracel OD, erfolgen.

Die Drehwerte wurden bei einer Wellenlänge von 589 nm bei 22 °C in Chloroform als Lösungsmittel und einer Konzentration der Testsubstanz von 1 mg/ml bestimmt.

### BEISPIEL 1A und BEISPIEL 1B:

Racematspaltung von 4-(1-Methyl-piperidin-4-yl)-piperazin-1-carbonsäure-[5-cyano-1-(2,4-dimethoxy-phenylsulfonyl)-3-(2-ethoxypyridin-3-yl)-6-fluor-2-oxo-2,3-dihydro-1 H-indol-3-yl]-amid

4-(1-Methyl-piperidin-4-yl)-piperazin-1-carbonsäure-[5-cyano-1-(2,4-dimethoxyphenyl-sulfonyl)-3-(2-ethoxypyridin-3-yl)-6-fluor-2-oxo-2,3-dihydro-1H-indol-3-yl]-amid aus Beispiel 1 wurde über eine chirale präparative Säule (Chiralcel OD, Fluss 45 ml /min) mit Heptan : Ethanol : tert-Butanol im Verhältnis 14:6:1 als Elutionsmittel getrennt. Man erhielt das Enantiomer (Beispiel 1A) mit positivem Drehwert (Drehwert bestimmt in Chloroform) und das Enantiomer (Beispiel 1 B) mit negativem Drehwert (Drehwert bestimmt in Chloroform).

### BEISPIEL 1A:

(+)-4-(1-Methyl-piperidin-4-yl)-piperazin-1-carbonsäure- [5-cyano-1-(2,4-dimethoxy-phenylsulfonyl)-3-(2-ethoxypyridin-3-yl)-6-fluor-2-oxo-2,3-dihydro-1 H-indol-3-yl]-amid α (CHCl₃): plusdrehend
ESI-MS: 722.25 [M+H]+

### BEISPIEL 1B:

(-)-4-(1-Methyl-piperidin-4-yl)-piperazin-1-carbonsäure-[5-cyano-1-(2,4-dimethoxy-phenylsulfonyl)-3-(2-ethoxy-pyridin-3-yl)-6-fluor-2-oxo-2,3-dihydro-1H-indol-3-yl]-amid α (CHCl₃): minusdrehend
ESI-MS: 722.25 [M+H]⁺

### BEISPIELE 2 bis 37:

Die Verbindungen der Formel I gemäß den Beispielen 2 bis 37 wurden in Analogie zu Beispiel 1 unter Verwendung der entsprechenden Isatine II, Sulfonsäurechloride VII und Amine X hergestellt.

Die erfindungsgemäßen Verbindungen I, worin X¹ für NH steht, können auch durch Kristallisation und / oder durch präparative HPLC (RP, Eluenten Acetonitril / Wasser, 0.1 % TFA oder 0.1 % Essigsäure) gereinigt werden. Die Verbindungen I fallen dann gegebenenfalls als Trifluoressigsäure-Salz, Bis(trifluoressigsäure)-Salz bzw. Essigsäure-Salz an.

### BEISPIEL 2:

4-(1-Ethyl-piperidin-4-yl)-piperazin-1-carbonsäure-[5-cyano-1-(2,4-dimethoxy-phenylsulfonyl)-3-(2-ethoxy-pyridin-3-yl)-6-fluor-2-oxo-2,3-dihydro-1H-indol-3-yl]-amid
ESI-MS: 736.15 [M+H]⁺

### BEISPIEL 3:

4-(4-Methyl-piperazin-1-yl)-piperidin-1-carbonsäure-[5-cyano-1-(2,4-dimethoxy-phenylsulfonyl)-3-(2-ethoxy-pyridin-3-yl)-6-fluor-2-oxo-2,3-dihydro-1H-indol-3-yl]-amid
ESI-MS: 722.25 [M+H]⁺
¹H-NMR (500 MHz, d₆-DMSO): δ [ppm] 8.15 (m, 1 H); 7.90 (d, 1 H); 7.80 (d, 1 H); 7.70 (m, 2H); 7.65 (s, 1 H); 7.05 (m, 1 H); 6.70 (m, 2H); 4.20 (m, 2H); 3.85 (s, 3H); 3.80 (m, 2H); 3.50 (s, 3H); 2.65 (m, 2H); 2.45-2.25 (m, 9H); 2.15 (s, 3H); 1.60 (m, 2H); 1.15 (m, 5H).

### BEISPIEL 4:

4-(4-Ethyl-piperazin-1-yl)-piperidin-1-carbonsäure-[5-cyano-1-(2,4-dimethoxy-phenylsulfonyl)-3-(2-ethoxy-pyridin-3-yl)-6-fluor-2-oxo-2,3-dihydro-1H-indol-3-yl]-amid ESI-MS: 736.20 [M+H]⁺

### BEISPIEL 5:

### 4-(1-Methyl-piperidin-4-yl)-piperazin-1-carbonsäure-[5-cyano-3-(2-ethoxy-pyridin-3-yl)-6-fluor-1-(4-methoxy-phenylsulfonyl)-2-oxo-2,3-dihydro-1H-indol-3-yl]-amid

### 5.1 3-Amino-3-(2-ethoxy-pyridin-3-yl)-6-fluor-1-(4-methoxy-phenylsulfonyl)-2-oxo-2,3-dihydro-1H-indol-5-carbonitril

Die Titelverbindung wurde unter Verwendung von 3-(2-Ethoxy-pyridin-3-yl)-6-fluor-3-hydroxy-2-oxo-2,3-dihydro-1H-indol-5-carbonitril als 3-Hydroxy-1,3-dihydroindol-2-on IV und 4-Methoxyphenylsulfonylchlorid als Sulfonsäurechlorid VII in Analogie zu Beispiel 1.1 bis 1.3 hergestellt.
ESI-MS: 483.10 [M+H]⁺
¹H-NMR (500 MHz, d₆-DMSO): δ [ppm] 8.25 (d, 1H); 8.15 (d, 2H); 8.10 (m, 1H); 7.90 (d, 1 H); 7.45 (d, 1 H); 7.25 (d, 2H); 7.15 (m, 1 H); 3.95 (m, 1 H); 3.90 (s, 3H); 3.55 (m, 1 H); 3.05 (m, 2H); 0.55 (t, 3H).

### 5.2 [5-Cyano-3-(2-ethoxy-pyridin-3-yl)-6-fluor-1-(4-methoxy-phenylsulfonyl)-2-oxo-2,3-dihydro-1 H-indol-3-yl]-carbaminsäurephenylester

Die Herstellung erfolgte in Analogie zu Beispiel 1.4.
ESI-MS: 603.15 [M+H]⁺

### 5.3 4-(1-Methyl-piperidin-4-yl)-piperazin-1-carbonsäure-[5-cyano-3-(2-ethoxy-pyridin-3-yl)-6-fluor-1-(4-methoxy-phenylsulfonyl)-2-oxo-2,3-dihydro-1 H-indol-3-yl]-amid

Die Herstellung erfolgte in Analogie zu Beispiel 1.5.
ESI-MS: 692.20 [M+H]⁺
¹H-NMR (500 MHz, d₆-DMSO): δ [ppm] 8.15 (m, 1H); 8.00 (d, 2H); 7.95 (d, 1H); 7.75 (d, 1 H); 7.65 (m, 2H); 7.15 (d, 2H); 7.05 (m, 1 H); 4.10 (m, 1 H); 4.05 (m, 1 H); 3.85 (s, 3H); 3.75 (m, 2H); 2.60 (m, 2H); 2.45 (m, 4H); 2.30 (m, 4H); 2.15 (m, 4H); 1.65 (m, 2H); 1.15 (m, 2H); 1.05 (t, 3H).

### BEISPIEL 6:

4-(1-Ethyl-piperidin-4-yl)-piperazin-1-carbonsäure-[5-cyano-3-(2-ethoxy-pyridin-3-yl)-6-fluor-1-(4-methoxy-phenylsulfonyl)-2-oxo-2,3-dihydro-1H-indol-3-yl]-amid
ESI-MS: 706.25 [M+H]⁺

### BEISPIEL 7:

4-(4-Methyl-piperazin-1-yl)-piperidin-1-carbonsäure-[5-cyano-3-(2-ethoxy-pyridin-3-yl)-6-fluor-1-(4-methoxy-phenylsulfonyl)-2-oxo-2,3-dihydro-1H-indol-3-yl]-amid
ESI-MS: 692.15 [M+H]⁺
¹H-NMR (500 MHz, d₆-DMSO): δ [ppm] 8.15 (m, 1H); 8.05 (d, 2H); 8.00 (d, 1H); 7.75 (d, 1 H); 7.70 (s, 1 H); 7.65 (m, 1 H); 7.15 (d, 2H); 7.05 (m, 1 H); 4.10 (m, 1 H); 4.05 (m, 1 H); 3.85 (s, 3H); 3.20 (m, 4H); 2.80 (m, 4H); 2.35 (m, 2H); 2.15 (m, 4H); 1.90 (m, 2H); 1.65 (m, 2H); 1.40 (m, 2H); 1.05 (t, 3H).

### BEISPIEL 8:

4-(4-Ethyl-piperazin-1-yl)-piperidin-1-carbonsäure-[5-cyano-3-(2-ethoxy-pyridin-3-yl)-6-fluor-1-(4-methoxy-phenylsulfonyl)-2-oxo-2,3-dihydro-1H-indol-3-yl]-amid
ESI-MS: 706.15 [M+H]⁺

### BEISPIEL 9:

4-(1-Methyl-piperidin-4-yl)-piperazin-1-carbonsäure-[5-cyano-1-(2,4-dimethoxy-phenylsulfonyl)-3-(2-ethoxy-phenyl)-6-fluor-2-oxo-2,3-dihydro-1 H-indol-3-yl]-amid als Trifluoressigsäure-Salz
ESI-MS: 721.20 [M+H]⁺
¹H-NMR (500 MHz, d₆-DMSO): δ [ppm] 10.35 (bs, 1 H); 8.00 (s, 1 H); 7.90 (d, 1 H); 7.75 (m, 2H); 7.45 (d, 1 H); 7.35 (t, 1 H); 7.00 (m, 2H); 6.70 (m, 2H); 4.05 (m, 1 H); 3.90 (m, 4H); 3.65-3.40 (m, 9H); 3.30-2.95 (m, 7H); 2.80 (s, 3H); 2.30 (m, 2H); 1.90 (m, 2H); 1.15 (t, 3H).

### BEISPIEL 10:

4-(1-Methyl-piperidin-4-yl)-piperazin-1-carbonsäure-[5-cyano-3-(2-ethoxyphenyl)-6-fluor-1-(4-methoxy-phenylsulfonyl)-2-oxo-2,3-dihydro-1H-indol-3-yl]-amid als Trifluoressigsäuresalz
ESI-MS: 691.20 [M+H]⁺
¹H-NMR (500 MHz, CH₃OD): δ [ppm] 8.05 (d, 2H); 7.85 (d, 1 H); 7.55 (d, 1 H); 7.45 (d, 1 H); 7.35 (t, 1 H); 7.10 (d, 2H); 7.05 (t, 1 H); 7.00 (d, 1 H); 4.05 (m, 1 H); 3.95 (m, 1 H); 3.90 (s, 3H); 3.70 (m, 2H); 3.60 (m, 4H); 3.45 (m, 1 H); 3.25 (m, 4H); 3.15 (m, 2H); 2.95 (s, 3H); 2.40 (m, 2H); 2.10 (m, 2H); 1.25 (t, 3H).

### BEISPIEL 11:

4-(1-Ethyl-piperidin-4-yl)-piperazin-1-carbonsäure-[5-cyano-1-(2,4-dimethoxy-phenylsulfonyl)-3-(2-ethoxyphenyl)-6-fluor-2-oxo-2,3-dihydro-1H-indol-3-yl]-amid
¹H-NMR (500 MHz, d₆-DMSO): δ [ppm] 7.90 (d, 1 H); 7.70 (m, 2H); 7.60 (s, 1 H); 7.35 (m, 2H); 6.95 (m, 2H); 6.70 (m, 2H); 4.05 (m, 1 H); 3.90 (m, 4H); 3.50 (bs, 3H); 3.20 (m, 4H); 3.00 (m, 2H); 2.45 (m, 2H); 2.35 (m, 4H); 2.20 (m, 1 H); 2.05 (m, 2H); 1.75 (m, 2H); 1.45 (m, 2H); 1.15 (t, 3H); 1.05 (t, 3H).

### BEISPIEL 12:

4-(1-Ethyl-piperidin-4-yl)-piperazin-1-carbonsäure-[5-cyano-3-(2-ethoxyphenyl)-6-fluor-1-(4-methoxy-phenylsulfonyl)-2-oxo-2,3-dihydro-1H-indol-3-yl]-amid als Trifluoressigsäure-Salz
ESI-MS: 705.20 [M+H]⁺
¹H-NMR (500 MHz, d₆-DMSO): δ [ppm] 10.05 (bs, 1 H); 8.05 (m, 3H); 7.75 (d, 1 H); 7.65 (m, 2H); 7.35 (t, 1 H); 7.15 (d, 2H); 7.00 (t, 1 H); 6.95 (d, 1 H); 3.95-2.90 (m, 20H); 2.30 (m, 2H); 1.90 (m, 2H); 1.25 (t, 3H); 1.10 (t, 3H).

### BEISPIEL 13:

4-(1-Ethyl-piperidin-4-yl)-piperazin-1-carbonsäure-[1-phenylsulfonyl-5-cyano-3-(2-ethoxyphenyl)-6-fluor-2-oxo-2,3-dihydro-1H-indol-3-yl]-amid als Trifluoressigsäure-Salz
¹H-NMR (500 MHz, d₆-DMSO): δ [ppm] 10.10 (bs, 1 H); 8.10 (d, 2H); 8.05 (s, 1 H); 7.80 (m, 2H); 7.65 (m, 4H); 7.35 (t, 1 H); 7.05 (t, 1 H); 6.95 (d, 1 H); 3.95-3.75 (m, 3H); 3.65 (m, 3H); 3.45-2.90 (m, 11H); 2.30 (m, 2H); 1.95 (m, 2H); 1.25 (t, 3H); 1.10 (t, 3H).

### BEISPIEL 14:

4-(4-Methyl-piperazin-1-yl)-piperidin-1-carbonsäure-[5-cyano-1-(2,4-dimethoxy-phenylsulfonyl)-3-(2-ethoxyphenyl)-6-fluor-2-oxo-2,3-dihydro-1H-indol-3-yl]-amid als Trifluoressigsäuresalz
ESI-MS: 721.20 [M+H]⁺
¹H-NMR (500 MHz, CH₃OD): δ [ppm] 8.00 (d, 1 H); 7.85 (d, 1 H); 7.65 (d, 1 H); 7.35 (t, 1 H); 7.25 (d, 1 H); 7.00 (d, 1 H); 6.95 (t, 1 H); 6.65 (d, 1 H); 6.60 (s, 1 H); 4.15 (m, 1 H); 4.05 (m, 3H); 3.90 (s, 3H); 3.65 (m, 4H); 3.55 (m, 6H); 3.35 (m, 2H); 2.95 (s, 3H); 2.80 (m, 2H); 2.05 (m, 2H); 1.55 (m, 2H); 1.30 (t, 3H).

### BEISPIEL 15:

4-(4-Methyl-piperazin-1-yl)-piperidin-1-carbonsäure-[5-cyano-3-(2-ethoxyphenyl)-6-fluor-1-(4-methoxy-phenylsulfonyl)-2-oxo-2,3-dihydro-1H-indol-3-yl]-amid als Trifluoressigsäure-salz
ESI-MS: 691.20 [M+H]⁺
¹H-NMR (500 MHz, d₆-DMSO): δ [ppm] 8.00 (d, 2H); 7.75 (m, 2H); 7.60 (m, 2H); 7.35 (t, 1 H); 7.15 (d, 2H); 7.00 (t, 1 H); 6.95 (d, 1 H); 3.95-3.80 (m, 7H); 3.55-3.05 (m, 8H); 2.85 (m, 4H); 2.60 (m, 2H); 1.90 (m, 2H); 1.30 (m, 2H); 1.10 (t, 3H).

### BEISPIEL 16:

4-(4-Ethyl-piperazin-1-yl)-piperidin-1-carbonsäure-[5-cyano-1-(2,4-dimethoxy-phenylsulfonyl)-3-(2-ethoxyphenyl)-6-fluor-2-oxo-2,3-dihydro-1H-indol-3-yl]-amid als Trifluoressigsäuresalz
ESI-MS: 735.25 [M+H]⁺
¹H-NMR (500 MHz, d₆-DMSO): δ [ppm] 7.90 (d, 1 H); 7.75 (m, 3H); 7.40 (d, 1 H); 7.35 (t, 1 H); 6.95 (m, 2H); 6.70 (m, 2H); 4.05 (m, 1 H); 3.95-3.85 (m, 6H); 3.55-2.95 (m, 14H); 2.65 (m, 2H); 1.85 (m, 2H); 1.30 (m, 2H); 1.20 (m, 6H).

### BEISPIEL 17:

4-(4-Ethyl-piperazin-1-yl)-piperidin-1-carbonsäure-[5-cyano-3-(2-ethoxyphenyl)-6-fluor-1-(4-methoxy-phenylsulfonyl)-2-oxo-2,3-dihydro-1H-indol-3-yl]-amid als Trifluoressigsäure-salz
ESI-MS: 705.20 [M+H]⁺
¹H-NMR (500 MHz, d₆-DMSO): δ [ppm] 8.00 (d, 2H); 7.80 (s, 1H); 7.75 (d, 1 H); 7.60 (m, 2H); 7.35 (t, 1 H); 7.15 (d, 2H); 7.00 (t, 1 H); 6.95 (d, 1 H); 3.95-3.80 (m, 7H); 3.70-3.15 (m, 11H); 2.65 (m, 2H); 1.95 (m, 2H); 1.35 (m, 2H); 1.25 (t, 3H); 1.10 (t, 3H).

### BEISPIEL 18:

4-(4-Ethyl-piperazin-1-yl)-piperidin-1-carbonsäure-[1-phenylsulfonyl-5-cyano-3-(2-ethoxyphenyl)-6-fluor-2-oxo-2,3-dihydro-1 H-indol-3-yl]-amid als Trifluoressigsäuresalz
¹H-NMR (500 MHz, d₆-DMSO): δ [ppm] 8.10 (d, 2H); 7.85 (s, 1H); 7.80 (m, 2H); 7.65 (m, 4H); 7.35 (t, 1 H); 7.00 (t, 1 H); 6.95 (d, 1 H); 4.25 (m, 2H); 3.95 (m, 2H); 3.85 (m, 2H); 3.70-3.15 (m, 11 H); 2.65 (m, 2H); 1.95 (m, 2H); 1.35 (m, 2H); 1.25 (t, 3H); 1.10 (t, 3H).

### BEISPIEL 19:

### 4-(1-Methyl-piperidin-4-yl)-piperazin-1-carbonsäure-[5-chlor-1-(2,4-dimethoxy-phenylsulfonyl)-3-(2-ethoxy-pyridin-3-yl)-6-fluor-2-oxo-2,3-dihydro-1H-indol-3-yl]-amid

### 19.1 3-Amino-5-chlor-1-(2,4-dimethoxy-phenylsulfonyl)-3-(2-ethoxy-pyridin-3-yl)-6-fluor-1,3-dihydro-indol-2-on

Man stellte 3-Amino-5-chlor-1-(2,4-dimethoxy-phenylsulfonyl)-3-(2-ethoxy-pyridin-3-yl)-6-fluor-1,3-dihydro-indol-2-on in Analogie zu Beispiel 1.1 bis 1.3 unter Verwendung von 5-Chlor-3-(2-ethoxypyridin-3-yl)-6-fluor-3-hydroxy-1,3-dihydroindol-2-on als 3-Hydroxy-1,3-dihydroindol-2-on IV und 2,4-Dimethoxyphenylsulfonylchlorid als Sulfonsäurechlorid VII her. 5-Chlor-3-(2-ethoxypyridin-3-yl)-6-fluor-3-hydroxy-1,3-dihydroindol-2-on wurde in Analogie zu Beispiel a.1 hergestellt.
ESI-MS: 522.10 [M+H]⁺

### 19.2 [5-Chlor-1-(2,4-dimethoxy-phenylsulfonyl)-3-(2-ethoxy-pyridin-3-yl)-6-fluor-2-oxo-2,3-dihydro-1H-indol-3-yl]-carbaminsäurephenylester

Man stellte die Titelverbindung in Analogie zu Beispiel 1.4 her.
ESI-MS: 642.10 [M+H]⁺

### 19.3 4-(1-Methyl-piperidin-4-yl)-piperazin-1-carbonsäure-[5-chlor-1-(2,4-dimethoxy-phenylsulfonyl)-3-(2-ethoxypyridin-3-yl)-6-fluor-2-oxo-2,3-dihydro-1 H-indol-3-yl]-amid

Man stellte die Titelverbindung in Analogie zu Beispiel 1.5 her.
ESI-MS: 731.20 [M+H]⁺

### BEISPIEL 20:

### 4-(1-Methyl-piperidin-4-yl)-piperazin-1-carbonsäure-[1-phenylsulfonyl-5-chlor-3-(2-ethoxy-pyridin-3-yl)-6-fluor-2-oxo-2,3-dihydro-1H-indol-3-yl]-amid

### 20.1 3-Amino-1-phenylsulfonyl-5-chlor-3-(2-ethoxy-pyridin-3-yl)-6-fluor-1,3-dihydro-indol-2-on

Man stellte die Titelverbindung in Analogie zu Beispiel 1.1 bis 1.3 unter Verwendung von 5-Chlor-3-(2-ethoxypyridin-3-yl)-6-fluor-3-hydroxy-1,3-dihydroindol-2-on als 3-Hydroxy-1,3-dihydroindol-2-on IV und Phenylsulfonylchlorid als Sulfonsäurechlorid VII her. 5-Chlor-3-(2-ethoxypyridin-3-yl)-6-fluor-3-hydroxy-1,3-dihydroindol-2-on wurde in Analogie zu Beispiel a.1 hergestellt.
ESI-MS: 462.00 [M+H]⁺

### 20.2 [1-Phenylsulfonyl-5-chlor-3-(2-ethoxy-pyridin-3-yl)-6-fluor-2-oxo-2,3-dihydro-1H-indol-3-yl]-carbaminsäurephenylester

Man stellte die Titelverbindung in Analogie zu Beispiel 1.4 her.
ESI-MS: 582.00 [M+H]⁺

### 20.3 4-(1-Methyl-piperidin-4-yl)-piperazin-1-carbonsäure-[1-phenylsulfonyl-5-chlor-3-(2-ethoxy-pyridin-3-yl)-6-fluor-2-oxo-2,3-dihydro-1H-indol-3-yl]-amid

Man stellte die Titelverbindung in Analogie zu Beispiel 1.5 her.
ESI-MS: 671.05 [M+H]⁺

### BEISPIEL 21:

4-(1-Ethyl-piperidin-4-yl)-piperazin-1-carbonsäure-[5-chlor-1-(2,4-dimethoxy-phenylsulfonyl)-3-(2-ethoxy-pyridin-3-yl)-6-fluor-2-oxo-2,3-dihydro-1H-indol-3-yl]-amid
¹H-NMR (500 MHz, d₆-DMSO): δ [ppm] 8.15 (m, 1 H); 7.90 (d, 1 H); 7.65 (m, 2H); 7.60 (s, 1 H); 7.50 (d, 1 H); 7.00 (m, 1 H); 6.70 (m, 2H); 4.20 (m, 2H); 3.85 (s, 3H); 3.50 (s, 3H); 3.25 (m, 4H); 2.90 (m, 2H); 2.35 (m, 6H); 2.15 (m, 1 H); 1.85 (m, 2H); 1.65 (m, 2H); 1.35 (m, 2H); 1.15 (t, 3H); 0.95 (t, 3H).

### BEISPIEL 22:

4-(1-Ethyl-piperidin-4-yl)-piperazin-1-carbonsäure-[1-phenylsulfonyl-5-chlor-3-(2-ethoxy-pyridin-3-yl)-6-fluor-2-oxo-2,3-dihydro-1H-indol-3-yl]-amid
ESI-MS: 685.20 [M+H]⁺

### BEISPIEL 23:

4-(4-Methyl-piperazin-1-yl)-piperidin-1-carbonsäure-[5-chlor-1-(2,4-dimethoxy-phenylsulfonyl)-3-(2-ethoxy-pyridin-3-yl)-6-fluor-2-oxo-2,3-dihydro-1H-indol-3-yl]-amid
ESI-MS: 731.20 [M+H]⁺

### BEISPIEL 24:

4-(4-Methyl-piperazin-1-yl)-piperidin-1-carbonsäure-[1-phenylsulfonyl-5-chlor-3-(2-ethoxy-pyridin-3-yl)-6-fluor-2-oxo-2,3-dihydro-1H-indol-3-yl]-amid
ESI-MS: 671.10 [M+H]⁺
¹H-NMR (500 MHz, d₆-DMSO): δ [ppm] 8.15 (m, 1H); 8.10 (d, 2H); 7.90 (d, 1H); 7.80-7.60 (m, 5H); 7.40 (d, 1 H); 7.05 (m, 1 H); 4.15 (m, 1 H); 4.05 (m, 1 H); 3.80 (m, 2H); 2.60 (m, 2H); 2.50-2.25 (m, 9H); 2.20 (s, 3H); 1.65 (m, 2H); 1.15 (m, 2H); 1.05 (t, 3H).

### BEISPIEL 25:

4-(4-Ethyl-piperazin-1-yl)-piperidin-1-carbonsäure-[1-phenylsulfonyl-5-chlor-3-(2-ethoxy-pyridin-3-yl)-6-fluor-2-oxo-2,3-dihydro-1H-indol-3-yl]-amid
ESI-MS: 685.20 [M+H]⁺

### BEISPIEL 26:

1'-Methyl-[4,4']bipiperidinyl-1-carbonsäure-[5-chlor-1-(2,4-dimethoxy-phenylsulfonyl)-3-(2-ethoxy-pyridin-3-yl)-6-fluor-2-oxo-2,3-dihydro-1H-indol-3-yl]-amid ESI-MS:

### BEISPIEL 27:

1'-Ethyl-[4,4']bipiperidinyl-1-carbonsäure-[5-chlor-1-(2,4-dimethoxy-phenylsulfonyl)-3-(2-ethoxy-pyridin-3-yl)-6-fluor-2-oxo-2,3-dihydro-1H-indol-3-yl]-amid
ESI-MS: 744.15 [M+H]⁺

### BEISPIEL 28:

4-(4-Methyl-piperazin-1-yl)-piperidin-1-carbonsäure-[5-chlor-3-[2-(2,2-difluor-ethoxy)-phenyl]-1-(2,4-dimethoxy-phenylsulfonyl)-6-fluor-2-oxo-2,3-dihydro-1H-indol-3-yl]-amid
ESI-MS: 766.20 [M+H]⁺
¹H-NMR (500 MHz, d₆-DMSO): δ [ppm] 8.00 (d, 1H); 7.75 (m, 2H); 7.65 (s, 1 H); 7.45 (m, 1 H); 7.20 (d, 1 H); 7.05 (d, 1 H); 7.00 (t, 1 H); 6.80 (m, 2H); 6.45 (t, J = 70 Hz, 1 H); 4.55 (m, 1 H); 4.40 (m, 1 H); 3.95 (s, 3H); 3.90 (m, 2H); 3.55 (s, 3H); 2.70 (m, 2H); 2.55 (m, 4H); 2.40 (m, 5H); 2.20 (s, 3H); 1.70 (m, 2H); 1.25 (m, 2H).

### BEISPIEL 29:

4-(1-Methyl-piperidin-4-yl)-piperazin-1-carbonsäure-[5-chlor-3-[2-(2,2-difluor-ethoxy)-phenyl]-1-(2,4-dimethoxy-phenylsulfonyl)-6-fluor-2-oxo-2,3-dihydro-1 H-indol-3-yl]-amid
ESI-MS: 766.20 [M+H]⁺
¹H-NMR (500 MHz, d₆-DMSO): δ [ppm] 7.95 (d, 1H); 7.65 (m, 2H); 7.60 (s, 1 H); 7.35 (t, 1 H); 7.10 (d, 1 H); 6.95 (m, 1 H); 6.90 (t, 1 H); 6.75 (d, 1 H); 6.70 (m, 1 H); 6.35 (t, J = 70 Hz, 1 H); 4.45 (m, 1 H); 4.30 (m, 1 H); 3.85 (s, 3H); 3.45 (s, 3H); 3.20 (m, 4H); 2.75 (m, 2H); 2.35 (m, 4H); 2.15 (m, 4H); 1.85 (m, 2H); 1.65 (m, 2H); 1.40 (m, 2H).

### BEISPIEL 30:

1'-Methyl-[4,4']bipiperidinyl-1-carbonsäure-[5-chlor-3-[2-(2,2-difluorethoxy)-phenyl]-1-(2,4-dimethoxy-phenylsulfonyl)-6-fluor-2-oxo-2,3-dihydro-1 H-indol-3-yl]-amid
ESI-MS: 765.20 [M+H]⁺
¹H-NMR (500 MHz, d₆-DMSO): δ [ppm] 7.90 (d, 1H); 7.65 (m, 2H); 7.55 (s, 1 H); 7.35 (t, 1 H); 7.10 (d, 1 H); 6.95 (m, 1 H); 6.90 (t, 1 H); 6.75 (d, 1 H); 6.70 (s, 1 H); 6.35 (t, J = 70 Hz, 1 H); 4.45 (m, 1 H); 4.30 (m, 1 H); 3.90 (s, 3H); 3.85 (m, 2H); 3.45 (s, 3H); 2.80 (m, 2H); 2.55 (m, 2H); 2.15 (s, 3H); 1.85 (m, 2H); 1.55 (m, 4H); 1.15 (m, 3H), 0.95 (m, 3H).

### BEISPIEL 31:

4-(1-Methyl-piperidin-4-yl)-piperazin-1-carbonsäure-[1-(2,4-dimethoxy-phenylsulfonyl)-3-(2-ethoxy-pyridin-3-yl)-5,6-difluor-2-oxo-2,3-dihydro-1H-indol-3-yl]-amid als Trifluoressigsäuresalz
ESI-MS: 715.20 [M+H]⁺
¹H-NMR (500 MHz, CDCl₃): δ [ppm] 8.05 (m, 2H); 7.85 (m, 1 H); 7.25 (t, 1 H); 7.10 (m, 2H); 6.75 (m, 1 H); 6.60 (d, 1 H); 6.40 (s, 1 H); 4.55 (m, 2H); 3.85 (s, 3H); 3.75-3.50 (m, 10H); 3.10 (m, 4H); 2.90 (m, 2H); 2.80 (s, 3H); 2.45 (m, 4H); 2.15 (m, 2H); 1.45 (t, 3H).

### BEISPIEL 32:

4-(1-Methyl-piperidin-4-yl)-piperazin-1-carbonsäure-[1-phenylsulfonyl-3-(2-ethoxy-pyridin-3-yl)-5,6-difluor-2-oxo-2,3-dihydro-1H-indol-3-yl]-amid
ESI-MS: 655.20 [M+H]⁺
¹H-NMR (500 MHz, CDCl₃): δ [ppm] 8.10 (m, 3H); 7.80 (m, 1 H); 7.65 (m, 1 H); 7.50 (m, 2H); 7.20 (d, 1 H); 7.15 (t, 1 H); 6.80 (m, 1 H); 6.70 (s, 1 H); 4.55 (m, 2H); 3.20 (m, 4H); 2.90 (m, 2H); 2.45 (m, 4H); 2.25 (m, 4H); 1.95 (m, 2H); 1.75 (m, 2H); 1.60 (m, 2H); 1.45 (t, 3H).

### BEISPIEL 33:

4-(1-Ethyl-piperidin-4-yl)-piperazin-1-carbonsäure-[1-(2,4-dimethoxy-phenylsulfonyl)-3-(2-ethoxy-pyridin-3-yl)-5,6-difluor-2-oxo-2,3-dihydro-1H-indol-3-yl]-amid
ESI-MS: 729.30 [M+H]⁺
¹H-NMR (500 MHz, d₆-DMSO): δ [ppm] 8.10 (m, 1H); 7.90 (d, 1 H); 7.75 (m, 2H); 7.70 (s, 1 H); 7.35 (t, 1 H); 7.00 (m, 1 H); 6.70 (d, 1 H); 6.65 (m, 1 H); 4.20 (m, 2H); 3.85 (s, 3H); 3.50 (s, 3H); 3.20 (m, 4H); 2.90 (m, 2H); 2.35 (m, 6 H); 2.15 (m, 1 H); 1.85 (m, 2H); 1.70 (m, 2H); 1.35 (m, 2H); 1.10 (t, 3H); 0.95 (t, 3H).

### BEISPIEL 34:

4-(1-Ethyl-piperidin-4-yl)-piperazin-1-carbonsäure-[1-phenylsulfonyl-3-(2-ethoxy-pyridin-3-yl)-5,6-difluor-2-oxo-2,3-dihydro-1H-indol-3-yl]-amid
ESI-MS: 669.20 [M+H]⁺

### BEISPIEL 35:

4-(4-Methyl-piperazin-1-yl)-piperidin-1-carbonsäure-[1-(2,4-dimethoxy-phenylsulfonyl)-3-(2-ethoxy-pyridin-3-yl)-5,6-difluor-2-oxo-2,3-dihydro-1H-indol-3-yl]-amid als Trifluoressigsäuresalz
ESI-MS: 715.20 [M+H]⁺
¹H-NMR (500 MHz, CDCl₃): δ [ppm] 8.05 (m, 2H); 7.80 (m, 1 H); 7.25 (t, 1 H); 7.00 (m, 2H); 6.75 (m, 1 H); 6.60 (d, 1 H); 6.40 (s, 1 H); 4.50 (m, 2H); 3.95 (m, 2H); 3.85 (s, 3H); 3.60 (m, 8H); 3.55 (s, 3H); 3.25 (m, 1 H); 3.35 (s, 3H); 2.25 (m, 2H); 2.00 (m, 2H); 1.60 (m, 2H); 1.45 (t, 3H).

### BEISPIEL 36:

4-(4-Methyl-piperazin-1-yl)-piperidin-1-carbonsäure-[1-phenylsulfonyl-3-(2-ethoxy-pyridin-3-yl)-5,6-difluor-2-oxo-2,3-dihydro-1H-indol-3-yl]-amid
ESI-MS: 655.20 [M+H]⁺
¹H-NMR (500 MHz, CDCl₃): δ [ppm] 8.10 (m, 3H); 7.80 (m, 1 H); 7.65 (m, 1 H); 7.55 (m, 2H); 7.30 (d, 1 H); 7.25 (t, 1 H); 6.80 (m, 1 H); 6.70 (s, 1 H); 4.55 (m, 2H); 3.75 (m, 2H); 2.70 (m, 2H); 2.60-2.40 (m, 8H); 2.35 (m, 1 H); 2.30 (s, 3H); 1.75 (m, 2H); 1.45 (t, 3H); 1.35 (m, 2H).

### BEISPIEL 37:

4-(4-Ethyl-piperazin-1-yl)-piperidin-1-carbonsäure-[1-(2,4-dimethoxy-phenylsulfonyl)-3-(2-ethoxy-pyridin-3-yl)-5,6-difluor-2-oxo-2,3-dihydro-1H-indol-3-yl]-amid als Trifluoressigsäuresalz
ESI-MS: 729.20 [M+H]⁺
¹H-NMR (500 MHz, d₆-DMSO): δ [ppm] 8.15 (m, 1 H); 7.90 (d, 1 H); 7.70 (s, 1 H); 7.65 (m, 1 H); 7.60 (d, 1 H); 7.35 (t, 1 H); 7.00 (m, 1 H); 6.70 (m, 2H); 4.20 (m, 2H); 3.95 (m, 2H); 3.85 (s, 3H); 3.55-2.85 (m, 14H); 2.65 (m, 2H); 1.85 (m, 2H); 1.30 (m, 2H); 1.20 (t, 3H); 1.15 (t, 3H).

### II.2 Verbindungen der Formel I, worin X¹ für O steht (Beispiele 38 bis 55)

### BEISPIEL 38:

4-(1-Methyl-piperidin-4-yl)-piperazine-1-carbonsäure-[5-cyano-1-(2,4-dimethoxy-benzenesulfonyl)-3-(2-ethoxypyridin-3-yl)-6-fluor-2-oxo-2,3-dihydro-1H-indol-3-yl]-ester

### 38.1 5-Cyano-3-(2-ethoxy-pyridin-3-yl)-6-fluor-2-oxo-3-phenoxycarbonyloxy-2,3-dihydro-indol-1-carbonsäurephenylester

Zu einer auf 0 °C gekühlten Lösung von 342 mg (0.66 mmol; 60 % Reinheit) 3-(2-Ethoxy-pyridin-3-yl)-6-fluor-3-hydroxy-2-oxo-2,3-dihydro-1H-indol-5-carbonitril (Mutterlauge aus Beispiel a.2) in 8 ml Pyridin tropfte man langsam 330 µl (2.62 mmol) Chlorameisensäurephenylester und rührte die Reaktionsmischung über Nacht bei Raumtemperatur nach. Man verdünnte das Reaktionsgemisch mit Dichlormethan und extrahierte mit Wasser. Man wusch die organische Phase mit Wasser und gesättigter Kochsalzlösung, trocknete über Magnesiumsulfat und engte unter vermindertem Druck ein. Der Rückstand wurde chromatographisch über Kieselgel (Redisep-Kartusche, Laufmittel-Gradient von 0 bis 5% Methanol in Dichlormethan) gereinigt. Man erhielt 291 mg der Titelverbindung.
ESI-MS: 554.15 [M+H]⁺

### 38.2 4-(1-Methyl-piperidin-4-yl)-piperazin-1-carbonsäure-[5-cyano-3-(2-ethoxy-pyridin-3-yl)-6-fluor-2-oxo-2,3-dihydro-1H-indol-3-yl]-ester

Eine Mischung aus 291 mg (0.53 mmol) 5-Cyano-3-(2-ethoxy-pyridin-3-yl)-6-fluor-2-oxo-3-phenoxycarbonyloxy-2,3-dihydro-indol-1-carbonsäurephenylester, 289 mg (1.58 mmol) 1-(1-Methylpiperidin-4-yl)-piperazin und 6 ml getrocknetes Tetrahydrofuran (THF) wurde über Nacht bei Raumtemperatur gerührt. Das Reaktionsgemisch wurde unter vermindertem Druck eingeengt und aus Methanol ausgerührt. Der ausgefallene Feststoff wurde abfiltriert und im Vakuumtrockenschrank getrocknet. Man erhielt 158 mg der Titelverbindung als weißen Feststoff.
ESI-MS: 523.20 [M+H]⁺

### 38.3 4-(1-Methyl-piperidin-4-yl)-piperazin-1-carbonsäure-[5-cyano-1-(2,4-dimethoxy-phenylsulfonyl)-3-(2-ethoxypyridin-3-yl)-6-fluor-2-oxo-2,3-dihydro-1 H-indol-3-yl]-ester

Zu einer Lösung von 50 mg (0.10 mmol) 4-(1-Methyl-piperidin-4-yl)-piperazin-1-carbonsäure-5-cyano-3-(2-ethoxy-pyridin-3-yl)-6-fluor-2-oxo-2,3-dihydro-1H-indol-3-yl-ester in 2 ml wasserfreiem Dimethylformamid gab man unter Stickstoffatmosphäre und unter Kühlung durch ein Eisbad 9.2 mg (0.19 mmol) Natriumhydrid (50% Dispersion in Mineralöl). Man rührte 10 min. bei 0 °C, gab anschließend 29 mg (0.12 mmol) 2,4-Dimethoxyphenylsulfonylchlorid zu und rührte weitere 30 min bei Raumtemperatur. Man goss die Reaktionsmischung in Eiswasser und extrahierte anschließend mit Ethylacetat. Die organische Phase wusch man mit gesättigter Natriumchlorid-Lösung, trocknete über Magnesiumsulfat und verdampfte das Lösungsmittel. Der Rückstand wurde chromatographisch über Kieselgel (Redisep-Kartusche, Laufmittel-Gradient von 0 bis 20% Methanol in Dichlormethan) gereinigt. Man erhielt 34 mg der Titelverbindung als weißen Feststoff.
ESI-MS: 723.25 [M+H]⁺
¹H-NMR (500 MHz, d₆-DMSO): δ [ppm] 8.15 (m, 2H); 7.90 (d, 1 H); 7.85 (m, 2H); 7.15 (m, 1 H); 6.70 (m, 2H); 4.15 (m, 2H); 3.85 (s, 3H); 3.55 (s, 3H); 3.05 (m, 2H); 2.80 (m, 2H); 2.45 (m, 2H); 2.30 (m, 2H); 2.15 (m, 4H); 1.90 (m, 2H); 1.65 (m, 2H); 1.40 (m, 2H); 1.05 (t, 3H).

### BEISPIELE 39 bis 55:

Die Verbindungen der Formel I, worin X¹ für O steht, gemäß den Beispielen 39 bis 55 wurden in Analogie zu Beispiel 38 unter Verwendung der entsprechenden 3-Hydroxy-1,3-dihydroindol-2-one der Formel IV, Sulfonsäurechloride VII und Amine X hergestellt.

### BEISPIEL 39:

4-(1-Methyl-piperidin-4-yl)-piperazin-1-carbonsäure-[5-cyano-3-(2-ethoxy-pyridin-3-yl)-6-fluor-1-(4-methoxy-phenylsulfonyl)-2-oxo-2,3-dihydro-1H-indol-3-yl]-ester
ESI-MS: 693.25 [M+H]⁺
¹H-NMR (500 MHz, CDCl₃): δ [ppm] 8.15 (m, 1 H); 8.05 (d, 2H); 7.95 (d, 1 H); 7.90 (d, 1 H); 7.20 (d, 1 H); 7.00 (m, 3H); 4.25 (m, 2H); 3.85 (s, 3H); 3.55 (m, 2H); 3.10 (m, 1 H); 3.05 (m, 1 H); 2.95 (m, 2H); 2.60 (m, 1 H); 2.50 (m, 1 H); 2.40 (m, 2H); 2.30 (m, 4H); 2.05 (m, 2H); 1.75 (m, 2H); 1.65 (m, 2H); 1.20 (t, 3H).

### BEISPIEL 40:

4-(1-Methyl-piperidin-4-yl)-piperazin-1-carbonsäure-[1-phenylsulfonyl-5-cyano-3-(2-ethoxy-pyridin-3-yl)-6-fluor-2-oxo-2,3-dihydro-1H-indol-3-yl]-ester
ESI-MS: 663.25 [M+H]⁺
¹H-NMR (500 MHz, d₆-DMSO): δ [ppm] 8.15 (m, 1H); 8.10 (d, 2H); 7.90 (d, 1H); 7.85 (d, 1 H); 7.70 (m, 2H); 7.60 (m, 1 H); 7.30 (m, 1 H); 7.15 (m, 1 H); 4.10 (m, 1 H); 3.95 (m, 1 H); 3.60 (m, 2H); 3.30 (m, 2H); 3.00 (m, 2H); 2.75 (m, 1 H); 2.65 (s, 3H); 2.45 (m, 3H); 2.35 (m, 2H); 1.85 (m, 2H); 1.65 (m, 2H); 0.95 (t, 3H).

### BEISPIEL 41:

4-(1-Methyl-piperidin-4-yl)-piperazin-1-carbonsäure-[5-chlor-1-(2,4-dimethoxy-phenylsulfonyl)-3-(2-ethoxy-pyridin-3-yl)-6-fluor-2-oxo-2,3-dihydro-1H-indol-3-yl]-ester

### 41.1 5-Chlor-3-(2-ethoxy-pyridin-3-yl)-6-fluor-2-oxo-3-phenoxycarbonyloxy-2,3-dihydro-indol-1-carbonsäurephenylester

ESI-MS: 563.10 [M+H]⁺ 41.2 4-(1-Methyl-piperidin-4-yl)-piperazin-1-carbonsäure-[5-chloro-3-(2-ethoxy-pyridin-3-yl)-6-fluoro-2-oxo-2,3-dihydro-1H-indol-3-yl]-ester
ESI-MS:

### 41.3 4-(1-Methyl-piperidin-4-yl)-piperazin-1-carbonsäure-[5-chlor-1-(2,4-dimethoxy-phenylsulfonyl)-3-(2-ethoxy-pyridin-3-yl)-6-fluor-2-oxo-2,3-dihydro-1H-indol-3-yl]-ester

ESI-MS: 732.25 [M+H]⁺
¹H-NMR (400 MHz, CH₃OD): δ [ppm] 8.15 (m, 2H); 7.95 (d, 1 H); 7.85 (d, 1 H); 7.20 (d, 1 H); 7.10 (t, 1 H); 6.65 (m, 2H); 4.25 (m, 2H); 3.90 (s, 3H); 3.65 (m, 5H); 3.20 (m, 2H); 2.95 (m, 2H); 2.60 (m, 2H); 2.45 (m, 2H); 2.30 (m, 4H); 2.05 (m, 2H); 1.85 (m, 2H); 1.55 (m, 2H); 1.20 (t, 3H).

### BEISPIEL 42:

4-(1-Methyl-piperidin-4-yl)-piperazin-1-carbonsäure-[5-chlor-3-(2-ethoxy-pyridin-3-yl)-6-fluor-1-(4-methoxy-phenylsulfonyl)-2-oxo-2,3-dihydro-1H-indol-3-yl]-ester
ESI-MS: 702.20 [M+H]⁺
¹H-NMR (500 MHz, CDCl₃): δ [ppm] 8.15 (m, 1 H); 8.05 (m, 2H); 7.95 (d, 1 H); 7.85 (d, 1 H); 6.95 (m, 4H); 4.25 (m, 2H); 3.85 (s, 3H); 3.55 (m, 2H); 3.10 (m, 2H); 2.90 (m, 2H); 2.65-2.35 (m, 4H); 2.25 (m, 4H); 1.95 (m, 2H); 1.75 (m, 2H); 1.55 (m, 2H); 1.25 (t, 3H).

### BEISPIEL 43:

4-(1-Methyl-piperidin-4-yl)-piperazin-1-carbonsäure-[5-chlor-3-(2-ethoxy-pyridin-3-yl)-6-fluor-1-(2-methoxy-phenylsulfonyl)-2-oxo-2,3-dihydro-1 H-indol-3-yl]-ester
ESI-MS: 702.15 [M+H]⁺
¹H-NMR (500 MHz, CDCl₃): δ [ppm] 8.15 (m, 2H); 7.90 (m, 2H); 7.55 (t, 1 H); 7.05 (t, 1 H); 7.00 (d, 1 H); 6.95 (m, 2H); 4.30 (m, 2H); 3.65 (s, 3H); 3.55 (m, 2H); 3.10 (m, 2H); 2.90 (m, 2H); 2.60-2.20 (m, 8H); 1.90 (m, 2H); 1.70 (m, 2H); 1.55 (m, 2H); 1.25 (m, 3H).

### BEISPIEL 44:

4-(1-Methyl-piperidin-4-yl)-piperazin-1-carbonsäure-[5-chlor-3-(2-ethoxy-pyridin-3-yl)-6-fluor-1-(4-methoxy-2-trifluormethoxy-phenylsulfonyl)-2-oxo-2,3-dihydro-1H-indol-3-yl]-ester
ESI-MS: 786.15 [M+H]⁺
¹H-NMR (500 MHz, CDCl₃): δ [ppm] 8.20 (d, 1 H); 8.15 (m, 1 H); 7.90 (m, 2H); 6.95 (m, 2H); 6.85 (m, 2H); 4.25 (m, 2H); 3.85 (s, 3H); 3.55 (m, 2H); 3.20-2.85 (m, 4H); 2.60-2.35 (m, 4H); 2.35 (m, 4H); 1.95 (m, 2H); 1.70 (m, 2H); 1.60 (m, 2H); 1.25 (t, 3H).

### BEISPIEL 45:

4-(1-Methyl-piperidin-4-yl)-piperazin-1-carbonsäure-[1-phenylsulfonyl-5-chlor-3-(2-ethoxy-pyridin-3-yl)-6-fluor-2-oxo-2,3-dihydro-1H-indol-3-yl]-ester
ESI-MS: 672.25 [M+H]⁺
¹H-NMR (500 MHz, d₆-DMSO): δ [ppm] 8.15 (m, 1H); 8.10 (d, 2H); 7.80 (m, 1H); 7.65 (m, 3H); 7.45 (d, 1 H); 7.30 (m, 1 H); 7.15 (m, 1 H); 4.10 (m, 1 H); 3.95 (m, 1 H); 3.60 (m, 2H); 3.20 (m, 2H); 3.00 (m, 2H); 2.85 (m, 2H); 2.65 (s, 3H); 2.45 (m, 3H); 2.30 (m, 2H); 1.85 (m, 2H); 1.65 (m, 2H); 0.95 (t, 3H).

### BEISPIEL 46:

4-(1-Methyl-piperidin-4-yl)-piperazin-1-carbonsäure-[6-chlor-1-(2,4-dimethoxy-phenylsulfonyl)-3-(2-ethoxy-pyridin-3-yl)-5-fluor-2-oxo-2,3-dihydro-1H-indol-3-yl]-ester

### 46.1 6-Chlor-3-(2-ethoxy-pyridin-3-yl)-5-fluor-2-oxo-3-phenoxycarbonyloxy-2,3-dihydro-indol-1-carbonsäurephenylester

ESI-MS: 563.50 [M+H]⁺

### 46.2 4-(1-Methyl-piperidin-4-yl)-piperazin-1-carbonsäure-[6-chlor-3-(2-ethoxy-pyridin-3-yl)-5-fluor-2-oxo-2,3-dihydro-1H-indol-3-yl]-ester

ESI-MS: 532.20 [M+H]⁺

### 46.3 4-(1-Methyl-piperidin-4-yl)-piperazin-1-carbonsäure-[6-chlor-1-(2,4-dimethoxy-phenylsulfonyl)-3-(2-ethoxy-pyridin-3-yl)-5-fluor-2-oxo-2,3-dihydro-1H-indol-3-yl]-ester

ESI-MS: 732.20 [M+H]⁺
¹H-NMR (500 MHz, CDCl₃): δ [ppm] 8.10 (m, 2H); 8.05 (d, 1 H); 7.90 (d, 1 H); 6.95 (m, 1 H); 6.80 (d, 1 H); 6.55 (d, 1 H); 6.40 (s, 1 H); 4.25 (m, 2H); 3.85 (s, 3H); 3.60 (s, 3H); 3.55 (m, 2H); 3.15 (m, 2H); 2.90 (m, 2H); 2.60-2.35 (m, 4H); 2.25 (m, 4H); 1.95 (m, 2H); 1.70 (m, 2H); 1.55 (m, 2H); 1.25 (t, 3H).

### BEISPIEL 47:

4-(1-Methyl-piperidin-4-yl)-piperazin-1-carbonsäure-[6-chlor-3-(2-ethoxy-pyridin-3-yl)-5-fluor-1-(4-methoxy-phenylsulfonyl)-2-oxo-2,3-dihydro-1H-indol-3-yl]-ester
ESI-MS: 702.20 [M+H]⁺
¹H-NMR (500 MHz, CDCl₃): δ [ppm] 8.15 (m, 1 H); 8.05 (m, 3H); 7.90 (d, 1 H); 6.95 (m, 3H); 6.80 (d, 1 H); 4.25 (m, 2H); 3.85 (s, 3H); 3.55 (m, 2H); 3.10 (m, 2H); 2.90 (m, 2H); 2.65-2.35 (m, 4H); 2.25 (m, 4H); 1.95 (m, 2H); 1.75 (m, 2H); 1.55 (m, 2H); 1.25 (t, 3H).

### BEISPIEL 48:

4-(1-Methyl-piperidin-4-yl)-piperazin-1-carbonsäure-[6-chlor-3-(2-ethoxy-pyridin-3-yl)-5-fluor-1-(4-methoxy-2-trifluormethoxy-phenylsulfonyl)-2-oxo-2,3-dihydro-1H-indol-3-yl]-ester
ESI-MS: 786.15 [M+H]⁺
¹H-NMR (500 MHz, MeOD): δ [ppm] 8.15 (d, 1 H); 8.10 (m, 2H); 8.05 (d, 1 H); 7.05 (m, 3H); 6.95 (s, 1 H); 4.25 (m, 2H); 3.90 (s, 3H); 3.60 (m, 2H); 3.10 (m, 2H); 2.90 (m, 2H); 2.55 (m, 2H); 2.40 (m, 2H); 2.25 (m, 4H); 2.00 (m, 2H); 1.80 (m, 2H); 1.55 (m, 2H); 1.20 (t, 3H).

### BEISPIEL 49:

4-(1-Methyl-piperidin-4-yl)-piperazin-1-carbonsäure-[1-phenylsulfonyl-6-chlor-3-(2-ethoxy-pyridin-3-yl)-5-fluor-2-oxo-2,3-dihydro-1H-indol-3-yl]-ester
ESI-MS: 672.20 [M+H]⁺
¹H-NMR (400 MHz, CDCl₃): δ [ppm] 8.15 (m, 3H); 8.05 (d, 1 H); 7.90 (d, 1 H); 7.85 (d, 1 H); 7.65 (t, 1 H); 7.55 (m, 2H); 6.95 (m, 1 H); 6.75 (d, 1 H); 4.25 (m, 2H); 3.55 (m, 2H); 3.00 (m, 4H); 2.60-2.25 (m, 8H); 2.05 (m, 2H); 1.75 (m, 2H); 1.65 (m, 2H); 1.25 (t, 3H).

### BEISPIEL 50:

4-(1-Methyl-piperidin-4-yl)-piperazin-1-carbonsäure-[6-chlor-3-(2-ethoxy-pyridin-3-yl)-5-fluor-1-(2-methoxy-phenylsulfonyl)-2-oxo-2,3-dihydro-1H-indol-3-yl]-ester
ESI-MS: 702.20 [M+H]⁺
¹H-NMR (500 MHz, CDCl₃): δ [ppm] 8.15 (m, 3H); 7.90 (d, 1 H); 7.55 (t, 1 H); 7.05 (t, 1 H); 6.95 (m, 2H); 6.80 (d, 1 H); 4.30 (m, 2H); 3.65 (s, 3H); 3.55 (m, 2H); 3.10 (m, 2H); 2.90 (m, 2H); 2.60-2.20 (m, 8H); 1.90 (m, 2H); 1.70 (m, 2H); 1.55 (m, 2H); 1.25 (m, 3H).

### BEISPIEL 51:

4-(1-Methyl-piperidin-4-yl)-piperazin-1-carbonsäure-[1-(2,4-dimethoxy-phenylsulfonyl)-3-(2-ethoxy-pyridin-3-yl)-5,6-difluor-2-oxo-2,3-dihydro-1H-indol-3-yl]-ester

### 51.1 4-(1-Methyl-piperidin-4-yl)-piperazin-1-carbonsäure-[3-(2-ethoxy-pyridin-3-yl)-5,6-difluor-2-oxo-2,3-dihydro-1H-indol-3-yl]-ester

ESI-MS: 516.25 [M+H]⁺

### 51.2 4-(1-Methyl-piperidin-4-yl)-piperazin-1-carbonsäure-[1-(2,4-dimethoxy-phenylsulfonyl)-3-(2-ethoxy-pyridin-3-yl)-5,6-difluor-2-oxo-2,3-dihydro-1H-indol-3-yl]-ester

ESI-MS: 716.30 [M+H]⁺
¹H-NMR (500 MHz, CDCl₃): δ [ppm] 8.15 (m, 1 H); 8.05 (d, 1 H); 7.95 (m, 2H); 6.95 (m, 1 H); 6.85 (t, 1 H); 6.55 (d, 1 H); 6.40 (s, 1 H); 4.30 (m, 2H); 3.85 (s, 3H); 3.65 (s, 3H); 3.55 (m, 2H); 3.15 (m, 2H); 2.90 (m, 2H); 2.65-2.35 (m, 4H); 2.25 (m, 4H); 1.95 (m, 2H); 1.75 (m, 2H); 1.55 (m, 2H); 1.25 (m, 3H).

### BEISPIEL 52:

4-(1-Methyl-piperidin-4-yl)-piperazin-1-carbonsäure-[3-(2-ethoxy-pyridin-3-yl)-5,6-difluor-1-(4-methoxy-phenylsulfonyl)-2-oxo-2,3-dihydro-1H-indol-3-yl]-ester
ESI-MS: 686.20 [M+H]⁺
¹H-NMR (500 MHz, CDCl₃): δ [ppm] 8.15 (m, 1 H); 8.05 (d, 2H); 7.90 (d, 1 H); 7.85 (m, 1 H); 6.95 (m, 3H); 6.80 (t, 1 H); 4.25 (m, 2H); 3.85 (s, 3H); 3.55 (m, 2H); 3.20-3.00 (m, 2H); 2.90 (m, 2H); 2.65-2.35 (m, 4H); 2.25 (m, 4H); 1.95 (m, 2H); 1.75 (m, 2H); 1.55 (m, 2H); 1.25 (m, 3H).

### BEISPIEL 53:

4-(1-Methyl-piperidin-4-yl)-piperazin-1-carbonsäure-[3-(2-ethoxy-pyridin-3-yl)-5,6-difluor-1-(2-methoxy-phenylsulfonyl)-2-oxo-2,3-dihydro-1 H-indol-3-yl]-ester
ESI-MS: 686.20 [M+H]⁺
¹H-NMR (500 MHz, CDCl₃): δ [ppm] 8.15 (m, 2H); 7.95 (m, 1 H); 7.90 (d, 1 H); 7.55 (t, 1 H); 7.05 (t, 1 H); 6.95 (m, 2H); 6.85 (t, 1 H); 4.30 (m, 2H); 3.65 (s, 3H); 3.55 (m, 2H); 3.10 (m, 2H); 2.90 (m, 2H); 2.65-2.20 (m, 8H); 1.95 (m, 2H); 1.75 (m, 2H); 1.55 (m, 2H); 1.30 (m, 3H).

### BEISPIEL 54:

4-(1-Methyl-piperidin-4-yl)-piperazin-1-carbonsäure-[3-(2-ethoxy-pyridin-3-yl)-5,6-difluor-1-(4-methoxy-2-trifluormethoxy-phenylsulfonyl)-2-oxo-2,3-dihydro-1H-indol-3-yl]-ester
ESI-MS: 770.20 [M+H]⁺
¹H-NMR (500 MHz, CDCl₃): δ [ppm] 8.25 (d, 1 H); 8.15 (m, 1 H); 7.90 (m, 2H); 6.95 (m, 1 H); 6.85 (m, 3H); 4.25 (m, 2H); 3.85 (s, 3H); 3.55 (m, 2H); 3.20-2.90 (m, 4H); 2.65-2.35 (m, 4H); 2.30 (m, 4H); 1.95 (m, 2H); 1.70 (m, 2H); 1.55 (m, 2H); 1.25 (m, 3H).

### BEISPIEL 55:

4-(1-Methyl-piperidin-4-yl)-piperazin-1-carbonsäure-[1-phenylsulfonyl-3-(2-ethoxy-pyridin-3-yl)-5,6-difluor-2-oxo-2,3-dihydro-1H-indol-3-yl]-ester
ESI-MS: 656.25 [M+H]⁺
¹H-NMR (500 MHz, CDCl₃): δ [ppm] 8.15 (m, 3H); 7.90 (m, 1 H); 7.85 (m, 1 H); 7.65 (m, 1 H); 7.55 (m, 2H); 6.95 (m, 1 H); 6.80 (t, 1 H); 4.20 (m, 2H); 3.55 (m, 2H); 3.10-2.95 (m, 4H); 2.55 (m, 1 H); 2.50 (m, 1 H); 2.35 (m, 2H); 2.30 (m, 4H); 2.05 (m, 2H); 1.75 (m, 2H); 1.65 (m, 2H); 1.20 (t, 3H).

### 11.3 Verbindungen der Formel I, worin X¹ für CH₂ steht

### BEISPIEL 56:

(+)-5-Chlor-1-(2,4-dimethoxy-phenylsulfonyl)-3-(2-ethoxy-pyridin-3-yl)-6-fluor-3-{2-[4-(1-methyl-piperidin-4-yl)-piperazin-1-yl]-2-oxo-ethyl}-1,3-dihydro-indol-2-on als Trifluoressigsäuresalz

### 56.1 2-[5-Chlor-3-(2-ethoxy-pyridin-3-yl)-6-fluor-2-oxo-2,3-dihydro-1H-indol-3-yl]-malonsäuredimethylester

Zu einer auf 10 °C gekühlten Suspension von 2.311 g (57.8 mmol, 60%w/w) Natriumhydrid in 150 ml Dimethylformamid tropfte man langsam 7.26 ml (63.5 mmol) Dimethylmalonat. Anschließend rührte man die Reaktionsmischung 30 Minuten bei Raumtemperatur und gab danach 6.57 g (19.26 mmol) 3,5-Dichlor-3-(2-ethoxy-pyridin-3-yl)-6-fluor-1,3-dihydro-indol-2-on (hergestellt unter Verwendung von 5-Chlor-3-(2-ethoxy-pyridin-3-yl)-6-fluor-3-hydroxy-1,3-dihydro-indol-2-on in Analogie zu Beispiel 1.1) portionsweise in Substanz zu. Die Reaktionslösung wurde noch weitere 15 Minuten bei Raumtemperatur gerührt. Der Reaktionsverlauf wurde dünnschichtchromatographisch kontrolliert (Kieselgel, Heptan / Ethylacetat 1 : 1). Der Ansatz wurde zur Aufarbeitung in kalte 1 N HCl eingerüht und mit Dichlormethan versetzt. Die Phasen wurden getrennt, und die wässrige Phase mit Dichlormethan (1 x) extrahiert. Die vereinigte organische Phase wurde zunächst mit Wasser (1 x) und anschließend mit gesättigter Natriumchlorid-Lösung (1 x) gewaschen, über Magnesiumsulfat getrocknet, filtriert und das Lösungsmittel im Vakuum entfernt. Der Rückstand wurde erneut in etwas Dichormethan angelöst und mit Pentan versetzt. Es fielen mehrere Fraktionen Kristallisat an. Man erhielt insgesamt 3.23 g der Titelverbindung als weißen Feststoff und 1.62 g als beigefarbenen Feststoff.
ESI-MS: 437.10 [M+H]⁺

### 56.2 [5-Chlor-3-(2-ethoxy-pyridin-3-yl)-6-fluor-2-oxo-2,3-dihydro-1H-indol-3-yl]-essigsäuremethylester

Zu einer Lösung von 4.85 g (11.10 mmol) 2-[5-Chlor-3-(2-ethoxy-pyridin-3-yl)-6-fluor-2-oxo-2,3-dihydro-1 H-indol-3-yl]-malonsäuredimethylester in 5 ml Ethanol gab man 50 ml 2 N Natronlauge und rührte 1 Stunde bei Raumtemperatur. Danach konnte gemäß DC-Kontrolle (Kieselgel, Heptan / Ethylacetat 1 : 1) kein Edukt mehr nachgewiesen werden. Die Reaktionsmischung wurde in eiskalte 1 N Salzsäure eingerührt und mit Dichlormethan versetzt. Man trennte die Phasen und extrahiert die wässrige Phase einmal mit Dichlormethan. Die vereinigte organische Phase wurde zunächst mit Wasser (1 x) und anschließend mit gesättigter Natriumchlorid-Lösung (1 x) gewaschen, über Magnesiumsulfat getrocknet, filtriert und das Lösungsmittel im Vakuum entfernt. Der erhaltene Feststoff, 2-[5-Chlor-3-(2-ethoxy-pyridin-3-yl)-6-fluor-2-oxo-2,3-dihydro-1H-indol-3-yl]-malonsäuremonomethylester (4.58 g), wurde im Vakuumtrockenschrank bei 40 °C getrocknet.
ESI-MS: 423.10 [M+H]⁺

Der erhaltene 2-[5-Chlor-3-(2-ethoxy-pyridin-3-yl)-6-fluor-2-oxo-2,3-dihydro-1H-indol-3-yl]-malonsäuremonomethylester (4.58 g) wurde in einem mit Stickstoff inertisierten Einhalskolben auf 150 °C erwärmt. Dabei bildete sich unter CO₂-Gasentwicklung [5-Chlor-3-(2-ethoxy-pyridin-3-yl)-6-fluor-2-oxo-2,3-dihydro-1H-indol-3-yl]-essigsäuremethylester. Der Reaktionsansatz wurde auf Raumtemperatur abgekühlt, und die Substanz mit Methanol versetzt. Es bildete sich ein Kristallisat, das im Kühlschrank bei 5 °C über Nacht aufbewahrt wurde. Der Feststoff wurde abgesaugt und mit mit wenig Methanol gewaschen. Man erhielt 3.286 g (8.68 mmol, 80 % Ausbeute) der Titelverbindung als weißer Feststoff. ESI-MS: 379.05 [M+H]+

### 56.3 5-Chlor-3-(2-ethoxy-pyridin-3-yl)-6-fluor-2-oxo-2,3-dihydro-1H-indol-3-yl]-essigsäure

Zu einer Lösung von 3.28 g (8.66 mmol) [5-Chlor-3-(2-ethoxy-pyridin-3-yl)-6-fluor-2-oxo-2,3-dihydro-1H-indol-3-yl]-essigsäuremethyl ester in 70 ml Ethanol gab man zunächst 5 ml Wasser und anschließend 2.3 ml einer 50%-igen Natronlauge. Man rührte die Reaktionsmischung 5 Stunden bei Raumtemperatur. Laut TLC-Kontrolle (Kieselgel, Dichlormethan / Methanol 9 : 1) war die Reaktion vollständig erfolgt. Zur Aufarbeitung wurde die Reaktionsmischung am Rotationsverdampfer eingeengt und mit 1 N Salzsäure auf pH 1-2 gestellt. Die weiße Suspension wurde für 4 Stunden im Kühlschrank aufbewahrt, der ausgefallene Feststoff abfiltriert und der erhaltene Feststoff wurde im Vakuumtrockenschrank bei 40 °C getrocknet. Man erhielt 3.12 g (8.55 mmol, 99 % d.Th. Ausbeute) der Titelverbindung .
ESI-MS: 365.15 [M+H]⁺

### Enantiomerentrennung

Zu einer Lösung von 3.12 g (8.55 mmol) [5-Chlor-3-(2-ethoxy-pyridin-3-yl)-6-fluor-2-oxo-2,3-dihydro-1H-indol-3-yl]-essigsäure in 30 ml Methanol wurden 2.518 g (8.55 mmol) (+)-Cinchonidine (Drehwert in CHCl₃: +218°) gegeben. Die Reaktionsmischung wurde 30 Minuten bei 64 °C gerührt und anschließend langsam abgekühlt. Der ausgefallene Feststoff wurde bei - 40 °C abfiltriert, mit warmen Methanol und Diethylether gewaschen und anschließend im Vakuumtrockenschrank bei 40 °C getrocknet (288 mg). Eine weitere Kristallisationsfraktion wurde bei Raumtemperatur abfiltriert, mit warmen Methanol und Diethylether gewaschen und anschließend im Vakuumtrockenschrank bei 40 °C getrocknet (1.24 g). Die Reaktionslösung wurde für einige Stunden im Kühlschrank aufgewahrt und anschließend wurde der ausgefallene Feststoff mit warmen Methanol und Diethylether gewaschen und im Vakuumtrockenschrank bei 40 °C getrocknet (175 mg). Nachdem die Mutterlauge auf 50 ml eingeengt worden war, wurde erneut der ausgefallene Feststoff abfiltriert, mit warmen Methanol und Diethylether gewaschen und anschließend im Vakuumtrockenschrank bei 40 °C getrocknet (355 mg).

Die 4 Kristallisationsfraktionen wurden jeweils in einem 1:1 Gemisch aus Essigsäureethylester und Wasser gelöst und anschließend mit konzentrierter HCl auf pH 1-0 eingestellt. Nach 15 Minuten rührens wurden die Phasen getrennt. Die wässrige Phase wurde mit Essigsäureethylester extrahiert. Die vereinigte organische Phase wurde zunächst mit Wasser (1 x) und anschließend mit gesättigter Natriumchlorid-Lösung (1 x) gewaschen, über Magnesiumsulfat getrocknet, filtriert und das Lösungsmittel im Vakuum entfernt.
1. Kristallisationsfraktion: 175 mg (Drehwert in CHCl₃: +70°)
2. Kristallisationsfraktion: 749 mg (Drehwert in CHCl₃: +65°)
3. Kristallisationsfraktion: 109 mg (Drehwert in CHCl₃: +74°)
4. Kristallisationsfraktion: 208 mg (Drehwert in CHCl₃: +72°)
ESI-MS: 365.15 [M+H]⁺

### 56.4 5-Chlor-3-(2-ethoxy-pyridin-3-yl)-6-fluor-3-{2-[4-(1-methyl-piperidin-4-yl)-piperazin-1-yl]-2-oxo-ethyl}-1,3-dihydro-indol-2-on

Zu einer Lösung von 150 mg (0.41 mmol) [5-Chlor-3-(2-ethoxy-pyridin-3-yl)-6-fluor-2-oxo-2,3-dihydro-1H-indol-3-yl]-essigsäure in 2 ml Dichlormethan gab man 200 mg (0.45 mmol) BOP, 1.07 ml (6.17 mmol) Diisopropylethylamin und anschließend 79 mg (0.43 mmol) 1-(1-Methylpiperidin-4-yl)-piperazin. Die Reaktionsmischung wurde bei Raumtemperatur über Nacht gerührt. Zu der Reaktionslösung wurden 6 ml einer 2-molaren NaOH zugegeben und 15 minuten bei Raumtemperatur gerührt. Der Reaktionsansatz wurde mit Ethylacetat verdünnt. Die Phasen wurden getrennt und die wässrige Phase mit Ethylacetat extrahiert (1 x). Die vereinigte organische Phase wurde noch mit Wasser (1 x) und gesättigter Natriumchlorid-Lösung (1 x) gewaschen, über Magnesiumsulfat getrocknet, filtriert und das Lösungsmittel im Vakuum verdampft. Der Rückstand wurde chromatographisch über Kieselgel (Redisep-Kartusche, Laufmittel-Gradient von 2 bis 50% Methanol in Dichlormethan) gereinigt. Man erhielt 181 mg der Titelverbindung als weißen Feststoff. ESI-MS: 530.50 [M+H]⁺

### 56.5 (+)-5-Chlor-1-(2,4-dimethoxy-phenylsulfonyl)-3-(2-ethoxy-pyridin-3-yl)-6-fluor-3-{2-[4-(1-methyl-piperidin-4-yl)-piperazin-1-yl]-2-oxo-ethyl}-1,3-dihydro-indol-2-on

Zu einer auf 0 °C gekühlten Lösung von 180 mg (0.34 mmol) 5-Chlor-3-(2-ethoxy-pyridin-3-yl)-6-fluor-3-{2-[4-(1-methyl-piperidin-4-yl)-piperazin-1-yl]-2-oxo-ethyl}-1,3-dihydro-indol-2-on in 2 ml Dimethylformamid gab man 16.3 mg (0.41 mmol, 60%w/w) Natriumhydrid und nach 10 Minuten 96 mg (0.41 mmol) 2,4-Dimethoxyphenylsulfonylchlorid. Den Reaktionsansatz ließ man auf Raumtemperatur erwärmen und rührte 50 Minuten nach. Der Reaktionsverlauf wurde dünnschichtchromatographisch verfolgt (Kieselgel, Dichlormethan / Methanol 15 : 5). Zu der Reaktionsmischung gab man Wasser und Ethylacetat. Die beiden Phasen wurden anschließend getrennt und die wässrige Phase nochmals mit Ethylacetat (1 x) extrahiert. Die vereinigte organische Phase wurde mit Wasser (1 x) und mit gesättigter Natriumchlorid-Lösung (1 x) gewaschen, über Magnesiumsulfat getrocknet, filtriert und das Lösungsmittel im Vakuum evaporiert. Der Rückstand wurde über präparative HPLC (RP, Eluenten Acetonitril / Wasser, 0.01 % TFA) gereinigt. Man erhielt 116 mg (0.16 mmol, 46 %, 99 % Reinheit) der Titelverbindung als weißen Feststoffs.
Drehwert α (CHCl₃): plusdrehend
ESI-MS: 730.55 [M+H]⁺
¹H-NMR (500 MHz, d₆-DMSO): δ [ppm] 10.20 (bs, 1 H); 8.10 (m, 1 H); 7.95 (m, 1 H); 7.85 (d, 1 H); 7.70 (d, 1 H); 7.35 (d, 1 H); 7.05 (t, 1 H); 6.65 (m, 2H); 4.10 (m, 2H); 3.95 (m, 2H); 3.85 (s, 3H); 3.65 (s, 3H); 3.60 (m, 2H); 3.35 (m, 4H); 3.00 (m, 5H); 2.80 (s, 3H); 2.50 (s, 2H); 2.25 (m, 2H); 1.85 (m, 2H); 1.00 (t, 3H).

### III. BESTIMMUNG DER BIOLOGISCHEN AKTIVITÄT

### 1. Vasopressin V1b Rezeptorbindungstest:

### Substanzen:

Die Testsubstanzen wurden in einer Konzentration von 10⁻² M in DMSO gelöst und in DMSO auf 5x10⁻⁴ M bis 5x10⁻⁹ M weiter verdünnt. Diese DMSO-Vorverdünnungsreihe wurde mit Testpuffer 1:10 verdünnt. Im Testansatz wurde die Substanzkonzentration nochmals 1:5 verdünnt (2 % DMSO im Ansatz).

### Membran-Präparation:

CHO-K1 Zellen mit stabil exprimiertem humanem Vasopressin V1 b Rezeptor (Klon 3H2) wurden abgeerntet und in 50 mM Tris-HCl und in Gegenwart von Proteaseinhibitoren (Roche complete Mini # 1836170) mit einem Polytron Homogenizer auf mittlerer Stellung 2x10 Sekunden homogenisiert und anschließend 1h bei 40.000 x g abzentrifugiert. Das Membranpellet wurde nochmals wie beschrieben homogenisiert und zentrifugiert und anschließend in 50 mM Tris-HCl, pH 7,4 aufgenommen, homogenisiert und in Aliquots bei -190°C in flüssigem Stickstoff eingefroren aufbewahrt.

### Bindungstest:

Der Bindungstest wurde in Anlehnung an die Methode von Tahara et al. (Tahara A et al., Brit. J. Pharmacol. 125, 1463-1470 (1998)) durchgeführt. Inkubationspuffer war: 50 mM Tris, 10 mM MgCl₂, 0.1% BSA, pH 7.4.

Im Testansatz (250 µl) wurden Membranen (50 µg/ml Protein in Inkubationspuffer) von CHO-K1-Zellen mit stabil exprimierten humanen V1b Rezeptoren (Zelllinie hV1b_3H2_CHO) mit 1,5 nM ³H-AVP (8-Arg-Vasopressin, PerkinElmer #18479) in Inkubationspuffer (50 mM Tris, 10 mM MgCl₂, 0.1 % BSA, pH 7.4) (totale Bindung) oder zusätzlich mit steigenden Konzentrationen an Testsubstanz (Verdrängungsexperiment) inkubiert. Die unspezifische Bindung wurde mit 1 □M VP (Bachem # H1780) bestimmt. Alle Bestimmungen wurden als Dreifachbestimmungen durchgeführt. Nach Inkubation (60 Minuten bei Raumtemperatur), wurde der freie Radioligand mittels Vakuumfiltration (Skatron cell harvester 7000) über Wathman GF/B Glasfaserfiltermatten abfiltriert und die Filter in Szintillationsgefäße überführt. Die Flüssigszintillationsmessung erfolgte in einem Tricarb-Gerät Model 2000 oder 2200CA (Packard). Die Umrechnung der gemessenen cpm in dpm wurde mit Hilfe einer Standardquenchreihe durchgeführt.

### Auswertung:

Die Bindungsparameter wurden durch nichtlineare Regression in SAS berechnet. Die Algorithmen des Programms arbeiten analog zum LIGAND Auswerteprogramm (Munson PJ und Rodbard D, Analytical Biochem. 107, 220-239 (1980)). Der Kd-Wert von ³H-AVP zu den rekombinanten humanen V1b-Rezeptoren beträgt 0,4 nM und wurde zur Bestimmung des Ki-Wertes herangezogen.

### 2. Vasopressin V1a Rezeptorbindungstest:

### Substanzen:

Die Testsubstanzen wurden in einer Konzentration von 10⁻² M in DMSO gelöst. Die weitere Verdünnung dieser DMSO-Lösungen erfolgte in Inkubationspuffer (50 mM Tris, 10 mM MgCl₂, 0.1 % BSA, pH 7,4).

### Membran-Präparation:

CHO-K1 Zellen mit stabil exprimiertem humanem Vasopressin V1a Rezeptor (Klon 5) wurden abgeerntet und in 50 mM Tris-HCl und in Gegenwart von Proteaseinhibitoren (Roche complete Mini # 1836170) mit einem Polytron Homogenizer auf mittlerer Stellung 2x10 Sekunden homogenisiert und anschließend 1 h bei 40.000 x g abzentrifugiert. Das Membranpellet wurde nochmals wie beschrieben homogenisiert und zentrifugiert und anschließend in 50 mM Tris-HCl, pH 7,4 aufgenommen, homogenisiert und in Aliquots bei -190°C in flüssigem Stickstoff eingefroren aufbewahrt.

### Bindungstest:

Der Bindungstest wurde in Anlehnung an die Methode von Tahara et al. (Tahara A et al., Brit. J. Pharmacol. 125, 1463-1470 (1998)) durchgeführt.
Inkubationspuffer war: 50 mM Tris, 10 mM MgCl₂, 0.1% BSA, pH 7.4.
Im Testansatz (250 µl) wurden Membranen (20 µg/ml Protein in Inkubationspuffer) von CHO-K1-Zellen mit stabil exprimierten humanen V1a Rezeptoren (Zelllinie hV1a_5_CHO) mit 0.04 nM ¹²⁵I-AVP (8-Arg-Vasopressin, NEX 128) in Inkubationspuffer (50 mM Tris, 10 mM MgCl₂, 0.1 % BSA, pH 7.4) (totale Bindung) oder zusätzlich mit steigenden Konzentrationen an Testsubstanz (Verdrängungsexperiment) inkubiert. Die unspezifische Bindung wurde mit 1 µM AVP (Bachem # H1780) bestimmt. Dreifachbestimmungen wurden durchgeführt.

Nach Inkubation (60 Minuten bei Raumtemperatur), wurde der freie Radioligand mittels Vakuumfiltration (Skatron cell harvester 7000) über Wathman GF/B Glasfaserfiltermatten abfiltriert und die Filter in Szintillationsgefäße überführt.

Die Flüssigszintillationsmessung erfolgte in einem Tricarb-Gerät Model 2000 oder 2200CA (Packard). Die Umrechnung der gemessenen cpm in dpm wurde mit Hilfe einer Standardquenchreihe durchgeführt.

### Auswertung:

Die Bindungsparameter wurden durch nichtlineare Regression in SAS berechnet. Die Algorithmen des Programms arbeiten analog zum LIGAND Auswerteprogramm (Munson PJ und Rodbard D, Analytical Biochem. 107, 220-239 (1980)). Der Kd-Wert von ¹²⁵I -AVP zu den rekombinanten hV1a-Rezeptoren wurde in Sättigungsexperimenten bestimmt. Ein Kd-Wert von 1,33 nM wurde zur Bestimmung des Ki-Wertes herangezogen.

### 3. Vasopressin V2 Rezeptorbindungstest:

### Substanzen:

Die Testsubstanzen wurden in einer Konzentration von 10⁻² M in DMSO gelöst. Die weitere Verdünnung dieser DMSO-Lösung erfolgte in Inkubationspuffer (50 mM Tris, 10 mM MgCl₂, 0.1 % BSA, pH 7,4).

### Membran-Präparation:

CHO-K1 Zellen mit stabil exprimiertem humanem Vasopressin V2 Rezeptor (Klon 23) wurden abgeerntet und in 50 mM Tris-HCl und in Gegenwart von Proteaseinhibitoren (Roche complete Mini # 1836170) mit einem Polytron Homogenizer auf mittlerer Stellung 2x10 Sekunden homogenisiert und anschließend 1 h bei 40.000 x g abzentrifugiert. Das Membranpellet wurde nochmals wie beschrieben homogenisiert und zentrifugiert und anschließend in 50 mM Tris-HCl, pH 7,4 aufgenommen, homogenisiert und in Aliquots bei -190°C in flüssigem Stickstoff eingefroren aufbewahrt.

### Bindungstest:

Der Bindungstest wurde in Anlehnung an die Methode von Tahara et al. (Tahara A et al., Brit. J. Pharmacol. 125, 1463-1470 (1998)) durchgeführt. Inkubationspufferwar: 50 mM Tris, 10 mM MgCl₂, 0.1% BSA, pH 7.4. Im Testansatz (250 µl) wurden Membranen (50 µg/ml Protein in Inkubationspuffer) von CHO-K1-Zellen mit stabil exprimierten humanen V2 Rezeptoren (Zelllinie hV2_23_CHO) mit 1-2 nM ³H-AVP (8-Arg-Vasopressin, PerkinElmer #18479) in Inkubationspuffer (50 mM Tris, 10 mM MgCl₂, 0.1 % BSA, pH 7.4) (totale Bindung) oder zusätzlich mit steigenden Konzentrationen an Testsubstanz (Verdrängungsexperiment) inkubiert. Die unspezifische Bindung wurde mit 1 µM AVP (Bachem # H1780) bestimmt. Dreifachbestimmungen wurden durchgeführt.

Nach Inkubation (60 Minuten bei Raumtemperatur), wurde der freie Radioligand mittels Vakuumfiltration (Skatron cell harvester 7000) über Wathman GF/B Glasfaserfiltermatten abfiltriert und die Filter in Szintillationsgefäße überführt.

Die Flüssigszintillationsmessung erfolgte in einem Tricarb-Gerät Model 2000 oder 2200CA (Packard). Die Umrechnung der gemessenen cpm in dpm wurde mit Hilfe einer Standardquenchreihe durchgeführt.

### Auswertung:

Die Bindungsparameter wurden durch nichtlineare Regression in SAS berechnet. Die Algorithmen des Programms arbeiten analog zum LIGAND Auswerteprogramm (Munson PJ und Rodbard D, Analytical Biochem. 107, 220-239 (1980)). Der Kd-Wert von ³H-AVP zu den rekombinanten hV2-Rezeptoren beträgt 2,4 nM und wurde zur Bestimmung des Ki-Wertes herangezogen.

### 4. Oxytozin-Rezeptorbindungstest

### Substanzen:

Die Substanzen wurden in einer Konzentration von 10⁻² M in DMSO gelöst und mit Inkubationspuffer (50 mM Tris, 10 mM MgCl₂, 0.1% BSA, pH 7,4) verdünnt.

### Zellpräparation:

Konfluente HEK-293 Zellen mit transient exprimierenden rekombinanten humanen Oxytozinrezeptoren wurden bei 750 x g für 5 Minuten bei Raumtemperatur zentrifugiert. Der Rückstand wurde in eiskaltem Lysispuffer (50 mM Tris-HCl, 10 % Glycerin, pH 7,4 und Roche Complete Protease-Inhibitor) aufgenommen und 20 Minuten bei 4 °C einem osmotischen Schock unterworfen. Danach wurden die lysierten Zellen bei 750 x g für 20 Minuten bei 4°C zentrifugiert, der Rückstand in Inkubationspuffer aufgenommen und Aliquots von 10⁷ Zellen/ml hergestellt. Die Aliquots wurden bis zur Verwendung bei -80°C eingefroren.

### Bindungstest:

Am Tag des Versuches wurden die Zellen aufgetaut, mit Inkubationspuffer verdünnt und mit einer Multipette Combitip (Eppendorf, Hamburg) homogenisiert. Der Reaktionsansatz von 0,250 ml setzte sich zusammen aus 2 bis 5x104 rekombinante Zellen, 3-4 nM ³H-Oxytozin (PerkinElmer, NET 858) in Anwesenheit von Testsubstanz (Hemmkurve) oder nur Inkubationspuffer (totale Bindung). Die unspezifische Bindung wurde mit 10⁻⁶ M Oxytozin (Bachem AG, H2510) bestimmt. Dreifachbestimmungen wurden angesetzt. Gebundener und freier Radioligand wurden getrennt durch Filtration unter Vakuum mit Whatman GF/B Glasfaserfilter mit Hilfe eines Skatron Cell Harvester 7000. Die gebundene Radioaktivität wurde durch Flüssigkeitszintillationsmessung in einem Tricarb Beta-Zählgerät, Modell 2000 oder 2200CA (Packard) bestimmt.

### Auswertung:

Die Bindungsparameter wurden durch nicht-lineare Regressionsanalyse berechnet (SAS), analog zum Programm LIGAND von Munson und Rodbard (Analytical Biochem 1980; 107: 220-239). Der Kd-Wert von ³H-Oxytozin zu den rekombinanten hOT-Rezeptoren beträgt 7.6 nM und wurde zur Bestimmung des Ki-Wertes herangezogen.

### 5. Bestimmung der mikrosomalen Halbwertszeit:

Die metabolische Stabilität der erfindungsgemäßen Verbindungen wurde in dem folgenden Test bestimmt.

Die Testsubstanzen werden in einer Konzentration von 0.5 µM wie folgt inkubiert: In Mikrotiterplatten werden 0.5 µM Testsubstanz zusammen mit Lebermikrosomen verschiedener Spezies (von Ratte, Mensch oder andere Spezies) (0.25 mg mikrosomales Protein/ml) in 0.05M Kalium-Phosphatpuffer pH 7,4 bei 37 °C für 5 min vorinkubiert. Der Start der Reaktion erfolgt durch die Zugabe von NADPH (1 mg/mL). Nach 0, 5, 10, 15, 20 und 30 min werden 50 µl Aliquots entnommen und die Reaktion wird sofort mit dem gleichen Volumen an Acetonitril abgestoppt und runtergekühlt. Die Proben werden bis zur Analyse eingefroren. Mittels MSMS wird die verbliebene Konzentration an nicht abgebauter Testsubstanz bestimmt. Aus der Steigung der Kurve Signal Testsubstanz/Zeiteinheit wird die Halbwertszeit (T1/2) ermittelt, wobei die Halbwertszeit der Testsubstanz unter Annahme einer Kinetik erster Ordnung aus der zeitlichen Abnahme der Konzentration der Verbindung berechnet werden kann. Die mikrosomale Clearance (mCl) berechnet sich aus mCl= In2/T1/2 / (Gehalt an mikrosomalem Protein in mg/ml) x 1000 [ml/min/mg] (modifiziert nach Literaturstellen: Di, The Society for Biomoleculaur Screening, 2003, 453-462; Obach, DMD, 1999 vol 27. N 11, 1350-1359).

### 6. Methoden zur in vitro Bestimmung der Cytochrom P450 (CYP) Inhibierung

### Lumineszenzsubstrate für 2C9 und 3A4:

0.4 mg/ml humane Lebermikrosomen werden 10 min mit den zu untersuchenden Testsubstanzen (0-20 µM), den CYP-spezifischen Substraten, in 0.05 M Kaliumphosphatpuffer pH 7,4 bei 37 °C vorinkubiert. Das Cyp-spezifische Substrat für CYP 2C9 ist Luciferin H, für CYP 3A4 Luciferin BE. Die Reaktion wird durch Hinzufügen von NADPH gestartet. Nach 30 min Inkubation bei RT wird das Luciferin Detektionsreagenz hinzugefügt, und das entstandene Lumineszenzsignal gemessen (modifiziert nach Literaturstelle: Promega, Technical Bulletin P450-GLO™ Assays).

### Midazolam CYP 3A4 Time dependent Inhibition

Der Test besteht aus 2 Teilen. Einmal wird die Testsubstanz mit den Lebermikrosomen vorinkubiert (mit NADPH = Prä-Inkubation, danach Zugabe des Substrates, im 2 .Teil wird das Substrat und die Testsubstanz gleichzeitig zugegeben = Co-Inkubation.

### Prä-Inkubation:

0.05 mg/ml mikrosomales Protein (humane Lebermikrosomen) werden mit 0-10 µM (bzw 50 µM) Testsubstanz in 50 mM Kaliumphosphatpuffer 5 Min vorinkubiert. Die Reaktion wird mit NADPH gestartet. Nach 30 min werden 4 µM Midazolam (Endkonzentration) hinzugefügt und für weitere 10 Min inkubiert. 75 µl der Reaktionslösung werden nach 10 min entnommen und mit 150 µl Acetonitrillösung abgestoppt.

### Co-Inkubation:

0.05 mg/ml mikrosomales Protein (humane Lebermikrosomen) werden mit 4 µM Midazolam (Endkonzentration) und 0-10 µM (bzw 50µM) Testsubstanz in 50 mM Kaliumphosphatpuffer 5 min vorinkubiert. Die Reaktion wird mit NADPH gestartet. 75 µl der Reaktionslösung werden nach 10 min entnommen und mit 150 µl Acetonitrillösung abgestoppt. Die Proben werden bis zur MSMS-Analytik eingefroren (modifiziert nach Literaturstellen: Obdach, Journal of Pharmacology & Experimental Therapeutics, Vol 316, 1, 336-348, 2006; Walsky, Drug Metabolism and Disposition Vol 32, 6, 647-660, 2004).

### 7. Methode zur Bestimmung der Wasserlöslichkeit (in mg/ml)

Die Wasserlöslichkeit der erfindungsgemässen Verbindungen kann zum Beispiel nach der sogenannten "shake flask" Methode bestimmt werden (gemäss *ASTM International: E 1148-02, Standard test methods for measurement of aqueous solubility, Book of Standards Volume 11.05.).* Dabei wird ein Überschuss der festen Verbindung in eine Pufferlösung mit einem bestimmten pH-Wert (zum Beispiel Phosphatpuffer pH 7.4) gegeben und die entstehende Mischung geschüttelt oder gerührt, bis sich das Gleichgewicht eingestellt hat (typischerweise 24 oder 48 Stunden, manchmal auch bis zu 7 Tage). Anschließend wird der ungelöste Feststoff durch Filtrieren oder Zentrifugieren abgetrennt und die Konzentration der gelösten Verbindung durch UV-Spektroskopie oder HochdruckflüssigkeitsChromatographie (HPLC) anhand einer entsprechenden Kalibrierkurve bestimmt.

### 8. Ergebnisse

Die Ergebnisse der Rezeptor-Bindungsuntersuchungen sind als Rezeptorbindungskonstanten [Kᵢ(V1b)] bzw. Selektivitäten [Kᵢ(V1a)/ Kᵢ(V1b)] ausgedrückt. Die Ergebnisse der Untersuchung der metabolischen Stabilität sind als mikrosomale Clearance (mCl) angegeben.

In diesen Tests zeigen die erfindungsgemäßen Verbindungen sehr hohe Affinitäten für den V1b-Rezeptor (höchstens 100 nM, oder höchstens 10 nM, häufig < 1 nM). Außerdem zeigen die Verbindungen auch hohe Selektivitäten gegenüber dem V1 a- und dem Oxytozin(OT)-Rezeptor und eine gute metabolische Stabilität, gemessen als mikrosomale Clearance.

Die Ergebnisse sind in Tabelle 2 aufgeführt. Die Nummern der Verbindungen beziehen sich auf die Synthesebeispiele.

**Tabelle 2**

| Beispiel | Kᵢ(h-V1b)* [nM] | Kᵢ(h-V1a)/Kᵢ(h-V1b)* | Kᵢ(h-OT)/Kᵢ(h-V1b)* |
|---|---|---|---|
| 1 | +++ | +++ | +++ |
| 2 | +++ | +++ | +++ |
| 3 | +++ | ++ | +++ |
| 4 | +++ | ++ | +++ |
| 5 | ++ | +++ | +++ |
| 6 | ++ | +++ | +++ |
| 7 | ++ | + | +++ |
| 8 | ++ | ++ | +++ |
| 10 | ++ | ++ | + |
| 11 | +++ | +++ | +++ |
| 12 | ++ | +++ | +++ |
| 13 | ++ | ++ | +++ |
| 14 | ++ | + | +++ |
| 15 | ++ | + | +++ |
| 16 | +++ | + | +++ |
| 17 | ++ | + | +++ |
| 18 | ++ | + | +++ |
| 19 | +++ | ++ | ++ |
| 20 | ++ | + | ++ |
| 21 | +++ | ++ | +++ |
| 22 | ++ | + | +++ |
| 23 | +++ | + | +++ |
| 26 | +++ | + | +++ |
| 31 | + | +++ | + |
| 33 | ++ | +++ | +++ |
| 35 | ++ | + | + |
| 38 | +++ | +++ | +++ |
| 39 | +++ | ++ | +++ |
| 40 | ++ | + | +++ |
| 41 | +++ | + | + |
| 42 | +++ | + | + |
| 43 | +++ | ++ | +++ |
| 45 | ++ | + | +++ |

| Beispiel | Kᵢ(h-V1b)* [nM] | Kᵢ(h-V1a)/Kᵢ(h-V1 b)* | Kᵢ(h-OT)/Kᵢ(h-V1b)* |
|---|---|---|---|
| 46 | ++ | ++ | + |
| 50 | + | + | ++ |
| 51 | ++ | +++ | ++ |
| 52 | ++ | ++ | + |
| 53 | ++ | ++ | ++ |

| | | | |
|---|---|---|---|
| * h = human | | | |

**Schlüssel:**

| | Kᵢ(V1b) | Kᵢ(h-V1a)/Kᵢ(h-V1b) | Kᵢ(h-OT)/Kᵢ(h-V1b) |
|---|---|---|---|
| + | > 10 - 100nM | 10 - < 25 | 10 - < 25 |
| ++ | 1 - 10nM | 25 - 75 | 25 - 75 |
| +++ | < 1 nM | > 75 | > 75 |

### Weitere Ausführungsformen ("E-x.") der Erfindung sind:

E-1. Verbindungen der Formel I 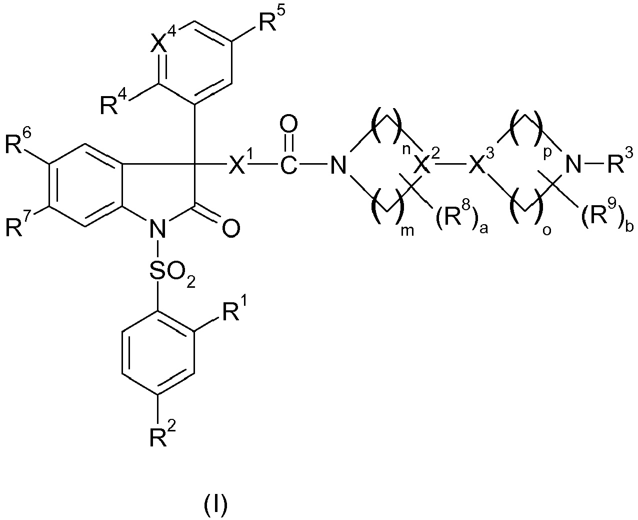 worin
   - R¹ und R²: unabhängig voneinander für Wasserstoff, C₁-C₃-Alkyl, C₁-C₃-Fluoralkyl, C₁-C₃-Alkoxy, C₁-C₃-Fluoralkoxy, Halogen oder CN stehen;
   - R³: für Wasserstoff oder C₁-C₄-Alkyl steht;
   - R⁴: für Ethoxy, fluoriertes Ethoxy oder Isopropoxy steht;
   - R⁵: für Wasserstoff oder Methyl steht;
   - R⁶: für Br, Cl, F oder CN steht;
   - R⁷: für Cl, F oder CN steht;
   - R⁸ und R⁹: unabhängig voneinander für C₁-C₃-Alkyl oder C₁-C₃-Fluoralkyl stehen;
   - X¹: für O, NH oder CH₂ steht;
   - X² und X³: für N oder CH stehen, unter der Maßgabe, dass X² und X³ nicht gleichzeitig für N stehen;
   - X⁴: für N oder CH steht;
   - a und b: unabhängig voneinander für 0, 1 oder 2 stehen; und
   - m, n, o und p: unabhängig voneinander für 1, 2 oder 3 stehen;
   sowie deren pharmazeutisch verträglichen Salze und Prodrugs davon.
E-2. Verbindungen nach Ausführungsform E-1, worin R¹ für Wasserstoff, Methoxy, Ethoxy, Fluormethoxy, Difluormethoxy oder Trifluormethoxy steht.
E-3. Verbindungen nach Ausführungsform E-2, worin R¹ für Wasserstoff, Methoxy oder Trifluormethoxy steht.
E-4. Verbindungen nach Ausführungsform E-3, worin R¹ für Wasserstoff oder Methoxy steht.
E-5. Verbindungen nach einer der vorhergehenden Ausführungsformen, worin R² für Wasserstoff oder Methoxy steht.
E-6. Verbindungen nach Ausführungsform E-5, worin R² für Methoxy steht.
E-7. Verbindungen nach einer der vorhergehenden Ausführungsformen, worin R¹ und R² für Methoxy stehen.
E-8. Verbindungen nach einer der vorhergehenden Ausführungsformen, worin R³ für Wasserstoff, Methyl oder Ethyl steht.
E-9. Verbindungen nach Ausführungsform E-8, worin R³ für Methyl oder Ethyl steht.
E-10.Verbindungen nach einer der vorhergehenden Ausführungsformen, worin R⁴ für Ethoxy steht und R⁵ für H steht.
E-11. Verbindungen nach einer der Ausführungsformen E-1 bis E-9, worin R⁴ für 2,2-Difluorethoxy oder 2,2,2-Trifluorethoxy steht und R⁵ für H steht.
E-12. Verbindungen nach einer der vorhergehenden Ausführungsformen, worin R⁶ und R⁷ nicht gleichzeitig für CN stehen.
E-13. Verbindungen nach einer der vorhergehenden Ausführungsformen, worin wenigstens einer der Reste R⁶ und R⁷ für F steht.
E-14. Verbindungen nach Ausführungsform E-13, worin R⁷ für F steht und R⁶ für F, Cl, Br oder CN steht.
E-15. Verbindungen nach Ausführungsform E-14, worin R⁷ für F steht und R⁶ für Cl oder CN steht.
E-16. Verbindungen nach einer der vorhergehenden Ausführungsformen, worin R⁸ und R⁹ für Methyl oder Ethyl stehen.
E-17. Verbindungen nach einer der vorhergehenden Ausführungsformen, worin X² für N steht und X³ für CH steht.
E-18. Verbindungen nach einer der Ausführungsformen E-1 bis E-16, worin X² für CH steht und X³ für N steht.
E-19. Verbindungen nach einer der Ausführungsformen E-1 bis E-16, worin X² für CH steht und X³ für CH steht.
E-20. Verbindungen nach einer der vorhergehenden Ausführungsformen, worin X¹ für O steht.
E-21. Verbindungen nach einer der Ausführungsformen E-1 bis E-19, worin X¹ für NH steht.
E-22. Verbindungen nach einer der vorhergehenden Ausführungsformen, worin X⁴ für N steht.
E-23. Verbindungen nach einer der vorhergehenden Ausführungsformen, worin a und b für 0 stehen.
E-24. Verbindungen nach einer der vorhergehenden Ausführungsformen, der Formel I.A 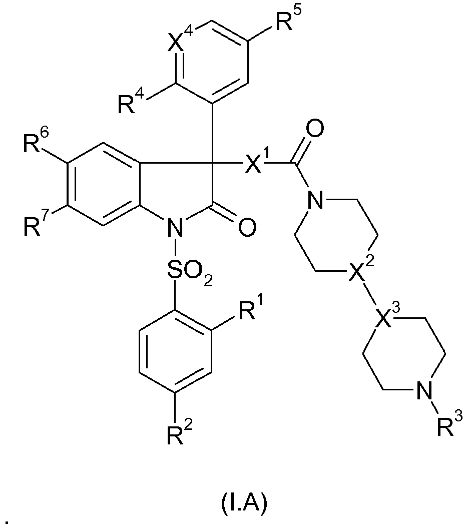 worin R¹, R², R³, R⁴, R⁵, R⁶, R⁷, X¹, X², X³ und X⁴ die in einer der vorhergehenden Ausführungsformen angegebenen Bedeutungen haben.
E-25. Verbindungen nach Ausführungsform 24, worin
   - R¹: für Methoxy steht;
   - R²: für Methoxy steht;
   - R³: für Methyl oder Ethyl steht;
   - R⁴: für Ethoxy steht;
   - R⁵: für H steht;
   - R⁶: für Cl oder CN steht;
   - R⁷: für F steht;
   - X¹: für NH steht;
   - X²: für N steht;
   - X³: für CH steht; und
   - X⁴: für N steht.
E-26. Verbindungen nach Ausführungsform 24, worin
   - R¹: für Methoxy steht;
   - R²: für Methoxy steht;
   - R³: für Methyl oder Ethyl steht;
   - R⁴: für Ethoxy steht;
   - R⁵: für H steht;
   - R⁶: für Cl oder CN steht;
   - R⁷: für F steht;
   - X¹: für NH steht;
   - X²: für CH steht;
   - X³: für N steht; und
   - X⁴: für N steht.
E-27. Verbindungen nach Ausführungsform 24, worin
   - R¹: für Methoxy steht;
   - R²: für Methoxy steht;
   - R³: für Methyl oder Ethyl steht;
   - R⁴: für Ethoxy steht;
   - R⁵: für H steht;
   - R⁶: für Cl oder CN steht;
   - R⁷: für F steht;
   - X¹: für CH₂ steht;
   - X²: für N steht;
   - X³: für CH steht; und
   - X⁴: für N steht.
E-28. Verbindungen nach Ausführungsform 24, worin
   - R¹: für Methoxy steht;
   - R²: für Methoxy steht;
   - R³: für Methyl oder Ethyl steht;
   - R⁴: für Ethoxy steht;
   - R⁵: für H steht;
   - R⁶: für Cl oder CN steht;
   - R⁷: für F steht;
   - X¹: für CH₂ steht;
   - X²: für CH steht;
   - X³: für N steht; und
   - X⁴: für N steht.
E-29. Verbindungen nach Ausführungsform 24, worin
   - R¹: für Methoxy oder H steht;
   - R²: für Methoxy steht;
   - R³: für Methyl oder Ethyl steht;
   - R⁴: für Ethoxy steht;
   - R⁵: für H steht;
   - R⁶: für Cl oder CN steht;
   - R⁷: für F steht;
   - X¹: für O steht;
   - X²: für N steht;
   - X³: für CH steht; und
   - X⁴: für N steht.
E-30. Verbindungen nach Ausführungsform 24, worin
   - R¹: für Methoxy oder H steht;
   - R²: für Methoxy steht;
   - R³: für Methyl oder Ethyl steht;
   - R⁴: für Ethoxy steht;
   - R⁵: für H steht;
   - R⁶: für Cl oder CN steht;
   - R⁷: für F steht;
   - X¹: für O steht;
   - X²: für CH steht;
   - X³: für N steht; und
   - X⁴: für N steht.
E-31. Verbindungen nach Ausführungsform 24, worin
   - R¹: für Methoxy steht;
   - R²: für Methoxy steht;
   - R³: für Methyl oder Ethyl steht;
   - R⁴: für Ethoxy steht;
   - R⁵: für H steht;
   - R⁶: für Cl oder CN steht;
   - R⁷: für F steht;
   - X¹: für NH steht;
   - X²: für N steht;
   - X³: für CH steht; und
   - X⁴: für CH steht.
E-32. Pharmazeutisches Mittel, enthaltend wenigstens eine Verbindung der Formel I gemäß der Definition in einer der vorhergehenden Ausführungsformen und/oder wenigstens ein pharmazeutisch verträgliches Salz oder Prodrug davon und wenigstens einen pharmazeutisch verträglichen Träger.
E-33. Verwendung von Verbindungen der Formel I gemäß der Definition in einer der Ausführungsformen E-1 bis E-31 oder von pharmazeutisch verträglichen Salzen oder Prodrugs davon zur Herstellung eines Medikaments zur Behandlung und/oder Prophylaxe von Vasopressin-abhängigen Krankheiten.
E-34. Verwendung von Verbindungen der Formel I gemäß der Definition in einer der Ausführungsformen E-1 bis E-31 oder von pharmazeutisch verträglichen Salzen oder Prodrugs davon zur Herstellung eines Medikaments zur Behandlung und/oder Prophylaxe von Krankheiten, die ausgewählt sind unter Diabetes, Insulin-Resistenz, Enuresis nocturna, Inkontinenz und Krankheiten, bei denen Blutgerinnungsstörungen auftreten, und/oder zur Verzögerung der Miktion.
E-35. Verwendung von Verbindungen der Formel I gemäß der Definition in einer der Ausführungsformen E-1 bis E-31 oder von pharmazeutisch verträglichen Salzen oder Prodrugs davon zur Herstellung eines Medikaments zur Behandlung und/oder Prophylaxe von Krankheiten, die ausgewählt sind unter Hypertonie, pulmonaler Hypertonie, Herzinsuffizienz, Myocardinfarkt, koronarem Spasmus, instabiler Angina, PTCA (percutaneous transluminal coronary angioplastie), Ischämien des Herzens, Störungen des renalen Systems, Ödemen, renalem Vasospasmus, Nekrose des renalen Cortex, Hyponatriämie, Hypokaliämie, Schwartz-Bartter Syndrom, Störungen des Gastrointestinaltraktes, gastritischem Vasospasmus, Hepatozirrhose, Magen- und Darmulkus, Emesis, auftretender Emesis bei der Chemotherapie, und Reisekrankheit.
E-36. Verwendung von Verbindungen der Formel I gemäß der Definition in einer der Ausführungsformen E-1 bis E-31 oder von pharmazeutisch verträglichen Salzen oder Prodrugs davon zur Herstellung eines Medikaments zur Behandlung und/oder Prophylaxe von Krankheiten, die ausgewählt sind unter affektiven Störungen.
E-37. Verwendung von Verbindungen der Formel I gemäß der Definition in einer der Ausführungsformen E-1 bis E-31 oder von pharmazeutisch verträglichen Salzen oder Prodrugs davon zur Herstellung eines Medikaments zur Behandlung und/oder Prophylaxe von Krankheiten, die ausgewählt sind unter Angststörungen und stressabhängigen Angststörungen.
E-38. Verwendung von Verbindungen der Formel I gemäß der Definition in einer der Ausführungsformen E-1 bis E-31 oder von pharmazeutisch verträglichen Salzen oder Prodrugs davon zur Herstellung eines Medikaments zur Behandlung und/oder Prophylaxe von Krankheiten, die ausgewählt sind unter Gedächtnisleistungsstörungen und Morbus Alzheimer.
E-39. Verwendung von Verbindungen der Formel I gemäß der Definition in einer der Ausführungsformen E-1 bis E-31 oder von pharmazeutisch verträglichen Salzen oder Prodrugs davon zur Herstellung eines Medikaments zur Behandlung und/oder Prophylaxe von Krankheiten, die ausgewählt sind unter Psychosen und psychotischen Störungen.
E-40. Verwendung von Verbindungen der Formel I gemäß der Definition in einer der Ausführungsformen E-1 bis E-31 oder von pharmazeutisch verträglichen Salzen oder Prodrugs davon zur Herstellung eines Medikaments zur Behandlung und/oder Prophylaxe von Krankheiten, die ausgewählt sind unter dem Cushing-Syndrom und sonstigen stress-abhängigen Krankheiten.
E-41. Verwendung von Verbindungen der Formel I gemäß der Definition in einer der Ausführungsformen E-1 bis E-31 oder von pharmazeutisch verträglichen Salzen oder Prodrugs davon zur Herstellung eines Medikaments zur Behandlung und/oder Prophylaxe von Krankheiten, die ausgewählt sind unter Schlafstörungen.
E-42. Verwendung von Verbindungen der Formel I gemäß der Definition in einer der Ausführungsformen E-1 bis E-31 oder von pharmazeutisch verträglichen Salzen oder Prodrugs davon zur Herstellung eines Medikaments zur Behandlung und/oder Prophylaxe von Krankheiten, die ausgewählt sind unter depressiven Erkrankungen.
E-43. Verwendung nach Ausführungsform E-42, zur Behandlung und/oder Prophylaxe von childhood onset mood disorders.
E-44. Verwendung von Verbindungen der Formel I gemäß der Definition in einer der Ausführungsformen E-1 bis E-31 oder von pharmazeutisch verträglichen Salzen oder Prodrugs davon zur Herstellung eines Medikaments zur Behandlung und/oder Prophylaxe von vasomotorischen Symptomen und/oder thermoregulatorischen Fehlfunktionen.
E-45. Verwendung von Verbindungen der Formel I gemäß der Definition in einer der Ausführungsformen E-1 bis E-31 oder von pharmazeutisch verträglichen Salzen oder Prodrugs davon zur Herstellung eines Medikaments zur Behandlung und/oder Prophylaxe von Drogen-, Arzneimittel- und/oder durch sonstige Faktoren vermittelten Abhängigkeiten; von Stress bedingt durch den Entzug von einem oder mehreren die Abhängigkeit vermittelnden Faktoren; und/oder von Stress-induzierten Rückfällen in die Drogen-, Arzneimittel- und/oder durch sonstige Faktoren vermittelte Abhängigkeiten.
E-46. Verwendung von Verbindungen der Formel I gemäß der Definition in einer der Ausführungsformen E-1 bis E-31 oder von pharmazeutisch verträglichen Salzen oder Prodrugs davon zur Herstellung eines Medikaments zur Behandlung und/oder Prophylaxe von Krankheiten, die ausgewählt sind unter Schizophrenie und Psychose.

## Patentansprüche

1. Verbindungen der Formel I 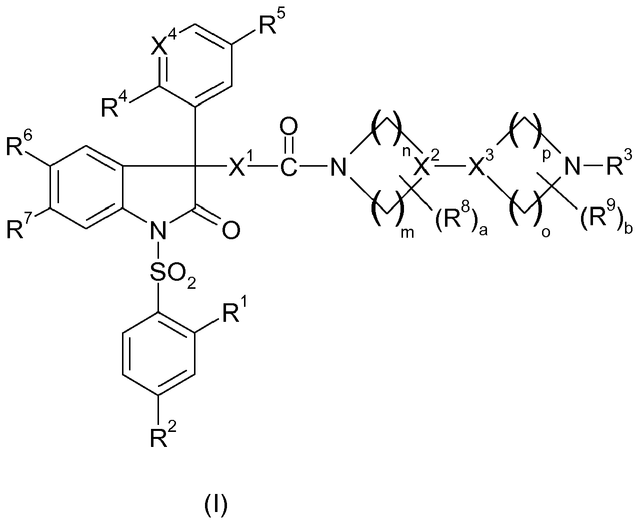 worin
R¹ und R² unabhängig voneinander für Wasserstoff, C₁-C₃-Alkyl, C₁-C₃-Fluoralkyl, C₁-C₃-Alkoxy, C₁-C₃-Fluoralkoxy, Halogen oder CN stehen;
R³ für Wasserstoff oder C₁-C₄-Alkyl steht;
R⁴ für Ethoxy, fluoriertes Ethoxy oder Isopropoxy steht;
R⁵ für Wasserstoff oder Methyl steht;
R⁶ für Br, Cl, F oder CN steht;
R⁷ für Cl, F oder CN steht;
R⁸ und R⁹ unabhängig voneinander für C₁-C₃-Alkyl oder C₁-C₃-Fluoralkyl stehen;
X¹ für O, NH oder CH₂ steht;
X² und X³ für N oder CH stehen, unter der Maßgabe, dass X² und X³ nicht gleichzeitig für N stehen;
X⁴ für N oder CH steht;
a und b unabhängig voneinander für 0, 1 oder 2 stehen; und
m, n, o und p unabhängig voneinander für 1, 2 oder 3 stehen;
sowie deren pharmazeutisch verträglichen Salze und Prodrugs davon.

2. Verbindungen nach Anspruch 1, worin R¹ für Wasserstoff, Methoxy, Ethoxy, Fluormethoxy, Difluormethoxy oder Trifluormethoxy steht.

3. Verbindungen nach Anspruch 2, worin R¹ für Wasserstoff, Methoxy oder Trifluormethoxy steht.

4. Verbindungen nach Anspruch 3, worin R¹ für Wasserstoff oder Methoxy steht.

5. Verbindungen nach einem der vorhergehenden Ansprüche, worin R² für Wasserstoff oder Methoxy steht.

6. Verbindungen nach Anspruch 5, worin R² für Methoxy steht.

7. Verbindungen nach einem der vorhergehenden Ansprüche, worin R¹ und R² für Methoxy stehen.

8. Verbindungen nach einem der vorhergehenden Ansprüche, worin R³ für Wasserstoff, Methyl oder Ethyl steht.

9. Verbindungen nach Anspruch 8, worin R³ für Methyl oder Ethyl steht.

10. Verbindungen nach einem der vorhergehenden Ansprüche, worin R⁴ für Ethoxy steht und R⁵ für H steht.

11. Verbindungen nach einem der Ansprüche 1 bis 9, worin R⁴ für 2,2-Difluorethoxy oder 2,2,2-Trifluorethoxy steht und R⁵ für H steht.

12. Verbindungen nach einem der vorhergehenden Ansprüche, worin R⁶ und R⁷ nicht gleichzeitig für CN stehen.

13. Verbindungen nach einem der vorhergehenden Ansprüche, worin wenigstens einer der Reste R⁶ und R⁷ für F steht.

14. Verbindungen nach Anspruch 13, worin R⁷ für F steht und R⁶ für F, Cl, Br oder CN steht.

15. Verbindungen nach Anspruch 14, worin R⁷ für F steht und R⁶ für Cl oder CN steht.

16. Verbindungen nach einem der vorhergehenden Ansprüche, worin R⁸ und R⁹ für Methyl oder Ethyl stehen.

17. Verbindungen nach einem der vorhergehenden Ansprüche, worin X⁴ für N steht.

18. Verbindungen nach einem der vorhergehenden Ansprüche, worin a und b für 0 stehen.

19. Verbindungen nach einem der vorhergehenden Ansprüche, der Formel I.A 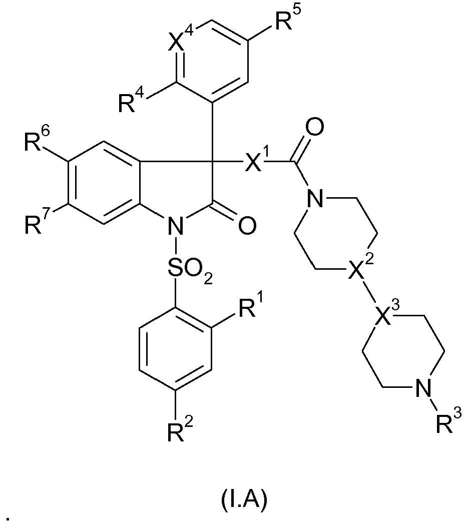 worin R¹, R2, R3, R4, R⁵, R⁶, R⁷, X¹, X², X³ und X⁴ die in einem der vorhergehenden Ansprüche angegebenen Bedeutungen haben.

20. Pharmazeutisches Mittel, enthaltend wenigstens eine Verbindung der Formel I gemäß der Definition in einem der vorhergehenden Ansprüche und/oder wenigstens ein pharmazeutisch verträgliches Salz oder Prodrug davon und wenigstens einen pharmazeutisch verträglichen Träger.

21. Verwendung von Verbindungen der Formel I gemäß der Definition in einem der Ansprüche 1 bis 19 oder von pharmazeutisch verträglichen Salzen oder Prodrugs davon zur Herstellung eines Medikaments zur Behandlung und/oder Prophylaxe von Vasopressin-abhängigen Krankheiten.

22. Verwendung von Verbindungen der Formel I gemäß der Definition in einem der Ansprüche 1 bis 19 oder von pharmazeutisch verträglichen Salzen oder Prodrugs davon zur Herstellung eines Medikaments zur Behandlung und/oder Prophylaxe von Krankheiten, die ausgewählt sind unter Diabetes, Insulin-Resistenz, Enuresis nocturna, Inkontinenz und Krankheiten, bei denen Blutgerinnungsstörungen auftreten;
Hypertonie, pulmonaler Hypertonie, Herzinsuffizienz, Myocardinfarkt, koronarem Spasmus, instabiler Angina, PTCA (percutaneous transluminal coronary angioplastie), Ischämien des Herzens, Störungen des renalen Systems, Ödemen, renalem Vasospasmus, Nekrose des renalen Cortex, Hyponatriämie, Hypokaliämie, Schwartz-Bartter Syndrom, Störungen des Gastrointestinaltraktes, gastritischem Vasospasmus, Hepatozirrhose, Magen- und Darmulkus, Emesis, auftretender Emesis bei der Chemotherapie, Reisekrankheit;
affektiven Störungen;
Angststörungen, stressabhängigen Angststörungen; Gedächtnisleistungsstörungen, Morbus Alzheimer;
Psychosen, psychotischen Störungen;
Cushing-Syndrom, sonstigen stress-abhängigen Krankheiten;
Schlafstörungen;
depressiven Erkrankungen, wie childhood onset mood disorders;
vasomotorischen Symptomen und/oder thermoregulatorischen Fehlfunktionen;
Drogen-, Arzneimittel- und/oder durch sonstige Faktoren vermittelten Abhängigkeiten; von Stress bedingt durch den Entzug von einem oder mehreren die Abhängigkeit vermittelnden Faktoren; und/oder von Stress-induzierten Rückfällen in die Drogen-, Arzneimittel- und/oder durch sonstige Faktoren vermittelte Abhängigkeiten;
Schizophrenie, Psychose;
und/oder zur Verzögerung der Miktion.
